Europäisches Patentamt
European Patent Office
Office européen des brevets

(11) Veröffentlichungsnummer: **0 255 047**
**A1**

(12) # EUROPÄISCHE PATENTANMELDUNG

(21) Anmeldenummer: **87110681.1**

(22) Anmeldetag: **23.07.87**

(51) Int. Cl.4: **C07D 239/55**, C07D 239/54, A01N 43/54

(30) Priorität: **31.07.86 CH 3090/86**
**12.06.87 CH 2207/87**

(43) Veröffentlichungstag der Anmeldung:
**03.02.88 Patentblatt 88/05**

(84) Benannte Vertragsstaaten:
**AT BE CH DE ES FR GB GR IT LI LU NL SE**

(71) Anmelder: **F. HOFFMANN-LA ROCHE & CO. Aktiengesellschaft**

**CH-4002 Basel(CH)**

(72) Erfinder: **Wenger, Jean Dr.,**
**Brunnenwiesenstrasse 1,**
**CH-8610 Uster,(CH)**
Erfinder: **Winternitz, Paul Dr.,**
**Am Pfisterhoelzli 50,**
**CH-8606 Greifensee,(CH)**

(74) Vertreter: **Lederer, Franz, Dr. et al**
**Patentanwält Dr. Franz Lederer Lucile Grahnstrasse 22**
**D-8000 München 80(DE)**

(54) **3-Aryluracile und deren Verwendung zur Unkrautbekämpfung.**

(57) Die Erfindung betrifft neue 3-Aryluracile der Formel

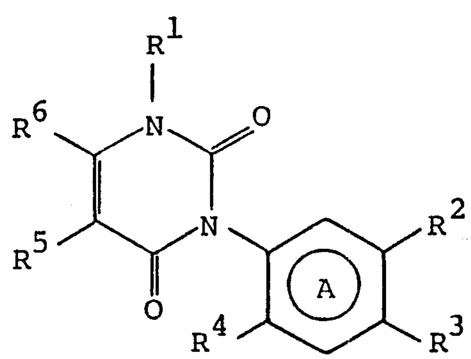

I

worin $R^1$, $R^2$, $R^3$, $R^4$, $R^5$ und $R^6$ die in der Beschreibung angegebenen Bedeutungen besitzen, sowie Salze davon und deren Herstellung, Unkrautbekämpfungsmittel, die solche Verbindungen als Wirkstoffe enthalten und die Verwendung der Wirkstoffe bzw. Mittel zur Unkrautbekämpfung. Die Erfindung betrifft ebenfalls herbizid wirksame Ausgangsmaterialien und diese enthaltende Unkrautbekämpfungsmittel.

EP 0 255 047 A1

# 3-Aryluracile und deren Verwendung zur Unkrautbekämpfung

Die vorliegende Erfindung betrifft heterocyclische Verbindungen, und zwar 3-Aryluracile der allgemeinen Formel

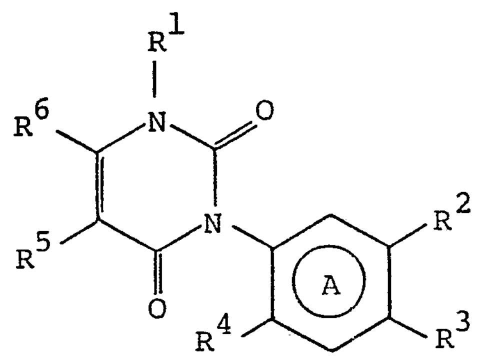

I

worin

$R^1$ Wasserstoff, $C_{1-4}$-Alkyl, $C_{1-4}$-Halogenalkyl, Formyl oder $C_{2-6}$-Alkanoyl bedeutet,

$R^2$ eine Aethergruppe oder einen eine (Thio)Carbonyloxygruppe oder Sulfonyloxygruppe enthaltenden Rest bedeutet, wobei dieser Rest unmittelbar über das Sauerstoffatom an den Benzolkern A geknüpft ist,

und

$R^3$ Halogen oder Cyano,

$R^4$ Wasserstoff oder Halogen,

$R^5$ Wasserstoff, Halogen oder $C_{1-4}$-Alkyl,

$R^6$ $C_{1-4}$-Alkyl oder $C_{1-4}$-Halogenalkyl,

oder

$R^5$ und $R^6$ zusammen Tri-oder Tetramethylen bedeuten,

sowie Salze derjenigen Verbindungen der Formel I, in denen $R^1$ Wasserstoff bedeutet.

Die erfindungsgemässen Verbindungen, d.h. die Verbindungen der Formel I sowie deren Salze, sind herbizid wirksam und eignen sich als Wirkstoffe von Unkrautbekämpfungsmitteln. Somit umfasst die Erfindung auch Unkrautbekämpfungsmittel, welche erfindungsgemässe Verbindungen als Wirkstoffe enthalten, Verfahren zur Herstellung dieser Verbindungen sowie die Verwendung solcher Verbindungen bzw. Mittel zur Bekämpfung von Unkräutern.

In der obigen Formel I umfasst "Halogen" Fluor, Chlor, Brom und Jod. Die Alkylreste können geradkettig oder verzweigt sein, wobei dies auch für den Alkylteil der Halogenalkyl-und Alkanoylgruppen gilt. Eine $C_{1-4}$-Halogenalkylgruppe kann ein oder mehrere Halogenatome aufweisen, wobei als Beispiel einer mehrfach halogenierten Alkylgruppe Trifluormethyl genannt sei.

Beispiele von $R^2$ als Aethergruppe sind folgende Reste:

$C_{1-8}$-Alkoxy
$C_{3-7}$-Cycloalkoxy
$C_{3-8}$-Alkenoxy
$C_{3-6}$-Alkinoxy
$C_{4-8}$-Cycloalkylalkoxy
gegebenenfalls substituiertes Phenyl-$C_{1-4}$-alkoxy
$C_{1-8}$-Halogenalkoxy
$C_{3-9}$-Halogenalkenoxy
$C_{3-8}$-Halogenalkinoxy
$C_{2-8}$-Cyanoalkoxy, insbesondere der Formel

```
       H/Alkyl
          |
     -O-(C)    -CN ,
           1-5
          |
       H/Alkyl
```

worin die Summe der Kohlenstoffatome 2 bis 8 beträgt;

$C_{1-8}$-Nitroalkoxy, insbesondere der Formel

```
       H/Alkyl
          |
     -O-(C)    -NO  ,
           1-5    2
          |
       H/Alkyl
```

worin die Summe der Kohlenstoffatome 1 bis 8 beträgt;

$C_{2-8}$-Alkoxyalkoxy
$C_{6-8}$-Cycloalkoxyalkoxy
$C_{4-8}$ -Alkenoxyalkoxy
$C_{5-8}$-Alkoxyalkenoxy
$C_{4-8}$-Alkinoxyalkoxy
$C_{5-8}$-Alkoxyalkinoxy
gegebenenfalls substituiertes Phenoxy-$C_{1-4}$-alkoxy
gegebenenfalls substituiertes Phenoxy-$C_{3-4}$-alkinoxy
gegebenenfalls substituiertes Benzyloxy-$C_{1-4}$-alkoxy
C $_{4-8}$-Alkoxyalkoxyalkoxy
$C_{4-9}$-Dialkoxyalkoxy, insbesondere der Formel

```
      H/Alkyl H/Alkyl
         |       |
    -O-(C)    -C-O-Alkyl  ,
          1-3
         |       |
      H/Alkyl C-Alkyl
```

worin die Summe der Kohlenstoffatome 4 bis 9 beträgt;

$C_{3-8}$ -Halogenalkoxyalkoxy
$C_{4-8}$-Halogenalkenoxyalkoxy
$C_{2-8}$-Alkylthioalkoxy
$C_{6-8}$-Cycloalkylthioalkoxy
$C_{4-8}$-Alkenylthioalkoxy
$C_{5-8}$-Alkylthioalkenoxy
$C_{4-8}$-Alkinthioalkoxy
$C_{5-8}$-Alkylthioalkinoxy
gegebenenfalls substituiertes Phenylthio-$C_{1-4}$-alkoxy
C $_{2-8}$-Alkylsulfinylalkoxy
gegebenenfalls substituiertes Phenylsulfinyl-$C_{1-4}$-alkoxy
$C_{2-8}$ -Alkylsulfonylalkoxy
gegebenenfalls substituiertes Phenylsulfonyl-$C_{1-4}$-alkoxy
$C_{3-8}$-Alkoxycarbonylalkoxy

Formyl-, Alkanoyl-oder gegebenenfalls substituiertes Benzoylalkoxy, insbesondere der Formel

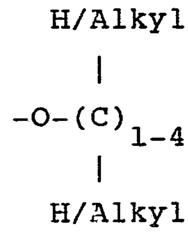

-CO-H/Alkyl/gegebenenfalls substituiertes Phenyl, worin die Summe der Kohlenstoffatome mit Ausnahme derjenigen einer allfällig vorhandenen gegebenenfalls substituierten Phenylgruppe 2 bis 8 beträgt;

gegebenenfalls N-monosubstituiertes oder N,N-disubstituiertes Carbamoylalkoxy, insbesondere der Formel

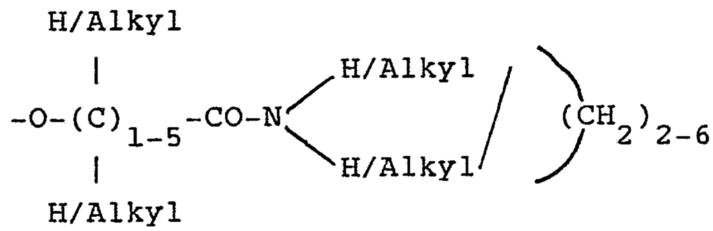 ,

worin die Summe der Kohlenstoffatome 2 bis 10 beträgt;

gegebenenfalls N-monosubstituiertes oder N,N-disubstituiertes β-Aminoäthoxy, insbesondere der Formel

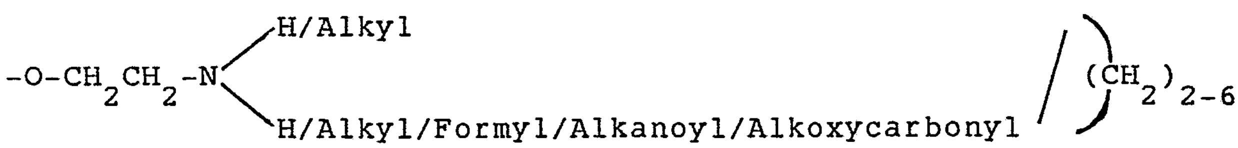 ,

worin die Summe der Kohlenstoffatome 2 bis 8 beträgt;

$C_{4-8}$-Trialkylsilylalkoxy, insbesondere der Formel

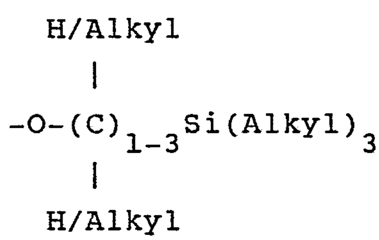 ,

worin die Alkylgruppen gleich oder verschieden sind und die Summe der Kohlenstoffatome 4 bis 8 beträgt;

eine Gruppe der Formel

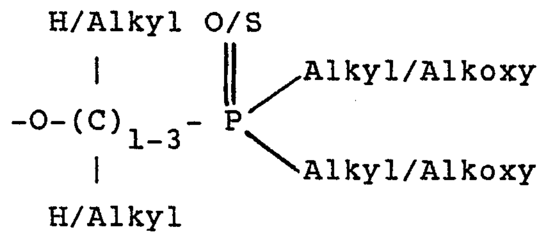 ,

worin die Summe der Kohlenstoffatome 3 bis 8 beträgt;
$C_{3-9}$-Alkylidenaminooxyalkoxy, insbesondere der Formel

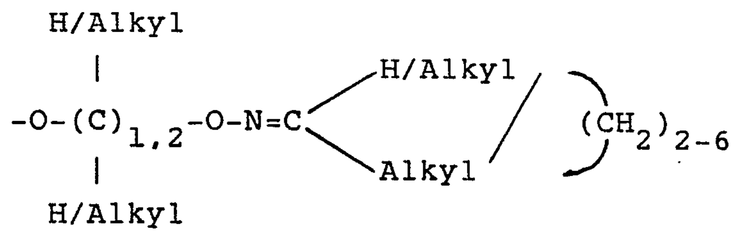 ,

worin die Summe der Kohlenstoffatome 3 bis 9 beträgt;
eine Gruppe der Formel

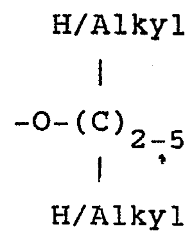

-O-H/Formyl/Alkanoyl/Halogenalkanoyl/Alkoxycarbonyl/

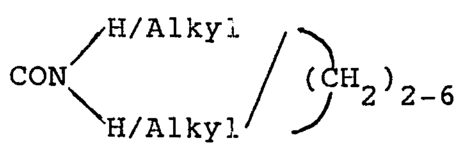

worin die Summe der Kohlenstoffatome 3 bis 10 beträgt;

Heterocyclyloxy oder Heterocyclylalkoxy, insbesondere der Formel $-O-(CH_2)_{0-3}$-Heterocyclyl, worin die Heterocyclylgruppe 3-bis 7-gliedrig, ungesättigt, teilweise gesättigt oder völlig gesättigt, und unsubstituiert oder substituiert ist und bis 4 aus Stickstoff, Sauerstoff und Schwefel ausgewählte Heteroatome enthält;

Beispiele von $R^2$ als einem eine (Thio)Carbonyloxygruppe enthaltenden Rest sind folgende Reste:

Formyloxy
$C_{2-8}$-Alkanoyloxy
$C_{2-5}$-Thioalkanoyloxy
$C_{4-8}$-Cycloalkanoyloxy
$C_{3-8}$-Alkenoyloxy
$C_{3-8}$ -Alkinoyloxy
$C_{5-8}$-Cycloalkylalkanoyloxy
gegebenenfalls substituiertes Benzoyloxy
gegebenenfalls substituiertes Phenyl-$C_{2-4}$-alkanoyloxy

$C_{2-8}$-Halogenalkanoyloxy
$C_{3-6}$-Cyanoalkanoyloxy
$C_{3-8}$-Alkoxyalkanoyloxy
$C_{4-9}$-Alkylcarbonylalkanoyloxy, insbesondere der Formel

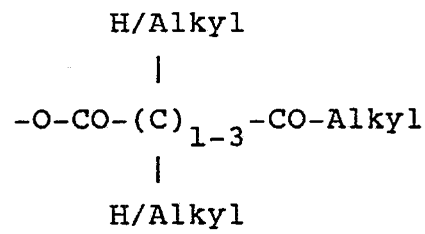

worin die Summe der Kohlenstoffatome 4 bis 9 beträgt;
$C_{2-8}$-Alkoxycarbonyloxy
C $_{6-8}$-Cycloalkoxycarbonyloxy
$C_{3-8}$-Alkenoxycarbonyloxy
$C_{4-8}$-Alkinoxycarbonyloxy
C $_{5-8}$-Cycloalkylalkoxycarbonyloxy
gegebenenfalls substituiertes Phenoxycarbonyloxy
gegebenenfalls substituiertes Phenyl-$C_{1-4}$-alkoxycarbonyloxy
$C_{3-8}$-Halogenalkoxycarbonyloxy
$C_{4-8}$ -Alkoxyalkoxycarbonyloxy
$C_{2-8}$-(Alkylthio)carbonyloxy
$C_{2-8}$-Alkoxythiocarbonyloxy
$C_{2-8}$-(Alkylthio)thiocarbonyloxy
N-monosubstituiertes oder N,N-disubstituiertes Carbamoyloxy, insbesondere der Formel -O-CO-$NR^{21}R^{22}$, worin $R^{21}$ Wasserstoff und $R^{22}$ Alkyl, Cycloalkyl, Cycloalkylalkyl, Alkenyl, Alkinyl, gegebenenfalls substituiertes Phenyl oder gegebenenfalls substituiertes Phenyl-$C_{1-4}$-alkyl, oder $R^{21}$ und $R^{22}$ unabhängig voneinander Alkyl oder Alkenyl, oder $R^{21}$ und $R^{22}$ zusammen -$(CH_2)_{2-6}$-bedeuten, und worin, falls keine gegebenenfalls substituierte Phenylgruppe vorhanden ist, die Summe der Kohlenstoffatome 2 bis 10 beträgt;
N-monosubstituiertes oder N,N-disubstituiertes Thiocarbamoyloxy, insbesondere der Formel

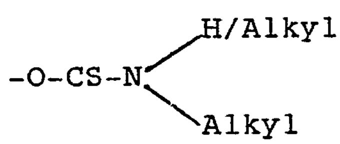

worin die Summe der Kohlenstoffatome 2 bis 9 beträgt;
eine Gruppe der Formel

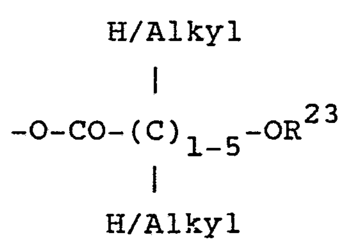 ,

worin $R^{23}$ Wasserstoff, Formyl, Alkanoyl, Halogenalkanoyl, Alkoxycarbonyl oder

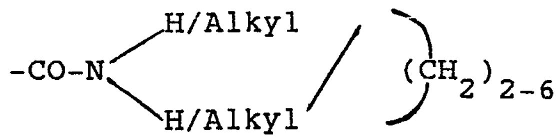

bedeutet und die Summe der Kohlenstoffatome 2 bis 10 beträgt;

eine Gruppe der Formel

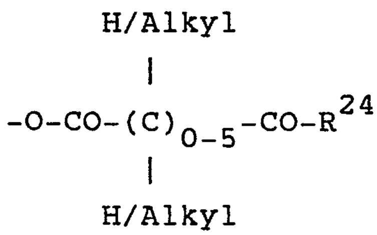

worin $R^{24}$ Hydroxy, Alkoxy, Halogenalkoxy oder

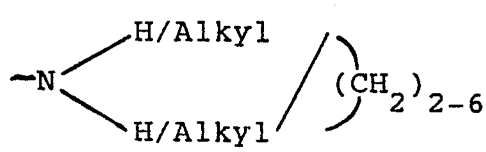

bedeutet und die Summe der Kohlenstoffatome 2 bis 10 beträgt;

Heterocyclylcarbonyloxy oder Heterocyclylalkanoyloxy, insbesondere der Formel $-O-CO-(CH_2)_{0-3}$ -Heterocyclyl, worin die Heterocyclylgruppe 3-bis 7-gliedrig, ungesättigt, teilweise gesättigt oder völlig gesättigt, und unsubstituiert oder substituiert ist und bis 4 aus Stickstoff, Sauerstoff und Schwefel ausgewählte Heteroatome enthält;

Beispiele von $R^2$ als einem eine Sulfonyloxygruppe enthaltenden Rest sind folgende Reste:

$C_{1-8}$ -Alkylsulfonyloxy

gegebenenfalls substituiertes Phenyl-$C_{1-4}$-alkylsulfonyloxy

gegebenenfalls substituiertes Phenylsulfonyloxy

$C_{1-5}$-Alkoxysulfonyloxy

N-monosubstituiertes oder N,N-disubstituiertes Sulfamoyloxy, insbesondere der Formel $-O-SO_2-NR^{25}R^{26}$, worin $R^{25}$ und $R^{26}$ Wasserstoff und Alkyl, unabhängig voneinander Alkyl, Wasserstoff und Cycloalkyl oder zusammen $-(CH_2)_{3-6}$-bedeuten und die Summe der Kohlenstoffatome 1 bis 8 beträgt.

In denjenigen obigen Formeln, in denen ein Schrägstrich (/) erscheint, bedeutet dieser Alternativen. So sind beispielsweise unter der Gruppe

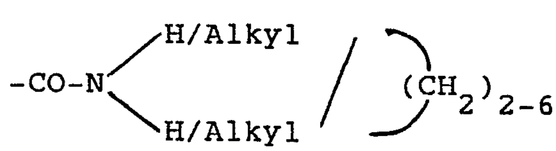

folgende Gruppen zu verstehen: $-CONH_2$, -CONHAlkyl, $-CON(Alkyl)_2$ und

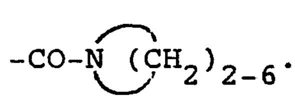

In den obigen Resten ist ein allfällig vorhandenes Halogenatom Fluor, Chlor, Brom oder Jod. Eine Halogenalkoxy-, Halogenalkenoxy-, Halogenalkinoxy-oder Halogenalkanoyloxygruppe, als solche oder als Teil eines grösseren Restes, kann ein oder mehrere (gleiche oder verschiedene) Halogenatome aufweisen. Die Alkyl-, Alkenyl-und Alkinylgruppen - als Teile grösserer Reste - können geradkettig oder verzweigt sein.

Bei gegebenenfalls substituiertem Phenyl, Phenoxy, Phenylthio, Benzyloxy oder Benzoyl - als Teil eines grösseren Restes - handelt es sich um die diesbezügliche aromatische Gruppe, welche die im Pflanzenschutz üblichen Substituenten am Benzolkern tragen kann, wie beispielsweise Halogenatome, insbesondere Fluor, Chlor und Brom, Nitro, Cyano und niedere Alkyl-, Halogenalkyl-und Alkoxygruppen. Als Beispiele der Heterocyclylgruppen - als Teile grösserer Reste - seien genannt: Oxiranyl, 2-Furyl, 2-Tetrahydrofuryl, 1,3-Dioxolan-2-yl, 1,3-Oxathiolan-2-yl, 1,3-Dithiolan-2-yl, Tetrahydro-2H-pyran-2-yl, 2-Oxo-tetrahydro-3-furyl, 2-Thienyl, 2-Pyridyl, 3-Pyridyl, 2-Pyrimidinyl und 1-Methyl-3-trifluormethyl-1H-1,2,4-triazol-5-yl.

Bei den Salzen der Verbindungen der Formel I handelt es sich insbesondere um Alkalimetallsalze, z.B. Natrium-und Kaliumsalze; Erdalkalimetallsalze, z.B. Calcium-und Magnesiumsalze; und Ammoniumsalze, d.h. unsubstituierte Ammoniumsalze und mono-oder mehrfach-substituierte Ammoniumsalze, z.B. Triäthylammonium-, Methylammonium-und (2-Hydroxyäthyl)ammoniumsalze.

Das mögliche Vorhandensein mindestens eines asymmetrischen Kohlenstoffatoms in den Verbindungen der Formel I hat zur Folge, dass die Verbindungen in optisch isomeren Formen auftreten können. Durch das Vorliegen einer allfälligen aliphatischen C=C-Doppelbindung kann auch geometrische Isomerie auftreten. Zudem ist bei denjenigen Verbindungen der Formel I, in denen $R^1$ Wasserstoff bedeutet, nicht ausgeschlossen, dass Keto-Enol-Tautomerie [-NH-CO- $\rightleftharpoons$ -N=C(OH)-] auftritt. Die Formel I soll all diese möglichen isomeren Formen sowie Gemische davon umfassen.

Vorzugsweise besitzt $R^1$ eine der oben angegebenen Bedeutungen, die verschieden von Wasserstoff sind, insbesondere die Bedeutung Methyl. Unabhängig von $R^1$ und voneinander bedeuten $R^2$ vorzugsweise $C_{1-8}$-Alkoxy, insbesondere $C_{1-3}$-Alkoxy; $C_{3-7}$-Cycloalkoxy; $C_{3-8}$-Alkenoxy, insbesondere Allyloxy; $C_{3-6}$-Alkinoxy, insbesondere Propargyloxy; $C_{4-8}$-Cycloalkylalkoxy; gegebenenfalls substituiertes Phenyl-$C_{1-4}$-alkoxy, insbesondere gegebenenfalls substituiertes Benzyloxy; $C_{1-8}$-Halogenalkoxy; $C_{3-9}$-Halogenalkenoxy; $C_{2-8}$-Alkoxyalkoxy; gegebenenfalls substituiertes Phenoxy-$C_{1-4}$-alkoxy; $C_{4-8}$-Halogenalkenoxyalkoxy; $C_{2-8}$-Alkylthioalkoxy; N-Alkyl-oder N,N-Dialkylcarbamoylalkoxy, worin die Summe der Kohlenstoffatome 2 bis 10 beträgt; $C_{3-8}$-Alkinoyloxy; N-Alkyl-oder N,N-Dialkylcarbamoyloxy, worin die Summe der Kohlenstoffatome 2 bis 10 beträgt; $C_{1-8}$-Alkylsulfonyloxy; gegebenenfalls substituiertes Phenylsulfonyloxy; oder N-Alkyl-oder N,N-Dialkylsulfamoyloxy, worin die Summe der Kohlenstoffatome 1 bis 8 beträgt; $R^3$ vorzugsweise Halogen, insbesondere Chlor oder Brom; $R^4$ vorzugsweise Halogen, insbesondere Fluor; $R^5$ vorzugsweise Wasserstoff, Fluor oder Methyl; und $R^6$ vorzugsweise Methyl oder Trifluormethyl. Zudem sind gewisse Kombinationen der Bedeutungen von $R^1$-$R^6$ bevorzugt, und zwar:

$R^1$ verschieden von Wasserstoff, $R^5$ Wasserstoff;
$R^1$ verschieden von Wasserstoff, $R^4$ Fluor, $R^5$ Wasserstoff;
$R^1$ verschieden von Wasserstoff, $R^5$ Wasserstoff, $R^6$ Trifluormethyl;
$R^1$ Methyl, $R^4$ Fluor, $R^5$ Wasserstoff, $R^6$ Trifluormethyl;
$R^1$ Methyl, $R^3$ Chlor, $R^4$ Fluor, $R^5$ Wasserstoff, $R^6$ Trifluormethyl.

Besonders bevorzugte einzelne Verbindungen der Formel I sind:

3-(4-Chlor-2-fluor-5-propargyloxyphenyl)-1-methyl-6-trifluormethyl-2,4(1H,3H)-pyrimidindion,
3-(4-Chlor-5-isopropoxyphenyl)-1-methyl-6-trifluormethyl-2,4(1H,3H)-pyrimidindion,
3-(5-Aethoxy-4-chlor-2-fluorphenyl)-1-methyl-6-trifluormethyl-2,4(1H,3H)-pyrimidindion,
3-(4-Chlor-2-fluor-5-methoxyphenyl)-1-methyl-6-trifluormethyl-2,4(1H,3H)-pyrimidindion,
3-(5-Butoxy-4-chlor-2-fluorphenyl)-1-methyl-6-trifluormethyl-2,4(1H,3H)-pyrimidindion,
3-(4-Chlor-2-fluor-5-propoxyphenyl)-1-methyl-6-trifluormethyl-2,4(1H,3H)-pyrimidindion,
3-(5-Allyloxy-4-chlor-2-fluorphenyl)-1-methyl-6-trifluormethyl-2,4(1H,3H)-pyrimidindion,
3-(4-Chlor-2-fluor-5-isopropoxyphenyl)-1-methyl-6-pentafluoräthyl-2,4(1H,3H)-pyrimidindion,
3-(4-Chlor-2-fluor-5-isopropoxyphenyl)-5-fluor-1-methyl-6-pentafluoräthyl-2,4(1H,3H)-pyrimidindion,
3-(4-Chlor-2-fluor-5-methoxymethoxy-phenyl)-1-methyl-6-trifluormethyl-2,4(1H,3H)-pyrimidindion,
2-Chlor-5-[3,6-dihydro-2,6-dioxo-3-methyl-4-trifluormethyl-1(2H)-pyrimidinyl]-4-fluorphenyl-N-tert.-butylcarbamat,
3-[5-(2-Bromäthoxy)-4-chlor-2-fluorphenyl]-1-methyl-6-trifluormethyl-2,4(1H,3H)-pyrimidindion,
{2-Chlor-5-[3,6-dihydro-2,6-dioxo-3-methyl-4-trifluormethyl-1(2H)-pyrimidinyl]-4-fluorphenyl}-p-chlorbenzolsulfonat,
3-[4-Chlor-5-(3,3-dichlorallyloxy)-2-fluorphenyl]-1-methyl-6-trifluormethyl-2,4(1H,3H)-pyrimidindion,
3-{4-Chlor-5-[2-(2,2-dichlorvinyloxy)-äthoxy]-2-fluorphenyl}-1-methyl-6-trifluormethyl-2,4(1H,3H)-pyrimidindion,
3-{4-Chlor-2-fluor-5-[2-(2-methoxyäthoxy)-äthoxy]-phenyl}-1-methyl-6-trifluormethyl-2,4(1H,3H)-pyrimidindion,
{2-Chlor-5-[3,6-dihydro-2,6-dioxo-3-methyl-4-trifluormethyl-1(2H)-pyrimidinyl]-4-fluorphenyl}-dimethylsulfamat,

{2-Chlor-5-[3,6-dihydro-2,6-dioxo-3-methyl-4-trifluormethyl-1(2H)-pyrimidinyl]-4-fluorphenyl}-methansulfonat,
3-(4-Chlor-2-fluor-5-methylthiomethoxy-phenyl)-1-methyl-6-trifluormethyl-2,4(1H,3H)-pyrimidindion,
3-(4-Chlor-5-difluormethoxy-2-fluorphenyl)-1-methyl-6-trifluormethyl-2,4(1H,3H)-pyrimidindion,
2-Chlor-5-[3,6-dihydro-2,6-dioxo-3-methyl-4-trifluormethyl-1(2H)-pyrimidinyl]-4-fluorphenyl-2-propiolat,
3-[4-Chlor-2-fluor-5-(2-methoxyäthoxy)-phenyl]-1-methyl-6-trifluormethyl-2,4(1H,3H)-pyrimidindion,
3-[5-(2-Aethoxyäthoxy)-4-chlor-2-fluorphenyl]-1-methyl-6-trifluormethyl-2,4(1H,3H)-pyrimidindion,
3-{4-Chlor-5-[(p-chlorphenoxy)methoxy]-2-fluorphenyl}-1-methyl-6-trifluormethyl-2,4(1H,3H)-pyrimidindion,
3-(5-Benzyloxy-4-chlor-2-fluorphenyl)-1-methyl-6-trifluormethyl-2,4(1H,3H)-pyrimidindion,
2-{2-Chlor-5-[3,6-dihydro-2,6-dioxo-3-methyl-4-trifluormethyl-1(2H)-pyrimidinyl]-4-fluorphenoxy}-N,N-dimethylacetamid,
3-(4-Chlor-2-fluor-5-isopropoxyphenyl)-1-difluormethyl-6-methyl-2,4(1H,3H)-pyrimidindion,
3-(4-Chlor-5-cyclohexylmethoxy-2-fluorphenyl)-1-methyl-6-trifluormethyl-2,4(1H,3H)-pyrimidindion,
3-(4-Chlor-5-cyclopentoxy-2-fluorphenyl)-1-methyl-6-trifluormethyl-2,4(1H,3H)-pyrimidindion und
2-Chlor-5-[3,6-dihydro-2,6-dioxo-3-methyl-4-trifluormethyl-1(2H)-pyrimidinyl]-4-fluorphenyl-isopropansulfonat.

Weitere Vertreter von Verbindungen der Formel I sind diejenigen 3-(4-Chlor-2-fluor-5-$R^{2'}$-phenyl)-1-methyl-6-trifluormethyl-2,4(1H,3H)-pyrimidindione, in denen $R^{2'}$ eine der folgenden Bedeutungen besitzt: 3-Chlor-2-propinoxy, 2-Cyano-n-propoxy, Nitromethoxy, 4-Methoxy-2-butinoxy, (2-Chloräthoxy)methoxy, 4-Methylthio-2-butenoxy, Propargylthiomethoxy, 4-Methylthio-2-butinoxy, Methoxycarbonylmethoxy, Isopropoxycarbonylmethoxy, 2-Acetamidoäthoxy, 2-Methoxycarbonyloxy-äthoxy, (2-Tetrahydrofuryl)methoxy, (1,3-Oxathiolan-2-yl)methoxy, (1,3-Dithiolan-2-yl)methoxy, Tetrahydro-2H-pyran-2-yloxy, 2-Thienyloxy, 2-Pyridyloxy, Thioacetoxy, Trichloracetoxy, (3-Chlorpropionyl)oxy, Acetoacetoxy, (2-Methoxyäthoxy)carbonyloxy, N-Phenylcarbamoyloxy, N-Methylthiocarbamoyloxy, 4-Hydroxybutyryloxy, 4-Formyloxybutyryloxy, 4-Acetoxybutyryloxy, 4-(Methoxycarbonyloxy)butyryloxy, 4-(N-Methylcarbamoyloxy)butyryloxy, 3-Carboxy-propionyloxy, Methoxycarbonylacetoxy, 3-Carbamoyl-propionyloxy, 3-(N,N-Dimethylcarbamoyl)-propionyloxy, Isopropoxysulfonyloxy und N-Cyclohexylsulfamoyloxy, sowie
3-(4-Chlor-2-fluor-5-propargyloxyphenyl)-1,6-dimethyl-2,4(1H,3H)-pyrimidindion,
3-(4-Chlor-2-fluor-5-propargyloxyphenyl)-1-methyl-5,6,7,8-tetrahydro-2,4(1H,3H)-chinazolindion,
3-(5-Aethoxy-4-chlor-2-fluorphenyl)-5-fluor-1-methyl-6-trifluormethyl-2,4(1H,3H)-pyrimidindion,
3-(5-Aethoxy-4-chlor-2-fluorphenyl)-5-chlor-1-methyl-6-trifluormethyl-2,4(1H,3H)-pyrimidindion,
5-Chlor-3-(4-chlor-2-fluor-5-isopropoxyphenyl)-1-methyl-6-trifluormethyl-2,4(1H,3H)-pyrimidindion,
3-(4-Chlor-2-fluor-5-propargyloxyphenyl)-5-fluor-1-methyl-6-trifluormethyl-2,4(1H,3H)-pyrimidindion,
5-Chlor-3-(4-chlor-2-fluor-5-propargyloxyphenyl)-1-methyl-6-trifluormethyl-2,4(1H,3H)-pyrimidindion,
3-(5-Aethoxy-4-chlor-2-fluorphenyl)-5-fluor-1-methyl-6-pentafluoräthyl-2,4(1H,3H)-pyrimidindion,
3-(5-Aethoxy-4-chlor-2-fluorphenyl)-5-chlor-1-methyl-6-pentafluoräthyl-2,4(1H,3H)-pyrimidindion,
5-Chlor-3-(4-chlor-2-fluor-5-isopropoxyphenyl)-1-methyl-6-pentafluoräthyl-2,4(1H,3H)-pyrimidindion,
3-(4-Chlor-2-fluor-5-propargyloxyphenyl)-5-fluor-1-methyl-6-pentafluoräthyl-2,4(1H,3H)-pyrimidindion,
5-Chlor-3-(4-chlor-2-fluor-5-propargyloxyphenyl)-1-methyl-6-pentafluoräthyl-2,4(1H,3H)-pyrimidindion,
3-(4-Chlor-2-fluor-5-isopropoxyphenyl)-1-formyl-6-trifluormethyl-2,4(1H,3H)-pyrimidindion,
1-Acetyl-3-(4-chlor-2-fluor-5-isopropoxyphenyl)-6-trifluormethyl-2,4(1H,3H)-pyrimidindion,
3-(4-Chlor-2-fluor-5-isopropoxyphenyl)-1-methoxycarbonyl-6-trifluormethyl-2,4(1H,3H)-pyrimidindion,
3-(4-Chlor-5-propargyloxyphenyl)-1-methyl-6-trifluormethyl-2,4(1H,3H)-pyrimidindion,
3-(5-Aethoxy-4-chlorphenyl)-1-methyl-6-trifluormethyl-2,4(1H,3H)-pyrimidindion,
3-(4-Chlor-5-isopropoxyphenyl)-1-methyl-6-trifluormethyl-2,4(1H,3H)-pyrimidindion,
3-(5-Benzyloxy-4-chlorphenyl)-1-methyl-6-trifluormethyl-2,3(1H,3H)-pyrimidindion,
3-[4-Chlor-2-fluor-5-(2-fluor-1-fluormethyläthoxy)phenyl]-1-methyl-6-trifluormethyl-2,4(1H,3H)-pyrimidindion,
3-(4-Chlor-2-fluor-5-pentafluoräthoxyphenyl)-1-methyl-6-trifluormethyl-2,4(1H,3H)-pyrimidindion und
3-(4-Chlor-2-fluor-5-trifluormethoxyphenyl)-1-methyl-6-trifluormethyl-2,4(1H,3H)-pyrimidindion.

Das erfindungsgemässe Verfahren zur Herstellung der Verbindungen der Formel I und ihrer Salze ist dadurch gekennzeichnet, dass man

a) eine Verbindung der allgemeinen Formel

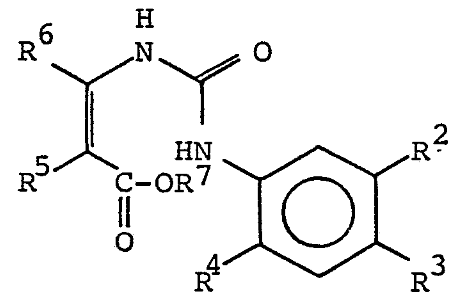

II

worin

$R^2$, $R^3$, $R^4$, $R^5$ und $R^6$ die oben angegebenen Bedeutungen besitzen,

und

$R^7$ nieder Alkyl, vorzugsweise $C_{1-4}$-Alkyl, bedeutet, einer Cyclisierung unter basischen Bedingungen unterwirft, und gegebenenfalls das so erhaltene Salz der allgemeinen Formel

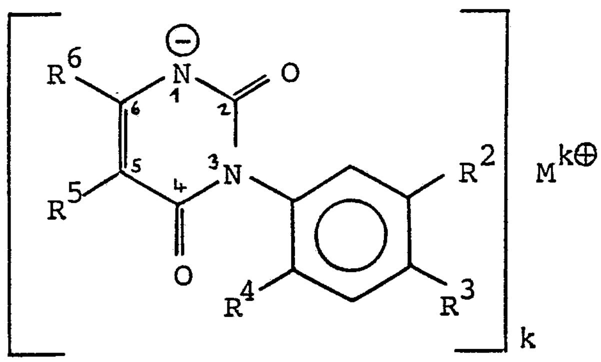

I'

worin

$R^2$, $R^3$, $R^4$, $R^5$ und $R^6$ die oben angegebenen Bedeutungen besitzen,

und

$M^{k\oplus}$ ein Kation

und

k die Wertigkeit des Kations bedeuten,

entweder in die entsprechende saure Form ($R^1$ = Wasserstoff) überführt oder mit dem gewünschten Rest $R^1$ N-substituiert, und eine so erhaltene, 5-unsubstituierte Verbindung der Formel I ($R^5$ = Wasserstoff) gegebenenfalls halogeniert, oder

b) in einer Verbindung der allgemeinen Formel

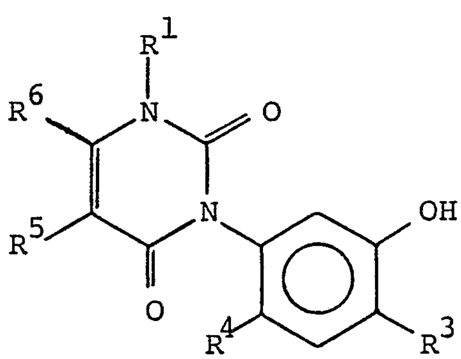

III

worin

$R^1$, $R^3$, $R^4$, $R^5$ und $R^6$ die oben angegebenen Bedeutungen besitzen,

die Hydroxygruppe in an sich bekannter Weise durch den Substituenten $R^2$ ersetzt und eine so erhaltene, 5-unsubstituierte Verbindung der Formel I ($R^5$ = Wasserstoff) gegebenenfalls halogeniert.

Die Cyclisierung nach Verfahrensvariante a) kann zweckmässigerweise durchgeführt werden, indem man die Verbindung der Formel II in einem inerten protischen organischen Lösungsmittel, wie einem Alkohol, z.B. Methanol, Aethanol oder Isopropanol; einem inerten aprotischen organischen Lösungsmittel, wie einem aliphatischen oder cyclischen Aether, z.B. 1,2-Dimethoxyäthan, Tetrahydrofuran oder Dioxan, oder einem Aromaten, z.B. Benzol oder Toluol; einem inerten aprotischen, polaren organischen Lösungsmittel, z.B. Dimethylformamid oder Dimethylsulfoxid, wobei solche Lösungsmittel gegebenenfalls im Zweiphasen-Gemisch mit einem Kohlenwasserstoff, z.B. n-Hexan, verwendet werden können; oder Wasser mit einer Base bei Temperaturen zwischen Raumtemperatur und der Rückflusstemperatur des Reaktionsgemisches behandelt. Als Basen kommen vorzugsweise Natriumalkoholate, Alkalimetallhydroxide, insbesondere Natriumhydroxid und Kaliumhydroxid, Alkalimetallcarbonate, insbesondere Natriumcarbonat und Kaliumcarbonat, und Natriumhydrid in Betracht. Bei der Verwendung der letztgenannten Base ist das Lösungsmittel vorzugsweise ein aliphatischer oder cyclischer Aether, Dimethylformamid oder Dimethylsulfoxid.

Dieses Verfahren eignet sich insbesondere zur Herstellung der erfindungsgemässen Verbindungen, in denen $R^2$ der Formel I einen basenstabilen Rest bedeutet. Unter den oben aufgeführten Resten $R^2$ gelten insbesondere die folgenden als basenstabil: Alkoxy, Cycloalkoxy, Alkenoxy, Alkinoxy, Cycloalkylalkoxy, gegebenenfalls substituiertes Phenylalkoxy, Cyanoalkoxy, Alkoxyalkoxy, Cycloalkoxyalkoxy, Alkenoxyalkoxy, Alkoxyalkenoxy, Alkinoxyalkoxy, Alkoxyalkinoxy, gegebenenfalls substituiertes Phenoxyalkoxy, gegebenenfalls substituiertes Phenoxyalkinoxy, gegebenenfalls substituiertes Benzyloxyalkoxy, Alkoxyalkoxyalkoxy, Dialkoxyalkoxy, Alkylthioalkoxy, Cycloalkylthioalkoxy, Alkenylthioalkoxy, Alkylthioalkenoxy, Alkinthioalkoxy, Alkylthioalkinoxy, gegebenenfalls substituiertes Phenylthioalkoxy, N,N-Dialkyl-$\beta$-aminoäthoxy und Trialkylsilylalkoxy.

Zur Veranschaulichung dieses Verfahrens dient als typisches Beispiel die Cyclisierung einer Verbindung der Formel II, in der $R^2$ $C_{1-8}$-Alkoxy bedeutet. Hierfür werden als Lösungsmittel polare, aprotische Lösungsmittel, wie Dimethylformamid, und Alkohole, z.B. Aethanol, insbesondere Dimethylformamid, und als Basen Natriumhydrid und Alkalimetall-Alkoholate, insbesondere Natriumäthylat, verwendet, und die Reaktion wird geeigneterweise bei Temperaturen zwischen -30°C und 30°C, vorzugsweise bei Raumtemperatur, durchgeführt.

Nach Beendigung der Cyclisierung liegt das Produkt je nach Natur der verwendeten Base in Form des entsprechenden Metallsalzes der Formel I' vor, beispielsweise im Falle der oben erwähnten Basen in Form des entsprechenden Alkalimetallsalzes. Dieses kann in an sich bekannter Weise isoliert und gereinigt werden.

Falls anschliessend eine Verbindung der Formel I, in der $R^1$ Wasserstoff bedeutet, gewünscht wird, kann man das nach der Cyclisierung entstandene Gemisch ansäuern, um das Salz I' in die entsprechende saure Form der Verbindung I ($R^1$ = Wasserstoff) überzuführen. Zu diesem Zweck verwendet man vorzugsweise eine Mineralsäure, wie Salzsäure, oder eine starke organische Säure, wie Essigsäure oder p-Toluolsulfonsäure. Auch in diesem Fall kann das Produkt in an sich bekannter Weise isoliert und gereinigt werden.

Als Alternative kann man das Salz der Formel I' durch Alkylierung bzw. Acylierung in die entsprechende N-substituierte Verbindung der Formel I überführen. Der Ausdruck "Alkylierung" oder "Acylierung" steht hier für die Einführung einer $C_{1-4}$-Alkyl-oder $C_{1-4}$-Halogenalkyl-bzw. einer Formyl-oder $C_{2-6}$-Alkanoylgruppe am unsubstituierten Stickstoffatom des Uracilkerns. Als Alkylierungsmittel wird zweckmässigerweise ein $C_{1-4}$-Alkyl-oder $C_{1-4}$-Halogenalkylhalogenid, insbesondere das diesbezügliche Chlorid oder Bromid, oder -sulfat verwendet. Als Acylierungsmittel kommt insbesondere ein Gemisch von Essigsäureanhydrid und Ameisensäure bzw. ein $C_{2-6}$-Alkancarbonsäurehalogenid in Frage, wobei das jeweilige Chlorid oder Bromid das bevorzugte Halogenid ist.

Die Alkylierung wird zweckmässigerweise in Gegenwart eines inerten, protischen organischen Lösungsmittels, wie eines niederen Alkanols, z.B. Aethanol, gegebenenfalls im Gemisch mit Wasser; eines inerten, aprotischen organischen Lösungsmittels, wie eines aliphatischen oder cyclischen Aethers, z.B. 1,2-Dimethoxyäthan, Tetrahydrofuran oder Dioxan, oder eines aliphatischen Ketons, z.B. Aceton; oder eines inerten, aprotischen, polaren organischen Lösungsmittels, z.B. Dimethylformamid oder Dimethylsulfoxid, bei Temperaturen zwischen 0°C und ca. 50°C, vorzugsweise bei Raumtemperatur, durchgeführt. In einer bevorzugten Ausführungsform wird das Salz der Formel I' im gleichen Lösungsmittel versetzt, in dem es durch Cyclisierung der Verbindung der Formel II hergestellt wurde, so dass auch noch überschüssige Base, wie im Falle der Umsetzung des Natriumsalzes der Formel I' ($M^{k\oplus}$ = $Na^{\oplus}$) Natriumhydrid, ein Natriumalkoholat oder Natriumcarbonat, im Reaktionsgemisch anwesend sein kann. Die Acylierung mit einem Halogenid kann auf ähnliche Weise erfolgen, allerdings wird in diesem Fall insbesondere in einem aprotischen Lösungsmittel und in Gegenwart von Natriumhydrid als Base gearbeitet.

Im Falle der Herstellung derjenigen Verbindungen der Formel I, in denen $R^1$ $C_{2-6}$-Alkanoyl bedeutet, kann anstelle des Salzes die saure Form der Verbindung der Formel I ($R^1$ = Wasserstoff) acyliert werden, und zwar mit dem entsprechenden Alkancarbonsäureanhydrid, geeigneterweise ohne Base, in an sich bekannter Weise.

Zwecks Herstellung derjenigen Verbindungen der Formel I, in denen $R^5$ Chlor, Brom oder Jod bedeutet, kann ein auf obige Weise erhaltenes Uracilderivat der Formel I, in der $R^5$ Wasserstoff bedeutet, also die 5-unsubstituierte Verbindung der Formel I, chloriert, bromiert bzw. jodiert werden.

Die Chlorierung bzw. Bromierung wird zweckmässigerweise mittels elementaren Chlors oder Sulfurylchlorids bzw. elementaren Broms oder Sulfurylbromids durchgeführt, und zwar in Gegenwart eines inerten organischen Lösungsmittels, wie einer aliphatischen Carbonsäure, z.B. Essigsäure, oder eines chlorierten aliphatischen Kohlenwasserstoffes, z.B. Methylenchlorid, Chloroform oder Tetrachlorkohlenstoff, und in einem Temperaturbereich von 0°C bis 60°C, vorzugsweise bei Raumtemperatur. Zudem kann die Umsetzung unter Zuhilfenahme eines säurebindenden Mittels erfolgen, zu welchem Zweck Natriumacetat und tertiäre Amine, wie Triäthylamin, Dimethylanilin und Pyridin, besonders bevorzugte säurebindende Mittel sind.

Die Jodierung erfolgt zweckmässigerweise unter Verwendung von elementarem Jod als Jodierungsmittel und von einer nieder siedenden aliphatischen Carbonsäure, wie Essigsäure, als Lösungsmittel und bei Temperaturen zwischen ca. 0°C und ca. 110°C, vorzugsweise bei Raumtemperatur. Zudem erweist es sich als zweckmässig, die Umsetzung in Gegenwart einer Säure, wie rauchender Salpetersäure, durchzuführen. Zur Beseitigung von überschüssigem Jod kann nach Beendung der Reaktion gesättigte wässrige Natriumhydrogensulfitlösung zugefügt werden.

Bei der Verfahrensvariante b) handelt es sich je nach Natur des Restes $R^2$ um eine Aetherbildung, eine Acylierung bzw. eine Sulfonylierung, die in an sich bekannter Weise durchgeführt werden kann. Nachträgliche Reaktionsstufen und/oder andersartige Substitutions-oder Additionsreaktionen an der Gruppe $R^2$ sind allerdings beim Ersatz der Hydroxygruppe durch den Substituenten $R^2$ nicht aus dieser Verfahrensvariante ausgeschlossen.

Als typisches Beispiel einer Aetherbildung wird eine Verbindung der Formel III in eine Verbindung der Formel I, in der $R^2$ $C_{3-6}$-Alkinoxy, z.B. Propargyloxy, bedeutet, übergeführt. Dies wird zweckmässigerweise durchgeführt, indem man die Verbindung III in einem inerten aprotischen, polaren Lösungsmittel, wie beispielsweise Dimethylformamid, Dimethylsulfoxid oder Acetonitril, mit einer Base, z.B. Natriumhydrid oder Kaliumcarbonat, behandelt und das resultierende Salz mit einem $C_{3-6}$ -Alkinhalogenid, insbesondere dem Chlorid oder Bromid, bei Temperaturen zwischen der Raumtemperatur und ca. 80°C umsetzt, und zwar geeigneterweise in demselben Lösungsmittel. Vorzugsweise wird in Dimethylformamid in Gegenwart von Natriumhydrid bei Raumtemperatur gerarbeitet.

Als typisches Beispiel für die Ueberführung einer Verbindung der Formel III in eine Verbindung der Formel I, in der $R^2$ einen eine (Thio)Carbonyloxygruppe enthaltenden Rest bedeutet, dient die Herstellung solcher Verbindungen I, in denen $R^2$ $C_{2-8}$-Alkoxycarbonyloxy, z.B. Methoxycarbonyloxy, bedeutet. Die Umsetzung wird zweckmässigerweise durchgeführt, indem man die Verbindung III in einem inerten aprotischen Lösungsmittel, wie Dimethylformamid, Dimethylsulfoxid, Acetonitril, einem aliphatischen Aether, z.B. 1,2-Dimethoxyäthan, oder einem chlorierten aliphatischen Kohlenwasserstoff, z.B. Methylenchlorid, mit einer Base, wie beispielsweise Natriumhydrid, Triäthylamin oder Pyridin, versetzt und anschliessend mit einem $C_{2-8}$-Alkoxycarbonylhalogenid in demselben Lösungsmittel bei Temperaturen zwischen der Raumtemperatur und ca. 80°C behandelt. Vorzugsweise wird in Methylenchlorid in Gegenwart von Triäthylamin oder Pyridin bei Raumtemperatur gearbeitet und anschliessend Chlorameisensäure-methylester auch bei Raumtemperatur eingesetzt.

Zur Veranschaulichung der Ueberführung einer Verbindung der Formel III in eine Verbindung der Formel I, in der $R^2$ einen eine Sulfonyloxygruppe enthaltenden Rest bedeutet, dient die Herstellung solcher Verbindungen I, in denen $R^2$ N-monosubstituiertes oder N,N-disubstituiertes Sulfamoyloxy, z.B. N,N-Dimethylsulfamoyloxy, bedeutet. Die Umsetzung erfolgt zweckmässigerweise unter etwa gleichen Reaktionsbedingungen wie für die Umsetzung mit einem $C_{2-8}$-Alkoxycarbonylhalogenid oben beschrieben.

Dieses Verfahren eignet sich insbesondere zur Herstellung der erfindungsgemässen Verbindungen der Formel I, in denen $R^1$ einen säurestabilen Rest bedeutet. Dies sind insbesondere Alkyl und Halogenalkyl.

Ausgehend von denjenigen Produkten der Verfahrensvariante b), in denen $R^5$ Wasserstoff bedeutet, können gewünschtenfalls die entsprechenden 5-Chlor-, 5-Brom-oder 5-Joduracile ($R^5$= Chlor, Brom bzw. Jod) durch Halogenierung hergestellt werden, und zwar wie oben näher beschrieben.

Wie oben gesagt, kann man in einer nachträglichen fakultativen Reaktionsstufe eine Verbindung der Formel I in eine Verbindung I, die sich von deren Vorläufer durch den Substituenten $R^2$ unterscheidet, überführen. Beispielsweise wird in einer Verbindung der allgemeinen Formel

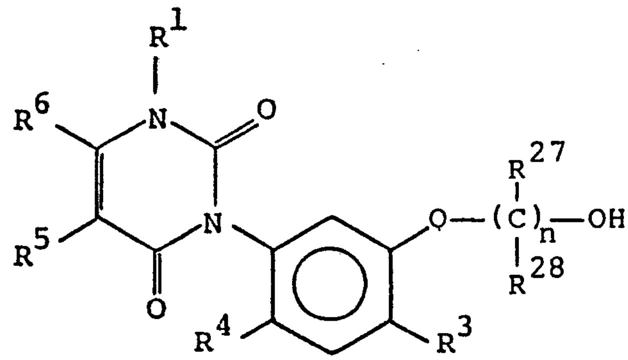

I"

worin $R^1$, $R^3$, $R^4$, $R^5$ und $R^6$ die oben angegebenen Bedeutungen besitzen und (jedes) $R^{27}$ und (jedes) $R^{28}$ unabhängig voneinander Wasserstoff oder nieder Alkyl, insbesondere $C_{1-4}$-Alkyl, und n 2-5 bedeuten, die Hydroxygruppe in an sich bekannter Weise durch einen anderen Substituenten ersetzt, z.B. durch Veresterung. Als typisches Beispiel einer solchen Veresterung wird eine Verbindung der Formel I", z.B. eine, worin $-O(CR^{27}R^{28})_n-OH$ für 2-Hydroxyäthyl steht, acyliert, z.B. mit Acetylchlorid. Die Acylierung erfolgt zweckmässigerweise in einem inerten aprotischen Verdünnungsmittel, wie einem aliphatischen oder cyclischen Aether, z.B. Diäthyläther, 1,2-Dimethoxyäthan oder Tetrahydrofuran, oder einem chlorierten aliphatischen Kohlenwasserstoff, z.B. Methylenchlorid, unter Verwendung einer Base, z.B. Triäthylamin oder Pyridin, bei Temperaturen zwischen 0°C und 50°C, vorzugsweise bei Raumtemperatur. Die Veresterung kann auch beispielsweise durch Behandlung einer Verbindung der Formel I" mit einem Isocyanat durchgeführt werden, wobei der entsprechende Carbaminsäureester hergestellt wird. Zur Veranschaulichung dieses Verfahrens dient die Herstellung des Methylcarbaminsäureesters: man kann die Verbindung der Formel I" mit Methylisocyanat unter ähnlichen Reaktionsbedingungen wie die oben im Zusammenhang mit der Acylierung der Verbindung I" beschriebenen, behandeln, wobei im Falle der Umsetzung mit dem Isocyanat bloss eine katalytische Menge Base verwendet wird.

Als weiteres Beispiel dient die Oxidation einer Verbindung der allgemeinen Formel

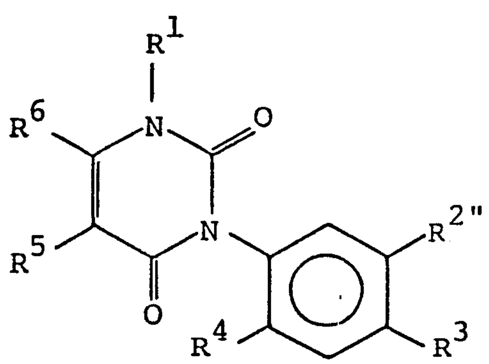

I'''

worin $R^1$, $R^3$, $R^4$, $R^5$ und $R^6$ die oben angegebenen Bedeutungen besitzen und $R^{2''}$ $C_{2-8}$-Alkylthioalkoxy oder gegebenenfalls substituiertes Phenylthio-$C_{1-4}$-alkoxy bedeutet, zur entsprechenden Sulfoxid-oder Sulfonverbindung, d.h. $R^2$ der Formel I bedeutet $C_{2-8}$-Alkylsulfinylalkoxy oder $C_{2-8}$-Alkylsulfonylalkoxy bzw. gegebenenfalls substituiertes Phenylsulfinyl-oder Phenylsulfonyl-$C_{1-4}$-alkoxy. Bei diesem Verfahren handelt es sich je nach Natur und Menge des verwendeten Oxidationsmittels sowie den sonstigen angewendeten Reaktionsbedingungen um eine gewöhnliche Oxidation des Sulfids I''' zum entsprechenden Sulfoxid oder aber Sulfon. Dies wird beispielsweise im Falle einer Verbindung der Formel I''', in der $R^{2''}$ $C_{2-8}$-Alkylthioalkoxy bedeutet, so durchgeführt, dass man die Verbindung I''' in einem inerten, aprotischen Verdünnungsmittel, wie einem aliphatischen chlorierten Kohlenwasserstoff, z.B. Methylenchlorid, Tetrachlormethan oder 1,2-Dichloräthan, durch Behandlung mit einer Persäure, z.B. 3-Chlorperbenzoesäure, bei Temperaturen zwischen -10°C und 30°C, vorzugsweise bei 0°C, zum Sulfon führt.

Zur weiteren Veranschaulichung der oben erwähnten nachträglichen Reaktionsstufe wird in einer Verbindung der allgemeinen Formel

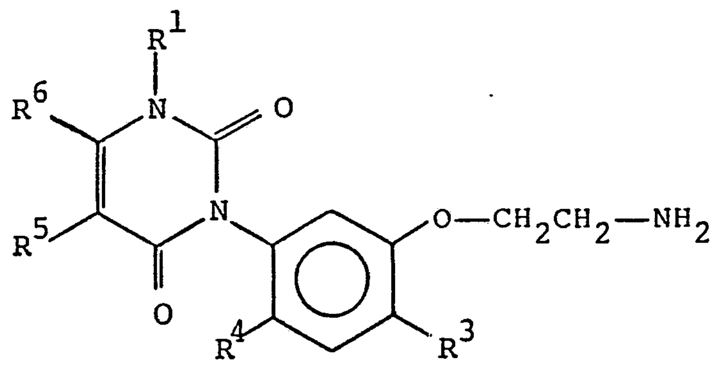

I''''

worin $R^1$, $R^3$, $R^4$, $R^5$ und $R^6$ die oben angegebenen Bedeutungen besitzen, die Aminogruppe in an sich bekannter Weise in eine mono-oder disubstituierte Aminogruppe übergeführt. Es kann sich dabei unter anderem um eine Alkylierung, Amidbildung oder Carbamidsäureester-Bildung handeln. Zwecks Alkylierung wird die Verbindung I'''' oder ein Salz davon beispielsweise mit einem Gemisch aus 36%igen Formaldehyd und Ameisensäure bei Temperaturen zwischen 50°C und der Rückflusstemperatur des Reaktionsgemisches, vorzugsweise bei ca. 100°C, behandelt. Eine Formylierung der Verbindung I'''' oder eines Salzes davon erfolgt üblicherweise unter Verwendung von Ameisensäure als Formylierungsmittel bei der Rückflusstemperatur des Reaktionsgemisches, während man eine Acylierung der Verbindung I'''' im allgemeinen in einem inerten aprotischen Verdünnungsmittel, wie einem aliphatischen chlorierten Kohlenwasserstoff, z.B. Methylenchlorid, oder einem aliphatischen oder cyclischen Aether, z.B. Diäthyläther, 1,2-Dimethoxyäthan oder Tetrahydrofuran, in Gegenwart einer Base, z.B. Triäthylamin oder Pyridin, bei Temperaturen zwischen -10°C und 50°C, vorzugsweise bei Raumtemperatur, vornimmt. Typische Acylierungsmittel sind die diesbezüglichen Säurechloride, z.B. Acetylchlorid, und Säureanhydride, z.B. Acetanhydrid.

Weitere nachträgliche Reaktionsstufen, die ausgehend von gewissen Verbindungen der Formel I zu anderen Verbindungen der Formel I führen, kommen in Frage.

Sofern sie nicht unmittelbar durch die oben beschriebene unter basischen Bedingungen durchgeführte Cyclisierung nach der Verfahrensvariante a) hergestellt werden, können die gewünschten Salze der Verbindungen der Formel I, in denen $R^1$ Wasserstoff bedeutet, auch aus diesen Verbindungen I in an sich bekannter Weise hergestellt werden, wie beispielsweise durch Auflösen der Verbindung der Formel I in einer Lösung einer diesbezüglichen anorganischen oder organischen Base. Die Salzbildung erfolgt in der Regel innert kurzer Zeit bei Raumtemperatur. In einer Ausführungsform wird das Natriumsalz durch Auflösen des Uracilderivats I in wässriger Natriumhydroxidlösung bei Raumtemperatur hergestellt, wobei äquivalente Mengen des Uracilderivats und des Natriumhydroxids verwendet werden. Das feste Salz kann dann durch Fällen mit einem geeigneten inerten Lösungsmittel oder durch Abdampfen des Lösungsmittels isoliert werden. Eine weitere Ausführungsform besteht darin, eine wässrige Lösung eines Alkalimetallsalzes des Uracilderivats I in eine wässrige Lösung eines Salzes, das ein anderes Metallkation als ein Alkalimetallkation aufweist, einzuführen, wobei das zweite Metallsalz des Uracilderivats hergestellt wird. Diese Ausführungsform dient im allgemeinen zur Herstellung von Uracil-Metallsalzen, die in Wasser unlöslich sind.

Die erhaltenen Verbindungen der Formel I sowie deren Salze können nach an sich bekannten Methoden isoliert und gereinigt werden.

Sofern keine gezielte Synthese zur Isolierung reiner Isomerer durchgeführt wird, kann das Produkt als Gemisch zweier oder mehrerer Isomerer anfallen. Die Isomeren können nach an sich bekannten Methoden aufgetrennt werden. Gewünschtenfalls können beispielsweise reine optisch aktive Isomere auch durch Synthese aus entsprechenden optisch aktiven Ausgangsmaterialien hergestellt werden.

Die Ausgangsmaterialien der Formel II, die neu sind, können in an sich bekannter Weise hergestellt werden, z.B. gemäss den nachfolgenden Reaktionsschemata [Methoden aa), bb), cc), und dd)]:

## Reaktionsschemata

aa)

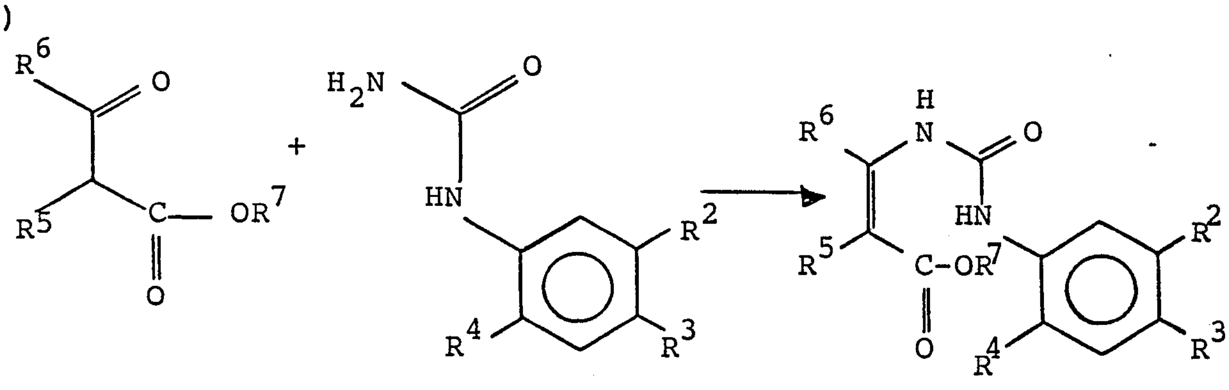

IV V II

bb)

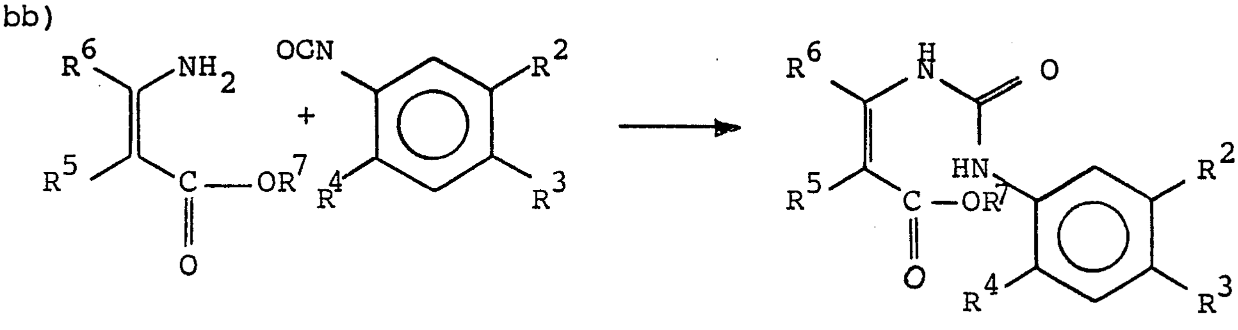

VI VII II

cc)

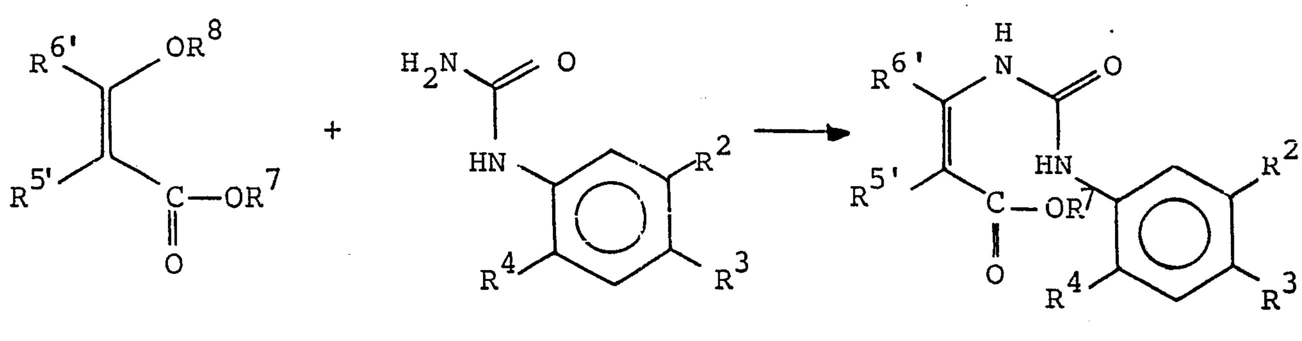

VIII V IIa

Reaktionsschemata (Fortsetzung)

dd)

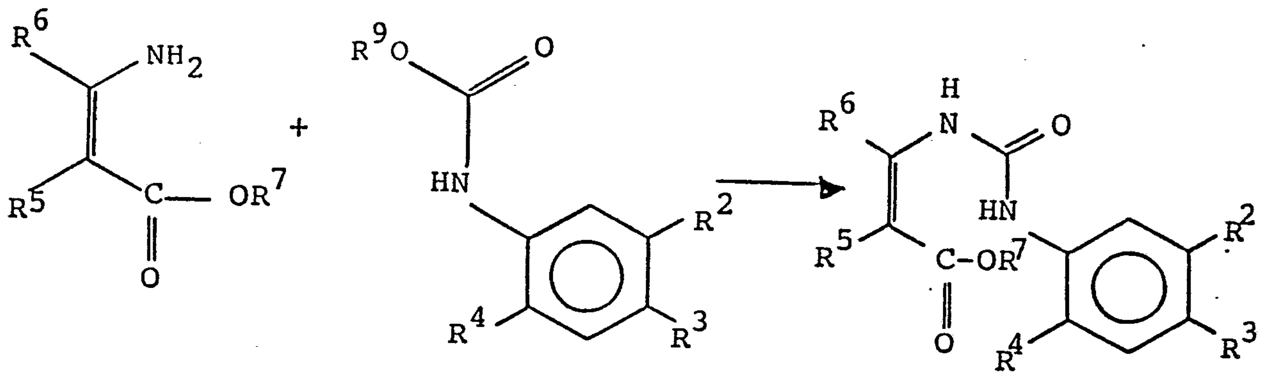

VI IX II

In den obigen Reaktionsschemata besitzen $R^2$, $R^3$, $R^4$, $R^5$, $R^6$ und $R^7$ die oben angegebenen Bedeutungen, $R^{5'}$ bedeutet Wasserstoff oder $C_{1-4}$ -Alkyl; $R^{6'}$ bedeutet $C_{1-4}$-Alkyl; und $R^8$ und $R^9$ bedeuten nieder Alkyl, vorzugsweise $C_{1-4}$-Alkyl.

Die Methode aa) wird zweckmässigerweise so durchgeführt, dass man die Verbindungen der Formeln IV und V in einem im wesentlichen wasserfreien Verdünnungsmittel und in Gegenwart eines sauren Katalysators bei erhöhter Temperatur miteinander reagieren lässt. Als Verdünnungsmittel kommen insbesondere mit Wasser azeotrope organische Lösungsmittel, wie Aromate, z.B. Benzol, Toluol und Xylole; halogenierte Kohlenwasserstoffe, wie Methylenchlorid, Chloroform, Tetrachlorkohlenstoff und Chlorbenzol; aliphatische und cyclische Aether, wie 1,2-Dimethoxyäthan, Tetrahydrofuran und Dioxan; und Cyclohexan, und als saure Katalysatoren insbesondere starke Mineralsäuren, wie Schwefelsäure und Salzsäure; organische Säuren, wie p-Toluolsulfonsäure; Phosphor enthaltende Säuren, wie Orthophosphorsäure und Polyphosphorsäure; und saure Kationenaustauscher, wie "Amberlyst 15" (Fluka), in Frage. Man arbeitet im allgemeinen in einem Temperaturbereich von etwa 70°C bis 120°C, vorzugsweise bei der Rückflusstemperatur des Reaktionsgemisches. Unter diesen Reaktionsbedingungen wird die erwünschte rasche Entfernung des in der Reaktion gebildeten Wassers erzielt.

Die Umsetzung nach der Methode bb) erfolgt zweckmässigerweise in Gegenwart eines im wesentlichen wasserfreien aprotischen organischen Lösungsmittels, wie eines aliphatischen oder cyclischen Aethers, z.B. Diäthyläther, 1,2-Dimethoxyäthan, Tetrahydrofuran oder Dioxan, eines aliphatischen oder aromatischen Kohlenwasserstoffs, z.B. n-Hexan, Benzol, Toluol oder eines Xylols, oder eines halogenierten, aliphatischen Kohlenwasserstoffs, z.B. Methylenchlorid, Chloroform, Tetrachlorkohlenstoff oder 1,2-Dichloräthan; eines aprotischen, polaren Lösungsmittels, wie Dimethylformamid, Hexamethylphosphorsäuretriamid oder Dimethylsulfoxid; oder eines Gemisches zweier oder mehrerer der erwähnten Lösungsmittel, sowie gegebenenfalls in Gegenwart einer organischen tertiären Base, wie Triäthylamin oder Pyridin, wobei letzteres sowohl als Lösungsmittel wie auch als Base dienen kann, oder einer Metallhydridbase, wie Natrium-oder Kaliumhydrid. Die Reaktionstemperaturen sind vorzugsweise im Bereich von etwa -70°C bis 50°C, wobei besonders bevorzugt bei Temperaturen zwischen -30°C und der Raumtemperatur gearbeitet wird.

Die Umsetzung nach der Methode cc) wird zweckmässigerweise in einem inerten, mit Wasser mischbaren, organischen Lösungsmittel, wie einem aliphatischen oder cyclischen Aether, z.B. 1,2-Dimethoxyäthan, Tetrahydrofuran oder Dioxan, oder einem niederen Alkanol, wie Aethanol, bei Temperaturen zwischen 50°C und 100°C, vorzugsweise bei der Rückflusstemperatur des Reaktionsgemisches, oder in einem aromatischen Lösungsmittel, wie Benzol, Toluol oder einem Xylol in Gegenwart eines sauren Katalysators, wie Salzsäure oder p-Toluolsulfonsäure, bei Temperaturen zwischen 50°C und 100°C, vorzugsweise 60°C bis 80°C, durchgeführt.

Die Umsetzung nach der Methode dd) erfolgt zweckmässigerweise in einem aprotischen polaren Verdünnungsmittel, wie Dimethylformamid, 2-Butanon, Dimethylsulfoxid oder Acetonitril, in Gegenwart einer Base, wie eines Alkalimetall-oder Erdalkalimetallalkoholats oder -carbonats, insbesondere Natriumalkanolat oder -carbonat, oder eines Metallhydrids, insbesondere Lithium oder Natriumhydrid, bei Temperaturen zwischen 80°C und 180°C, vorzugsweise bei der Rückflusstemperatur des Reaktionsgemisches. Falls ein Alkoholat als Base verwendet wird, destilliert man zweckmässigerweise den im Laufe der Reaktion freigesetzten Alkohol kontinuierlich ab. Bei dieser Methode dd) wird die so hergestellte Verbindung der Formel II in der Regel in situ hergestellt, da die verwendeten Reaktionsbedingungen die Cyclisierung der Verbindung II zu einem Salz der oben angegebenen Formel I' begünstigen, und zwar nach der Verfahrensvariante a).

Die in der Verfahrensvariante b) benötigten Ausgangsmaterialien der Formel III sind ebenfalls neu und können durch säurekatalysierte Hydrolyse der entsprechenden geschützten Phenole der Formel

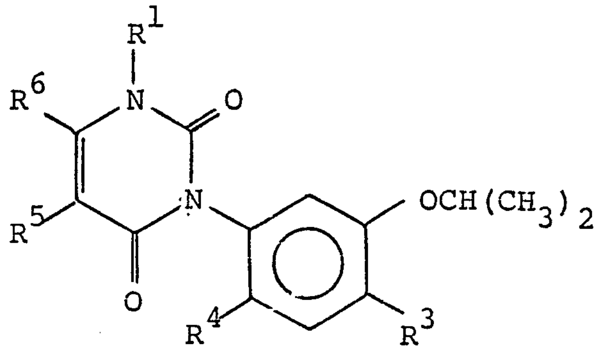

I'''''

worin $R^1$, $R^3$, $R^4$, $R^5$ und $R^6$ die oben angegebenen Bedeutungen besitzen,
hergestellt werden. Die Hydrolyse erfolgt zweckmässigerweise in Gegenwart von Schwefelsäure als saurem Katalysator, in einem chlorierten aliphatischen Kohlenwasserstoff, vorzugsweise Methylenchlorid, als Lösungsmittel und bei Temperaturen zwischen -30°C und 30°C, vorzugsweise bei Raumtemperatur. Ohne zusätzliches Lösungsmittel kann überschüssige Schwefelsäure selbst als Lösungsmittel dienen.

Die Hydrolyse eignet sich insbesondere zur Herstellung derjenigen Verbindungen der Formel III, in denen $R^1$ verschieden von Wasserstoff ist und zudem einen säurestabilen Rest bedeutet. Unter den oben aufgeführten Resten $R^1$ gelten insbesondere $C_{1-4}$-Alkyl und $C_{1-4}$-Halogenalkyl als säurestabil.

Diejenigen Ausgangsmaterialien der Formel III, in denen $R^1$ $C_{1-4}$-Alkyl oder $C_{1-4}$-Halogenalkyl bedeutet, können auch noch gemäss dem nachfolgenden Reaktionsschema hergestellt werden:

## Reaktionsschema

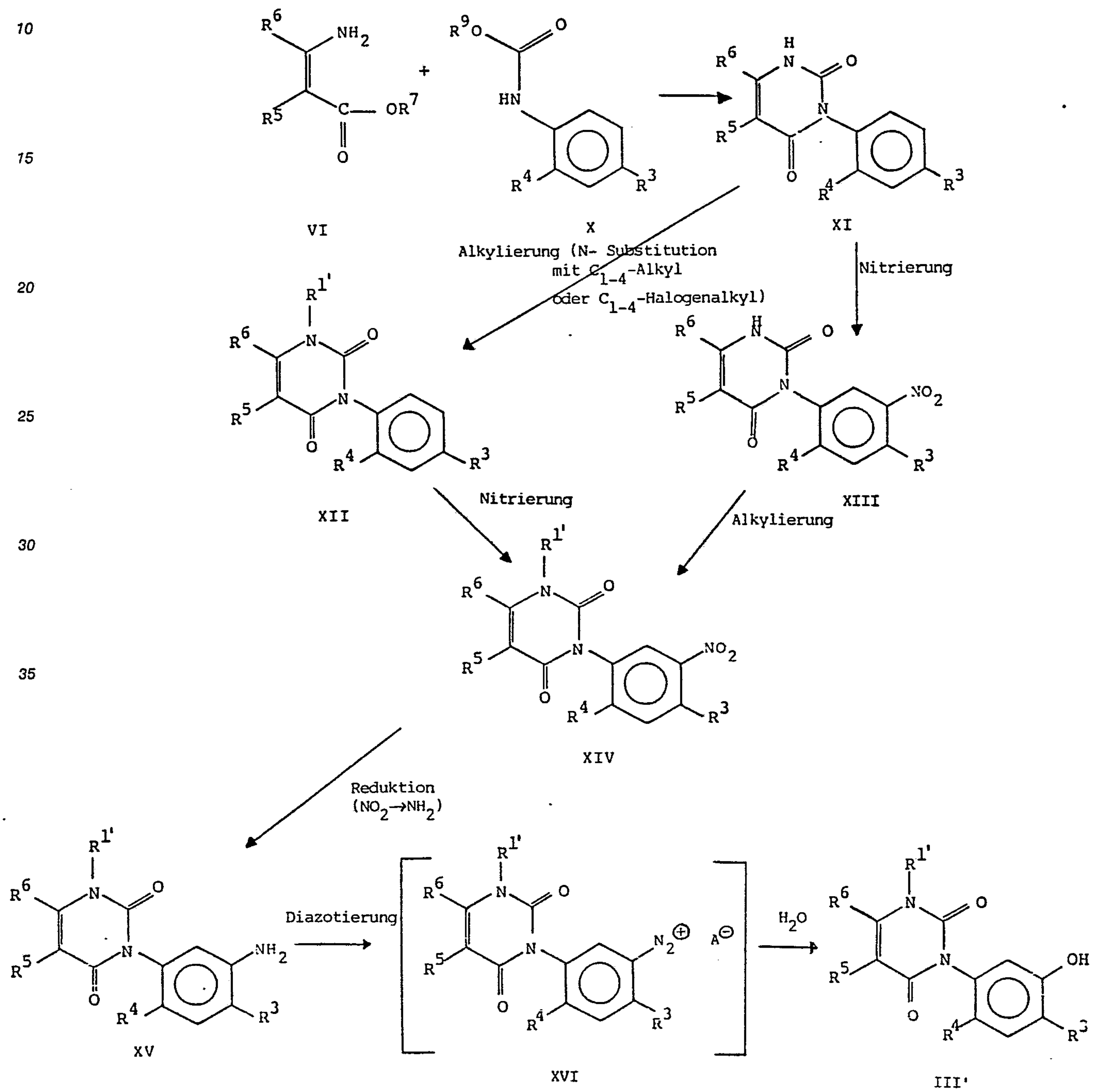


VI X XI XII XIII XIV XV XVI III'

In dem obigen Reaktionsschema besitzen $R^3$, $R^4$, $R^5$, $R^6$, $R^7$ und $R^9$ die oben angegebenen Bedeutungen; $R^{1'}$ bedeutet $C_{1-4}$-Alkyl oder $C_{1-4}$-Halogenalkyl und $A^{\ominus}$ein Anion, z.B. Halogenid, Hydrogensulfat oder Tetrafluorborat.

Die Umsetzung der Verbindungen der Formeln VI und X und die Alkylierung erfolgt zweckmässigerweise unter den oben im Zusammenhang mit der Methode dd) bzw. Verfahrensvariante a) beschriebenen Reaktionsbedingungen, während die Nitrierung, die Reduktion, die Diazotierung und die Hydrolyse nach an sich bekannten Methoden dieser am Benzolring geschehenden Reaktionen durchgeführt werden können (siehe beispielsweise Houben-Weyl, Methoden der organischen Chemie, Bd 6/1c, Seiten 247-265 für die Diazotierung und darauffolgende Hydrolyse).

Die Zwischenprodukte der Formeln XI, XII, XIII, XIV, XV und XVI sind neu.

Bei den obigen Ausgangsmaterialien der Formel I'''' handelt es sich um eine Untergruppe von Verbindungen der Formel I. Die restlichen in den Verfahrensvarianten a) und b), in den Reaktionsschemata [Methoden aa)-dd)] sowie in der ersten Stufe des nachträglich erscheinenden Reaktionsschema (Ausgangsmaterialien VI und X) involvierten Ausgangsmaterialien bzw. Reagenzien sind entweder bekannt oder können nach an sich bekannten Methoden hergestellt werden.

Die erfindungsgemässen Verbindungen der Formel I (einschliesslich der Salze) besitzen herbizide Eigenschaften und eignen sich zur Bekämpfung von Unkräutern, einschliesslich Ungräsern, insbesondere Setaria faberii, Digitaria sanguinalis, Poa annua, Chenopodium album, Amaranthus retroflexus, Abutilon theopharasti, Sinapsis alba und Datura stramonium, in diversen Nutzpflanzenkulturen, insbesondere in Baumwolle-und Soyakulturen. Zudem sind die Verbindungen sowohl Vorauflauf-als auch Nachauflauf-Herbizide.

Auch gewisse Verbindungen der Formeln III und XI-XVI besitzen herbizide Eigenschaften und können auf ähnliche Weise wie die Verbindungen I zur Bekämpfung von Ungräsern und Unkräutern, insbesondere den obenerwähnten, eingesetzt werden. Diese neuen Verbindungen bilden einen weiteren Gegenstand der vorliegenden Erfindung. Aufgrund ihrer besonders ausgeprägten herbiziden Aktivität stellt 3-(4-Chlor-2-fluor-5-hydroxyphenyl)-1-methyl-6-trifluormethyl-2,4-(1H,3H)-pyrimidindion eine bevorzugte Verbindung der Formel III dar.

Ueblicherweise genügt eine Konzentration von 0,005-6,0 kg Wirkstoff der Formel I bzw. III, XI, XII, XIII, XIV, XV oder XVI/ha, vorzugsweise 0,005-2,0 kg Wirkstoff der Formel I bzw. III, XI, XII, XIII, XIV, XV oder XVI/ha, um den gewünschten herbiziden Effekt zu erzielen, wobei im allgemeinen die Verbindungen der Formel I bedeutend wirksamer sind als die herbizid aktiven Verbindungen der Formel III bzw. XI-XVI. Besonders bevorzugt ist die Konzentrationsreihe 0,01-1,5 kg Wirkstoff der Formel I bzw. III, XI-XVI/ha.

Das erfindungsgemässe Unkrautbekämpfungsmittel ist dadurch gekennzeichnet, dass es eine wirksame Menge mindestens einer Verbindung der Formel I (einschliesslich der Salze) bzw. III, XI, XII, XIII, XIV, XV oder XVI, wie oben definiert, sowie Formulierungshilfsstoffe enthält. Das Mittel enthält zweckmässigerweise zumindest einen der folgenden Formulierungshilfsstoffe: feste Trägerstoffe; Lösungs-bzw. Dispersionsmittel; Tenside (Netz-und Emulgiermittel); Dispergatoren (ohne Tensidwirkung); und Stabilisatoren. Unter Verwendung solcher und anderer Hilfsstoffe können diese Verbindungen, also die herbiziden Wirkstoffe, in die üblichen Formulierungen übergeführt werden, wie Stäube, Pulver, Granulate, Lösungen, Emulsionen, Suspensionen, emulgierbare Konzentrate, Pasten und dergleichen.

Die Verbindungen der Formel I bzw. III, XI, XII, XIII, XIV, XV oder XVI (im folgenden lediglich als Wirkstoff(e) bezeichnet) sind im allgemeinen wasserunlöslich und können nach den für wasserunlösliche Verbindungen üblichen Methoden unter Verwendung der diesbezüglichen Formulierungshilfsstoffe konfektioniert werden. Die Herstellung der Mittel kann in an sich bekannter Weise durchgeführt werden, z.B. durch Vermischen des jeweiligen Wirkstoffes mit festen Trägerstoffen, durch Auflösen oder Suspendieren in geeigneten Lösungs-bzw. Dispersionsmitteln, eventuell unter Verwendung von Tensiden als Netz-oder Emulgiermitteln und/oder von Dispergatoren, durch Verdünnen bereits vorbereiteter emulgierbarer Konzentrate mit Lösungs-bzw. Dispersionsmitteln usw.

Als feste Trägerstoffe kommen im wesentlichen in Frage: natürliche Mineralstoffe, wie Kreide, Dolomit, Kalkstein, Tonerden und Kieselsäure und deren Salze (beispielsweise Kieselgur, Kaolin, Bentonit, Talkum, Attapulgit und Montmorrillonit); synthetische Mineralstoffe, wie hochdisperse Kieselsäure, Aluminiumoxid und Silikate; organische Stoffe, wie Cellulose, Stärke, Harnstoff und Kunstharze; und Düngemittel, wie Phosphate und Nitrate, wobei solche Trägerstoffe z.B. als Pulver oder als Granulate vorliegen können.

Als Lösungs-bzw. Dispersionsmittel kommen im wesentlichen in Frage: Aromaten, wie Benzol, Toluol, Xylole und Alkylnaphthaline; chlorierte Aromaten und chlorierte aliphatische Kohlenwasserstoffe, wie Chlorbenzole, Chloräthylene und Methylenchlorid; aliphatische Kohlenwasserstoffe, wie Cyclohexan und Paraffine, z.B. Erdölfraktionen; Alkohole, wie Butanol und Glykol, sowie deren Aether und Ester; Ketone, wie Aceton, Methyläthylketon, Methylisobutylketon und Cyclohexanon; und stark polare Lösungs-bzw. Dispersionsmittel, wie Dimethylformamid, N-Methylpyrrolidon und Dimethylsulfoxid, wobei solche Lösungsmittel vorzugsweise Flammpunkte von mindestens 30°C und Siedepunkte von mindestens 50°C aufweisen, und Wasser. Unter den Lösungs-bzw. Dispersionsmitteln kommen auch in Frage sogenannte verflüssigte

gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase, wie Halogenkohlenwasserstoffe, z.B. Dichlordifluormethan. Liegt das erfindungsgemässe Unkrautbekämpfungsmittel in Form einer Drucksgaspackung vor, so wird zweckmässigerweise zusätzlich zum Treibgas ein Lösungsmittel verwendet.

Die Tenside (Netz-und Emulgiermittel) können nicht-ionische Verbindungen sein, wie Kondensationsprodukte von Fettsäuren, Fettalkoholen oder fettsubstituierten Phenolen mit Aethylenoxid; Fettsäureester und -äther von Zuckern oder mehrwertigen Alkoholen; die Produkte, die aus Zuckern oder mehrwertigen Alkoholen durch Kondensation mit Aethylenoxid erhalten werden; Blockpolymere von Aethylenoxid und Propylenoxid; oder Alkyldimethylaminoxide.

Die Tenside können auch anionische Verbindungen sein, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate, z.B. Calcium-dodecylbenzolsulfonat, und Butylnaphthalinsulfonate; und komplexere Fettsulfonate, z.B. die Amidkondensationsprodukte von Oelsäure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und äthoxylierte quaternäre Ammoniumchloride.

Als Dispergatoren (ohne Tensidwirkung) kommen im wesentlichen in Frage: Lignin, Natrium-und Ammoniumsalze von Ligninsulfonsäuren, Natriumsalze von Maleinsäureanhydrid-Diisobutylen-Copolymeren, Natrium-und Ammoniumsalze von sulfonierten Polykondensationsprodukten aus Naphthalin und Formaldehyd, und Sulfitablaugen.

Als Dispergatoren, die sich insbesondere als Verdickungs-bzw. Antiabsetzmittel eignen, können z.B. Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel, z.B. Epichlorhydrin, Phenylglycidäther und Soyaepoxide; Antioxidantien, z.B. Gallussäureester und Butylhydroxytoluol; UV-Absorber, z.B. substituierte Benzophenone, Diphenylacrylonitrilsäureester und Zimtsäureester; und Deaktivatoren, z.B. Salze der Aethylendiaminotetraessigsäure und Polyglykole.

Die erfindungsgemässen Unkrautbekämpfungsmittel können zusätzlich zu den erfindungsgemässen Wirkstoffen Synergisten und andere Wirkstoffe, z.B. Insektizide, Akarizide, Fungizide, Pflanzenwachstumsregulatoren und Düngemittel, enthalten. Solche Kombinationsmittel eignen sich zur Verstärkung der Aktivität bzw. zur Verbreiterung des Wirkungsspektrums.

Die erfindungsgemässen Unkrautbekämpfungsmittel enthalten im allgemeinen zwischen 0.01 und 95 Gewichtsprozent, vorzugsweise zwischen 0.5 und 75 Gewichtsprozent einer bzw. mehrerer erfindungsgemässer Wirkstoffe. Sie können z.B. in einer Form vorliegen, die sich für die Lagerung und den Transport eignet. In solchen Formulierungen, z.B. emulgierbaren Konzentraten, ist die Wirkstoffkonzentration normalerweise im höheren Bereich, vorzugsweise zwischen 1 und 50 Gewichtsprozent, insbesondere zwischen 10 und 20 Gewichts prozent. Diese Formulierungen können dann, z.B. mit gleichen oder verschiedenen inerten Stoffen, bis zu Wirkstoffkonzentrationen verdünnt werden, die sich für den praktischen Gebrauch eignen, also vorzugsweise ca. 0.01 bis 10 Gewichtsprozent, insbesondere ca. 0.5 bis 5 Gewichtsprozent. Die Wirkstoffkonzentrationen können jedoch auch kleiner oder grösser sein.

Wie oben erwähnt, kann die Herstellung der erfindungsgemässen Unkrautbekämpfungsmittel in an sich bekannter Weise durchgeführt werden.

Zur Herstellung pulverförmiger Präparate kann der Wirkstoff, d.h. mindestens eine Verbindung der Formel I, III, XI, XII, XIII, XIV, XV oder XVI, mit festem Trägerstoff vermischt werden, z.B. durch Zusammenmahlen; oder man kann den festen Trägerstoff mit einer Lösung oder Suspension des Wirkstoffes imprägnieren und dann das Lösungs-bzw. Dispersionsmittel durch Abdunsten, Erhitzen oder Absaugen unter vermindertem Druck entfernen. Durch Zusatz von Tensiden bzw. Dispergatoren kann man solche pulverförmige Mittel mit Wasser leicht benetzbar machen, so dass sie in wässrige Suspensionen, die sich z.B. als Spritzmittel eignen, übergeführt werden können.

Der Wirkstoff kann auch mit einem Tensid und einem festen Trägerstoff zur Bildung eines netzbaren Pulvers vermischt werden, welches in Wasser dispergierbar ist, oder sie kann mit einem festen vorgranulierten Trägerstoff zur Bildung eines granulatförmigen Produktes vermischt werden.

Wenn gewünscht, kann der Wirkstoff in einem mit Wasser nicht mischbaren Lösungsmittel, wie beispielsweise einem hochsiedenden Kohlenwasserstoff, gelöst werden, das zweckmässigerweise gelöste Emulgiermittel enthält, so dass die Lösung bei Zugabe zu Wasser selbstemulgierend wirkt. Andernfalls kann der Wirkstoff mit einem Emulgiermittel vermischt und das Gemisch dann mit Wasser auf die gewünschte Konzentration verdünnt werden. Zudem kann der Wirkstoff in einem Lösungsmittel gelöst und danach mit einem Emulgiermittel gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Die Verwendung der erfindungsgemässen Unkrautbekämpfungsmittel, die einen weiteren Gegenstand der vorliegenden Erfindung bildet, kann nach üblichen Applikationsmethoden, wie Spritzen, Sprühen, Stäuben, Giessen oder Streuen, erfolgen. Das erfindungsgemässe Verfahren zur Bekämpfung von Unkräutern ist dadurch gekennzeichnet, dass man das gegen Unkräuter zu schützende Gut und/oder die Unkräuter mit einem erfindungsgemässen Wirkstoff bzw. mit einem erfindungsgemässen Unkrautbekämpfungsmittel behandelt.

Die nachfolgenden Beispiele dienen zur näheren Erläuterung der Erfindung.

I. Herstellung der Verbindungen der Formel I:

Beispiel 1

Eine Lösung von 1.0 g 3-Amino-4,4,4-trifluorcrotonsäure-äthylester in 10 ml absolutem Toluol wird unter Rühren bei 0°C während 15 Minuten zu einer Suspension von 0.24 g einer 55%igen Natriumhydrid-Dispersion in 20 ml absolutem Dimethylformamid zugetropft, und das Gemisch wird 15 Minuten bei 0°C nachgerührt. Anschliessend wird das Reaktionsgemisch auf -30°C abgekühlt und mit einer Lösung von 1.26 g 4-Chlor-2-fluor-5-isopropoxyphenylisocyanat in 10 ml absolutem Toluol versetzt. Die Temperatur steigt rasch auf -10°C an, und das Reaktiosgemisch wird danach 2 Stunden bei Raumtemperatur nachgerührt. Das Reaktionsgemisch wird auf eine Lösung von 3 ml 2N Salzsäure und 500 ml Wasser gegossen und das wässrige Gemisch zweimal mit je 100 ml Essigsäure-äthylester extrahiert, und die organischen Phasen werden zweimal mit je 50 ml Wasser gewaschen, über wasserfreiem Natriumsulfat getrocknet und unter vermindertem Druck zur Trockene eingedampft. Der Rückstand wird aus Diäthyläther/n-Hexan umkristallisiert. Ueber den 3-[3-(4-Chlor-2-fluor-5-isopropoxyphenyl)ureido]-4,4,4-trifluorcrotonsäure-äthylester (Smp. 144-146°C), welcher nicht isoliert wird, erhält man also das 3-(4-Chlor-2-fluor-5-isopropoxyphenyl)-6-trifluormethyl -2,4(1H, 3H)-pyrimidindion, Smp. 195-197°C.

In analoger Weise erhält man beim Einsatz von 3-Amino--4,4,4-trifluorcrotonsäure-äthylester und:

- 4-Chlor-2-fluor-5-methoxyphenylisocyanat das 3-(4-Chlor-2-fluor-5-methoxyphenyl)-6-trifluormethyl-2,4-(1H,3H)-pyrimidindion, Smp. 172-174°C;
- 4-Chlor-2-fluor-5-propoxyphenylisocyanat das 3-(4-Chlor-2-fluor-5-propoxyphenyl)-6-trifluormethyl-2,4-(1H,3H)-pyrimidindion, Smp. 164-166°C,
- 5-Butoxy-4-chlor-2-fluorphenylisocyanat das 3-(5-Butoxy-4-chlor-2-fluorphenyl)-6-trifluormethyl-2,4-(1H,3H)-pyrimidindion, Smp. 153-155°C;
- 5-Aethoxy-4-chlor-2-fluorphenylisocyanat das 3-(5-Aethoxy-4-chlor-2-fluorphenyl)-6-trifluormethyl-2,4-(1H,3H)-pyrimidindion, Smp. 162-165°C;
- 2-(2-Chlor-4-fluor-5-isocyanatophenoxy)propionsäure-äthylester den 2-[2-Chlor-5-(3,6-dihydro-2,6-dioxo-4-trifluormethyl -1(2H)-pyrimidinyl)-4-fluorphenoxy]-propionsäure-äthylester, Smp. 144-146°C;
- 5-Allyloxy-4-chlor-2-fluorphenylisocyanat das 3-(5-Allyloxy-4-chlor-2-fluorphenyl)-6-trifluormethyl-2,4-(1H,3H)-pyrimidindion, Smp. 119-122°C;
- 4-Chlor-2-fluor-5-propargyloxyphenylisocyanat das 3-(4-Chlor-2-fluor-5-propargyloxyphenyl)-6-trifluormethyl-2,4(1H,3H)-pyrimidindion, Smp. 137-140°C
- 4-Brom-2-fluor-5-isopropoxyphenylisocyanat das 3-[4-Brom-2-fluor-5-isopropoxyphenyl)-6-trifluormethyl-2,4(1H,3H)-pyrimidindion, Smp. 182°C.

In analoger Weise erhält man beim Einsatz von:

- 3-Amino-2,4-difluorcrotonsäure-äthylester und 4-Chlor-2-fluor-5-isopropoxyphenylisocyanat das 3-(4-Chlor-2-fluor-5-isopropoxyphenyl)-5-fluor-6 -fluormethyl-2,4(1H,3H)-pyrimidindion, Smp. 152-154°C;
- 3-Amino-4,4,5,5,5-pentafluor-2-pentensäure-äthylester und 4-Chlor-2-fluor-5-isopropoxyphenylisocyanat das 3-(4-Chlor-2-fluor-5-isopropoxyphenyl)-6-pentafluoräthyl -2,4(1H, 3H)-pyrimidindion, Smp. 141-143°C;
- 3-Amino-2,4,4,5,5,5-hexafluor-2-pentensäure-äthylester und 4-Chlor-2-fluor-5-isopropoxyphenylisocyanat das 3-(4-Chlor-2-fluor-5-isopropoxyphenyl)-5-fluor-6 -pentafluoräthyl-2,4(1H,3H)-pyrimidindion, Smp. 160-162°C;
- 3-Amino-2,4,4,4-tetrafluorcrotonsäure-äthylester und 4-Chlor-2-fluor-5-isopropoxyphenylisocyanat das 3-(4-Chlor-2-fluor-5-isopropoxyphenyl)-5-fluor-6 -trifluormethyl-2,4(1H,3H)-pyrimidindion, Smp. 133-135°C.

Beispiel 2

7,00 g 4-Chlor-2-fluor-5-isopropoxyphenylharnstoff und 4,44 g Cyclopentanon-2-carbonsäure-äthylester werden mit 0.3 g Toluol-4-sulfonsäure-Monohydrat in 150 ml Benzol 1,5 Stunden auf Rückflusstemperatur erhitzt. Das sich bildende Wasser wird mittels eines Wasserabscheiders entfernt. Anschliessend wird das Reaktionsgemisch zur Trockene eingedampft und der Rückstand an einer Kieselgel-Säule unter Verwendung von Methylenchlorid als Laufmittel chromatographisch gereinigt und aus Diäthyläther/n-Hexan umkristallisiert. Man erhält den 2-[3-(4-Chlor-2-fluor-5-isopropoxyphenyl)ureido]-1-cyclopentencarbonsäure-äthylester. Smp. 166-169°C.

Eine Suspension von 6,0 g des 2-[3-(4-Chlor-2-fluor-5-isopropoxyphenyl)ureido] -1-cyclopentencarbonsäure-äthylesters in 100 ml absolutem Methanol wird unter Rühren bei Raumtemperatur mit 7,8 ml einer 2N Natriummethylat-Lösung versetzt, und das Reaktionsgemisch wird 2 Stunden gerührt. Die Lösung wird mit 7,8 ml 2N Salzsäure versetzt und zur Trockene eingedampft. Der Rückstand wird in 200 ml Essigsäure-äthylester gelöst und die Lösung mit Wasser gewaschen, über wasserfreiem Natriumsulfat getrocknet und unter vermindertem Druck zur Trockene eingedampft. Der Rückstand wird aus Methylenchlorid/Diäthyläther umkristallisiert. Man erhält das 3-(4-Chlor-2-fluor-5-isopropoxyphenyl)-1,5,6,7-tetrahydro -2H-cyclopenta[d]-pyrimidin-2,4(3H)-dion, Smp. 215-217°C.

In analoger Weise erhält man beim Einsatz von:

- 4-Chlor-2-fluor-5-isopropoxyphenylharnstoff und Acetessigsäure-äthylester under Verwendung von fein pulverisiertem Amberlyst®-15 (ein freie Sulfongruppen aufweisendes, organisches, polymerisches Harz) als Katalysator den 3-[3-(4-Chlor-2-fluor-5-isopropoxyphenyl)ureido] -crotonsäure-äthylester, Smp. 142-144°C, und aus diesem Zwischen produkt in Dimethylformamid mit Natriumhydrid das 3-(4-Chlor-2-fluor-5-isopropoxyphenyl)-6-methyl -2,4(1H, 3H)-pyrimidindion, Smp. 228-230°C:
- 4-Chlor-2-fluor-5-isopropoxyphenylharnstoff und 2-Acetylpropionsäure-äthylester unter Verwendung von fein pulverisiertem Amberlyst®-15 als Katalysator den 3-[3-(4-Chlor-2-fluor-5-isopropoxyphenyl)ureido] -2-methyl-crotonsäure-äthylester, und aus diesem Zwischenprodukt in Dimethylformamid mit Natriumhydrid das 3-(4-Chlor-2-fluor-5-isopropoxyphenyl)-5,6-dimethyl -2,4(1H,3H)-pyrimidindion, Smp. 125-127°C;
- 4-Chlor-2-fluor-5-isopropoxyphenylharnstoff und 3-Oxopentansäure-äthylester unter Verwendung von fein pulverisiertem Amberlyst®-15 als Katalysator in Cyclohexan den 3-[3-(4-Chlor-2-fluor-5-isopropoxyphenyl)ureido]-2-pentensäure-äthylester, Smp. 118-120°C, und aus diesem Zwischenprodukt mit Natriumhydrid in Dimethylformamid das 6-Aethyl-3-(4-chlor-2-fluor-5-isopropoxyphenyl)-2,4(1H,3H)-pyrimidindion, Smp. 177-179°C;
- 4-Chlor-2-fluor-5-isopropoxyphenylharnstoff und 3-Oxohexansäure-äthylester unter Verwendung von fein pulverisiertem Amberlyst®-15 als Katalysator in Cyclohexan den 3-[3-(4-Chlor-2-fluor-5-isopropoxyphenyl)-ureido]-2-hexensäure-äthylester, Smp. 103-105°C, und aus diesem Zwischenprodukt mit Natriumhydrid in Dimethylformamid das 3-(4-Chlor-2-fluor-5-isopropoxyphenyl)-6-propyl-2,4(1H,3H)-pyrimidindion, Smp. 199-200°C;
- 4-Chlor-2-fluor-5-isopropoxyphenylharnstoff und Cyclohexanon-2-carbonsäure-äthylester unter Verwendung von fein pulverisiertem Amberlyst®-15 als Katalysator in Cyclohexan den 2-[3-(4-Chlor-2-fluor-5-isopropoxyphenyl)ureido]-1-cyclohexencarbonsäure-äthylester, Smp. 133-135°C, und aus diesem Zwischenprodukt mit Natriumhydrid in Dimethylformamid das 3-(4-Chlor-2-fluor-5-isopropoxyphenyl)-5,6,7,8-tetrahydro-2,4(1H,3H)-chinazolindion, Smp. 201-203°C.

Beispiel 3

Eine Lösung von 6,00 g 4-Chlor-2-fluor-5-isopropoxyphenylisocyanat in 100 ml absolutem Diäthyläther wird unter Rühren bei Raumtemperatur zu einer Lösung von 4,00 g 3-Amino-2-fluorcrotonsäure-äthylester während 1 Stunde zugetropft, und das Reaktionsgemisch wird 16 Stunden nachgerührt. Das Gemisch wird dann unter vermindertem Druck zur Trockene eingedampft, der Rückstand in 50 ml absolutem Dimethylformamid gelöst und die Lösung unter Rühren bei Raumtemperatur zu einer Lösung von 1,31 g einer 55%igen Natriumhydrid-Dispersion in 50 ml Dimethylformamid und 100 ml Isopropylalkohol zugefügt. Anschliessend wird 4 Stunden bei Raumtemperatur nachgerührt, mit konzentrierter Essigsäure auf pH ~4 eingestellt und unter vermindertem Druck eingeengt. Der Rückstand wird auf 1 l Wasser gegossen und zweimal mit je 200 ml Essigsäure-äthylester ausgeschüttelt, und die organischen Phasen werden mit Wasser gewaschen, über wasserfreiem Natriumsulfat getrocknet und unter vermindertem Druck zur Trockene eingedampft. Der Rückstand wird an einer Kieselgel-Säule unter Verwendung von Essigsäure-äthylester/n-Hexan als Laufmittel chromatographisch gereinigt und anschliessend aus Essigsäure-äthylester/Diäthyläther umkristallisiert. Man erhält das 3-(4-Chlor-2-fluor-5-isopropoxyphenyl)-5-fluor-6-methyl -2,4(1H,3H)-pyrimidindion, Smp. 162-164°C.

In analoger Weise erhält man beim Einsatz von:

- 4-Chlor-2-fluor-5-isopropoxyphenylisocyanat in Diäthyläther mit 3-Amino-2-methyl-2-pentensäure-äthylester den 3-[3-(4-Chlor-2-fluor-5-isopropoxyphenyl)ureido]-2-methyl-2-pentensäure-äthylester, Smp. 104-106°C, und aus diesem Zwischenprodukt mit Natriumhydrid in Dimethylformamid das 6-Aethyl-3-(4-chlor-2-fluor-5-isopropoxyphenyl)-5-methyl-2,4(1H,3H)-pyrimidindion, Smp. 196-198°C;
- 4-Chlor-2-fluor-5-isopropoxyphenylisocyanat in Dimethylformamid mit 3-Amino-2-äthylcrotonsäure-äthylester den 2-Aethyl-3-[3-(4-chlor-2-fluor-5-isopropoxyphenyl)ureido]-crotonsäure-äthylester. Smp. 112-114°C, und aus diesem Zwischenprodukt mit Natriumhydrid in Dimethylformamid das 5-Aethyl-3-(4-chlor-2-fluor-5-isopropoxyphenyl)-6-methyl-2,4(1H,3H)-pyrimidindion, Smp. 128-130°C.

Beispiel 4

Eine Lösung von 10.5 g 3-Amino-4,4,4-trifluorcrotonsäure-äthylester in 25 ml absolutem Dimethylformamid wird unter Rühren bei 0°C während 5 Minuten zu einer Suspension von 2,5 g einer 55%igen Natriumhydrid-Dispersion in 25 ml absolutem Dimethylformamid zugetropft. Die Temperatur steigt auf 20°C an. Anschliessend wird 30 Minuten bei dieser Temperatur nachgerührt. Danach werden 14.4 g 4-Chlor-2-fluor-5-isopropoxyphenyl-äthylcarbamat zugefügt, 30 Minuten bei Raumtemperatur nachgerührt und 2.5 Stunden auf 150°C (Badtemperatur) erhitzt. Das Reaktionsgemisch wird auf 50°C abgekühlt, mit 3,5 ml konzentrierter Essigsäure neutralisiert und unter vermindertem Druck bei 60°C weitgehend eingeengt. Der Rückstand wird auf eine Lösung von 200 ml Wasser und 30 ml 2N Salzsäure gegossen und das wässrige Gemisch zweimal mit je 150 ml Diäthyläther extrahiert, und die organischen Phasen werden zweimal mit je 50 ml Wasser gewaschen, über wasserfreiem Natriumsulfat getrocknet und unter vermindertem Druck zur Trockene eingedampft. Der Rückstand wird aus Diäthyläther/n-Hexan umkristallisiert. Man erhält das 3-(4-Chlor-2-fluor-5-isopropoxyphenyl)-6-trifluormethyl-2,4(1H,3H)-pyrimidindion, Smp. 195-197°C.

Beispiel 5

Zu einer Lösung von 1.10 g einer 55%igen Natriumhydrid-Dispersion in 100 ml Isopropanol/Dimethylformamid (1:1) wird eine Lösung von 9,00 g 3-[3-(4-Chlor-2-fluor-5-isopropoxyphenyl)-ureido] -crotonsäure-äthylester in 100 ml wasserfreiem Dimethylformamid unter Rühren bei Raumtemperature zugetropft und 1 Stunde nachgerührt. Anschliessend wird das Reaktionsgemisch mit 4,70 g Dimethylsulfat versetzt und 2 Stunden bei Raumtemperatur gerührt. Das Lösungsmittel wird weitgehend unter vermindertem Druck abdestilliert und der Rückstand auf 500 ml Wasser gegossen und zweimal mit je 200 ml Essigsäure-äthylester extrahiert. Die organischen Phasen werden mit Wasser gewaschen, über wasserfreiem Natriumsulfat getrocknet und unter vermindertem Druck zur Trockene eingedampft. Der Rückstand wird aus Diäthyläther/n-Hexan umkristallisiert. Man erhält das 3-(4-Chlor-2-fluor-5-isopropoxyphenyl)-1,6-dimethyl -2,4(1H,3H)-pyrimidindion, Smp. 149-151°C.

Beispiel 6

8,0 g 3-(4-Chlor-2-fluor-5-propargyloxyphenyl) -6-trifluormethyl-2,4(1H,3H)-pyrimidindion wird unter Rühren bei 25°C zu einer Suspension von 0,96 g einer 55%igen Natriumhydrid-Dispersion in 25 ml absolutem Dimethylformamid zugefügt. Nach beendeter Wasserstoffenwicklung wird das Reaktionsgemisch mit 4,16 g Dimethylsulfat versetzt, und die Temperatur steigt während 15 Minuten auf 30°C an. Anschliessend wird 2 Stunden bei Raumtemperatur nachgerührt. Das Reaktionsgemisch wird auf 800 ml Wasser gegossen und zweimal mit je 100 ml Essigsäure-äthylester extrahiert, und die organischen Phasen werden zweimal mit je 50 ml Wasser gewaschen und über wasserfreiem Natriumsulfat getrocknet. Die Lösung wird unter vermindertem Druck zur Trockene eingedampft und der Rückstand an einer Kieselgel-Säule unter Verwendung von Diäthyläther/n-Hexan (1:1) als Laufmittel chromatographisch gereinigt. Man erhält das 3-(4-chlor-2-fluor-5-propargyloxyphenyl)-1-methyl -6-trifluormethyl--2,4(1H,3H)-pyrimidindion, Smp. 87-90°C.

In analoger Weise erhält man beim Einsatz von:

- 3-(4-Chlor-2-fluor-5-isopropoxyphenyl)-6-trifluormethyl-2,4(1H,3H)-pyrimidindion und Dimethylsulfat das 3-(4-Chlor-2-fluor-5-isopropoxyphenyl)-1-methyl -6-trifluormethyl-2,4(1H,3H)-pyrimidindion, Smp. 90-93°C;
- 3-(5-Aethoxy-4-chlor-2-fluorphenyl)-6-trifluormethyl-2,4(1H,3H)-pyrimidindion und Dimethylsulfat das 3-(5-Aethoxy-4-chlor-2-fluorphenyl)-1-methyl-6-trifluormethyl-2,4(1H,3H)-pyrimidindion, Smp. 118-121°C;
- 3-(4-Chlor-2-fluor-5-methoxyphenyl)-6-trifluormethyl-2,4(1H,3H)-pyrimidindion und Dimethylsulfat das 3-(4-Chlor-2-fluor-5-methoxyphenyl)-1-methyl-6-trifluormethyl-2,4(1H,3H)-pyrimidindion. Smp. 128-130°C;
- 2-[2-Chlor-5-(3,6-dihydro-2,6-dioxo-4-trifluormethyl-1(2H)-pyrimidinyl)-4-fluorphenoxy]-propionsäureäthylester und Dimethylsulfat den 2-[2-Chlor-5-(3,6-dihydro-2,6-dioxo-3-methyl-4-trifluormethyl -1(2H)-pyrimidinyl)-4-fluorphenoxy]-propionsäure-äthylester, $^1$H-NMR ($CDCl_3$, 400 MHz(: 7,32 ppm (d,1H), 6,83 ppm (d,0,5H), 6,81 ppm (d,0,5H), 6,34 ppm (2s,1H), 4,53-4,72 ppm (m,1H). 4,13-4,27 ppm (m,2H), 3,50-3,59 ppm (m,3H), 1,67 ppm (d,3H), 1,18-1,28 ppm( m,3H);
- 3-(5-Butoxy-4-chlor-2-fluorphenyl)-6-trifluormethyl-2,4(1H,3H)-pyrimidindion und Dimethylsulfat das 3-(5-(Butoxy-4-chlor-2-fluorphenyl)-1-methyl -6-trifluormethyl-2,4(1H,3H)-pyrimidindion. Smp. 72-74°C;
- 3-(4-Chlor-2-fluor-5-propoxyphenyl)-6-trifluormethyl-2,4(1H,3H)-pyrimidindion und Dimethylsulfat das 3-(4-Chlor-2-fluor-5-propoxyphenyl)-1-methyl -6-trifluormethyl-2,4(1H,3H)-pyrimidindion. Smp. 103-104°C;
- 3-(5-Allyloxy-4-chlor-2-fluorphenyl)-6-trifluormethyl-2,4(1H, 3H)-pyrimidindion und Dimethylsulfat das 3-(5-Allyloxy-4-chlor-2-fluorphenyl)-1-methyl -6-trifluormethyl-2,4(1H,3H)-pyrimidindion. Smp. 108-110°C;
- 3-(4-Chlor-2-fluor-5-isopropoxyphenyl) -1,5,6,7-tetrahydro-2H-cyclopenta[d]pyrimidin-2,4(3H)-dion in Natriummethylat/Methanol mit Methyljodid das 3-(4-Chlor-2-fluor-5-isopropoxyphenyl)-1,5,6,7-tetrahydro -1-methyl-2H-cyclopenta[d]pyrimidin-2,4(3H)-dion, Smp. 125-127°C;
- 3-(4-Chlor-2-fluor-5-isopropoxyphenyl)-5,6-dimethyl-2,4(1H,3H)-pyrimidindion und Dimethylsulfat das 3-(4-Chlor-2-fluor-5-isopropoxyphenyl)-1,5,6-trimethyl -2,4(1H,3H)-pyrimidindion, Smp. 105-107°C
- 3-(4-Chlor-2-fluor-5-isopropoxyphenyl)-5-fluor-6-fluormethyl-2,4(1H,3H)-pyrimidindion und Dimethylsulfat das 3-(4-Chlor-2-fluor-5-isopropoxyphenyl)-5-fluor-6 -fluormethyl-1-methyl-2,4(1H,3H)-pyrimidindion, Smp. 88-90°C;
- 3-(4-Chlor-2-fluor-5-isopropoxyphenyl)-6-pentafluoräthyl-2,4(1H, 3H)-pyrimidindion in Aceton mit Natriumhydrogencarbonat und Dimethylsulfat das 3-(4-Chlor-2-fluor-5-isopropoxyphenyl)-1-methyl-6 -pentafluoräthyl-2,4(1H,3H)-pyrimidindion, Smp. 79-81°C;
- 3-(4-Chlor-2-fluor-5-isopropoxyphenyl)-5-fluor-6-pentafluoräthyl-2,4(1H,3H)-pyrimidindion in Aceton mit Natriumhydrogencarbonat und Dimethylsulfat das 3-(4-Chlor-2 -fluor-5-isopropoxyphenyl)-5-fluor-1-methyl -6-pentafluoräthyl-2,4(1H,3H)-pyrimidindion, Smp. 107-109°C;
- 3-(4-Chlor-2-fluor-5-isopropoxyphenyl)-5-fluor-6-trifluormethyl-2,4(1H,3H)-pyrimidindion und Dimethylsulfat das 3-(4-Chlor-2-fluor-5-isopropoxyphenyl)-5-fluor-1-methyl-6-trifluormethyl-2,4(1H,3H)-pyrimidindion. Smp. 104-107°C;
- 3-(4-Chlor-2-fluor-5-isopropoxyphenyl)-5-fluor-6-methyl-2,4(1H,3H)-pyrimidindion und Dimethylsulfat das 3-(4-Chlor-2-fluor-5-isopropoxyphenyl)-1,6-dimethyl-5-fluor-2,4(1H,3H)-pyrimidindion, Smp. 139-141°C;
- 6-Aethyl-3-(4-chlor-2-fluor-5-isopropoxyphenyl)-2,4-(1H,3H)-pyrimidindion und Dimethylsulfat das 6-Aethyl-3-(4-chlor-2-fluor-5-isopropoxyphenyl)-1-methyl-2,4(1H,3H)-pyrimidindion, Smp. 72-74°C;
- 6-Aethyl-3-(4-chlor-2-fluor-5-isopropoxyphenyl)-5-methyl-2,4(1H,3H)-pyrimidindion und Dimethylsulfat das 6-Aethyl-3-(4-chlor-2-fluor-5-isopropoxyphenyl)-1,5-dimethyl-2,4(1H,3H)-pyrimidindion, Smp. 88-91°C;
- 5-Aethyl-3-(4-chlor-2-fluor-5-isopropoxyphenyl)-6-methyl-2,4(1h, 3H)-pyrimidindion und Dimethylsulfat das 5-Aethyl-3-(4-chlor-2-fluor-5-isopropoxyphenyl)-1,6-dimethyl-2,4(1H,3H)-pyrimidindion, Smp. 124-125°C;
- 3-(4-Chlor-2-fluor-5-isopropoxyphenyl)-6-propyl-2,4(1H,3H)-pyrimidindion und Dimethylsulfat das 3-(4-Chlor-2-fluor-5-isopropoxyphenyl)-1-methyl-6-propyl-2,4(1H,3H)-pyrimidindion, Smp. 115-117°C;
- 3-(4-Brom-2-fluor-5-isopropoxyphenyl)-6-trifluor-methyl-2,4(1H,3H)-pyrimidindion mit Kaliumcarbonat und Methyljodid in Acetonitril das 3-(4-Brom-2-fluor-5 -isopropoxyphenyl)-1-methyl-6-trifluormethyl-2,4(1H,3H)-pyrimindion, Smp. 79°C;
- 3-(4-Chlor-2-fluor-5-isopropoxyphenyl)-6-trifluormethyl-2,4(1H,3H)-pyrimidindion mit Kaliumcarbonat und Chlorameisensäure-äthylester in Aceton das 1-Aethoxycarbonyl-3-(4-chlor-2-fluor-5-isopropoxyphenyl)-6-trifluormethyl-2,4(1H,3H)-pyrimidindion, $^1$H-NMR ($CDCl_3$, 400 MHz): 7,30 ppm (d,1H), 6,83 ppm (d,1H), 6,37 ppm (s,1H), 4.51 ppm (q,2H), 4.45 ppm (m,1H), 1,42 ppm (t,3H), 1,37 ppm (d,6H);
- Allylbromid und Natriumhydrid in Dimethylformamid das 1-Allyl-3-(4-chlor-2-fluor-5-isopropoxyphenyl)-6-trifluormethyl-2,4(1H,3H)-pyrimidindion, $^1$H-NMR ($CDCl_3$, 400 MHz): 7,29 ppm (d, 1H), 6,81 ppm (d, 1H), 6,37 ppm (s, 1H), 5,2 ppm (m,1H), 5,25-5,34 ppm (m,2H), 4,56 ppm (m,2H), 4,45 ppm (m,1H), 1,37 ppm (d,6H);
- Chlormethyl-methyl-äther und Natriumhydrid in Dimethylformamid das 3-(4-Chlor-2-fluor-5-isopropoxyphenyl)-1-methoxymethyl-6-trifluormethyl-2,4(1H,3H)-pyrimidindion, $^1$H-NMR ($CDCl_3$, 400 MHz): 7,30 ppm

(d,1H), 6,82 ppm (d,1H), 6,40 ppm (s,1H), 5,37 ppm (q,2H), 4,45 ppm (m,1H), 3,49 ppm (s,3H), 1,37 ppm (d,6H);

- 3.Brom-1-propin und Natriumhydrid in Dimethylformamid das 3-(4-Chlor-2-fluor-5-isopropoxyphenyl)-1-(2-propinyl)-6-trifluormethyl-2,4(1H,3H)-pyrimidindion, $^1$H-NMR ($CDCl_3$, 400 MHz): 7,30 ppm (d,1H), 6,84 ppm (d,1H), 6,40 ppm (s,1H), 4,72 ppm (m,2H), 4,46 ppm (m,1H), 2,36 ppm (t,1H), 1,38 ppm (d,3H), 1,37 ppm (d,3H);

- 3-(4-Chlor-2-fluor-5-isopropoxyphenyl)-5,6,7,8-tetrahydro-2,4(1H,3H)-chinazolindion mit Dimethylsulfat und Natriumhydrid in Dimethylformamid das 3-(4-Chlor-2-fluor-5 -isopropoxyphenyl)-1-methyl-5,6,7,8-tetrahydro-2,4(1H,3H)-chinazolindion, Smp. 143-145°C.

Beispiel 7

Zu einer Suspension von 0,28 g einer 55%igen Natriumhydrid-Dispersion in 10 ml Dimethylformamid wird bei Raumtemperatur unter Rühren eine Lösung von 2,00 g 3-(4-Chlor-2-fluor-5-isopropoxyphenyl)-6-methyl -2,4(1H,3H)-pyrimidindion in 20 ml Dimethylformamid innert 30 Minuten zugetropft. Anschliessend wird während 30 Minuten nachgerührt und danach werden während 18 Stunden 5.26 g Chlordifluormethan unter Rühren bei 55°C eingeleitet. Das Reaktionsgemisch wird abgekühlt, auf eine Lösung von 20 ml 2N Salzäure in 300 ml Wasser gegossen und mit 150 ml Essigsäure-äthylester extrahiert. Die organische Phase wird zweimal mit je 100 ml Wasser gewaschen, über wasserfreiem Natriumsulfat getrocknet und unter vermindertem Druck zur Trockene eingedampft. Der Rückstand wird in 100 ml Diäthyläther gelöst und die Lösung mit Aktivkohle behandelt und filtriert. Das Filtrat wird zur Trockene eingedampft und der Rückstand aus Diäthyläther/n-Hexan umkristallisiert. Man erhält das 3-(4-Chlor-2-fluor-5-isopropoxyphenyl)-1-difluormethyl -6-methyl-2,4(1H,3H)-pyrimidindion, Smp. 97-99°C.

Beispiel 8

Zu einer Suspension von 0.26 g einer 55%igen Natriumhydrid-Dispersion in 20 ml absolutem Dimethylformamid wird eine Lösung von 2,00 g 3-(4-Chlor-2-fluor-5-hydroxyphenyl)-1-methyl-6-trifluormethyl -2,4-(1H,3H)-pyrimidindion in 20 ml Dimethylformamid zugefügt, und das Gemisch wird bis zur Beendigung der Wasserstoffenwicklung bei Raumtemperatur gerührt. Anschliessend wird 0,57 g Chlordimethyläther zugefügt und 2 Stunden bei Raumtemperatur gerührt. Das Reaktionsgemisch wird unter vermindertem Druck zur Tröckene eingedampft, der Rückstand in 100 ml Essigsäure-äthylester aufgenommen, und die Lösung zweimal mit Wasser gewaschen, über wasserfreiem Natriumsulfat getrocknet und unter vermindertem Druck zur Trockene eingedampft. Der Rückstand wird an einer Kieselgel-Säule unter Verwendung von Diäthyläther/n-Hexan als Laufmittel chromatographisch gereinigt. Man erhält das 3-(4-Chlor-2-fluor-5-methoxymethoxy-phenyl)-1-methyl -6-trifluormethyl-2,4(1H,3H)-pyrimidindion, $^1$H-NMR ($CDCl_3$, 400 MHz): 7,31 ppm (d,1H), 7,12 ppm (d,1H), 6,36 ppm (s,1H), 5,22 ppm (d,1H), 5,19 ppm (d,1H), 3,55 ppm (d,3H), 3,51 ppm (s,3H).

In analoger Weise erhält man beim Einsatz von:

- Chlorameisensäure-methylester mit Pyridin in Methylenchlorid das [2-Chlor-5-(3,6-dihydro-3-methyl-4-trifluormethyl -2,6-dioxo-1(2H)-pyrimidinyl)-4-fluorphenyl]methylcarbonat, $^1$H-NMR ($CDCl_3$, 400 MHz): 7.38 ppm (d,1H), 7,24 ppm (d,1H), 6,36 ppm (s,1H), 3,93 ppm (s,3H), 3,55 ppm (d,3H);
- Chlorameisensäure-butylester mit Pyridin in Methylenchlorid das Butyl-[2-chlor-5-(3,6-dihydro-3-methyl-4-trifluormethyl -2,6-dioxo-1(2H)-pyrimidinyl)-4-fluorphenyl]-carbonat, $^1$H-NMR ($CDCl_3$, 400 MHz): 7,37 ppm (d,1H), 7,24 ppm (d,1H), 6,35 ppm (s,1H), 4,29 ppm (t,2H), 3,55 ppm (d,3H), 1,73 ppm (m,2H), 1,45 ppm (m,2H), 0,97 ppm (t,3H);
- Chlorameisensäure-isopropylester mit Pyridin in Methylenchlorid das [2-Chlor-5-(3,5-dihydro-3-methyl-4-trifluormethyl -2,6-dioxo-1(2H)-pyrimidinyl)-4-fluorphenyl]isopropylcarbonat. $^1$H-NMR ($CDCl_3$, 400 MHz): 7,37 ppm (d,1H), 7,24 ppm (d,1H), 6,35 ppm (s,1H), 4,99 ppm (m,1H), 3,55 ppm (d,3H), 1,38 ppm (d,6H):
- Chlorameisensäure-vinylester mit Pyridin in Methylenchlorid das [2-Chlor-5-(3,6-dihydro-3-methyl-4-trifluormethyl -2,6-dioxo-1(2H)-pyrimidinyl)-4-fluorphenyl]vinylcar bonat, $^1$H-NMR ($CDCl_3$, 400 MHz): 7,40 ppm (d,1H), 7,28 ppm (d,1H), 7,11 ppm (q,1H), 6,36 ppm (s,1H), 5,08 ppm (q,1H), 4,73 ppm (q,1H), 3,55 ppm (d,3H);
- Chlorameisensäure-cyclohexylester mit Pyridin in Methylenchlorid das [2-Chlor-5-(3,6-dihydro-3-methyl-4-trifluormethyl -2,6-dioxo-1(2H)-pyrimidinyl)-4-fluorphenyl]cyclohexylcarbonat, $^1$H-NMR ($CDCl_3$, 400 MHz): 7,37 ppm (d,1H), 7,24 ppm (d,1H), 6,36 ppm (s,1H), 4,69 ppm (m,1H), 3,56 ppm (d,3H), 1,22-2,04 ppm

(4m,10H);

- Chlorameisensäure-allylester mit Pyridin in Methylenchlorid das Allyl-[2-chlor-5-(3,6-dihydro-3-methyl-4-trifluormethyl -2,6-dioxo-1(2H)-pyrimidinyl)-4-fluorphenyl]-carbonat, $^1$H-NMR ($CDCl_3$, 400 MHz): 7.38 ppm (d,1H), 7,25 ppm (d,1H), 6,36 ppm (s,1H), 5,93-6,05 ppm (m,1H), 5,40-5,48 ppm (m,1H), 5,32-5,38 ppm (m,1H), 4,73-4,79 ppm (m,2H), 3,55 ppm (d,3H);
- Chlorameisensäure-2-propinylester mit Pyridin in Methylenchlorid das [2-Chlor-5-(3,6-dihydro-3-methyl-4-trifluormethyl -2,6-dioxo-1(2H)-pyrimidinyl)-4-fluorphenyl] -2-propinylcarbonat, $^1$H-NMR ($CDCl_3$, 400 MHz): 7,39 ppm (d,1H), 7,27 ppm (d,1H), 6,36 ppm (s,1H), 4,86 ppm (d,2H), 3,55 ppm (d,3H), 2,60 ppm (t,1H);
- Acetylchlorid mit Pyridin in Methylenchlorid das 2-Chlor-5-[3,6-dihydro-3-methyl-4-trifluormethyl -2,6-dioxo-1(2H)-pyrimidinyl]-4-fluorphenylacetat, Smp. 99-101°C;
- Isobuttersäurechlorid mit Pyridin in Methylenchlorid das 2-Chlor-5-[3,6-dihydro-3-methyl-4-trifluormethyl-2,6-dioxo-1(2H)-pyrimidinyl]-4-fluorphenylisobutyrat, $^1$H-NMR ($CDCl_3$, 60 MHz): 7,35 ppm (d,1H), 7,13 ppm (d,1H), 6,34 ppm (s,1H), 3,56 ppm (d,3H), 2,50-3,20 ppm (m,1H), 1,34 ppm (d,6H);
- 2,4-Dichlorbenzoylchlorid mit Pyridin in Methylenchlorid das 2-Chlor-5-[3,6-dihydro-3-methyl-4-trifluormethyl-2,6-dioxo-1(2H)-pyrimidinyl]-4-fluorphenyl-2,4-dichlorbenzoat, $^1$H-NMR ($CDCl_3$, 400 MHz): 8,11 ppm (d,1H), 7,57 ppm (d,1H), 7,43 ppm (d,1H), 7,41 ppm (q,1H), 7,33 ppm (d,1H), 6,37 ppm (s,1H), 3,57 ppm (d,3H);
- Acrylsäurechlorid mit Natriumhydrid in Dimethylformamid das 2-Chlor-5-[3,6-dihydro-3-methyl-4-trifluormethyl-2,6-dioxo-1(2H)-pyrimidinyl]-4-fluorphenylacrylat, $^1$H-NMR ($CDCl_3$, 400 MHz): 7,39 ppm (d,1H), 7,22 ppm (d,1H), 6,67 ppm (q,1H), 6,36 ppm (s,1H), 6,33 ppm (q,1H), 6,09 ppm (q,1H), 3,55 ppm (d,3H);
- 1,2-Dibromäthen mit Natriumhydrid in Dimethylformamid das 3-[5-(2-Bromäthoxy)-4-chlor-2-fluorphenyl]-1-methyl-6-trifluormethyl-2,4(1H,3H)-pyrimidindion, Smp. 125-127°C;
- 1-Brom-3,3-dichlor-2-propen mit Natriumhydrid in Dimethylformamid das 3-[4-Chlor-5-(3,3-dichlor-2-propenyloxy)-2-fluorphenyl] -1-methyl-6-trifluormethyl-2,4(1H,3H)-pyrimidindion, Smp. 126-127°C;
- 2-(2,2-Dichlorvinyloxy)äthylbromid mit Natriumhydrid in Dimethylformamid das 3-{4-Chlor-5-[2-(2,2-dichlorvinyloxy)äthoxy] -2-fluorphenyl}-1-methyl-6-trifluormethyl-2,4(1H,3H)-pyrimidindion, Smp. 97-99°C;
- 2-(2-Methoxyäthoxy)äthylbromid mit Natriumhydrid in Dimethylformamid das 3-{4-Chlor-2-fluor-5-[2-(2-methoxyäthoxy)äthoxy]-phenyl} -1-methyl-6-trifluormethyl-2.4(1H,3H)-pyrimidindion, $^1$H-NMR ($CDCl_3$, 400 MHz): 7,30 ppm (d,1H), 6,86 ppm (d,1H), 6,36 ppm (s,1H), 4,17 ppm (t,2H), 3,89 ppm (t,2H), 3,72-3,78 ppm (m,2H), 3,53-3,59 ppm (m,5H), 3,39 ppm (s,3H);
- 2-Vinyloxyäthylchlorid mit Natriumhydrid in Dimethylformamid das 3-{4-Chlor-2-fluor-5-[2-(vinyloxy)-äthoxy]-phenyl} -1-methyl-6-trifluormethyl-2,4(1H,3H)-pyrimidindion, $^1$H-NMR ($CDCl_3$, 400 MHz): 7,32 ppm (d,1H), 6,85 ppm (d,1H), 6,53 ppm (q,1H), 6,37 ppm (s,1H), 4,19-4,29 ppm (m,3H), 4,03-4,10 ppm (m,3H), 3,56 ppm (d,3H);
- Chloraceton mit Natriumhydrid in Dimethylformamid das 3-(5-Acetonyloxy-4-chlor-2-fluorphenyl)-1-methyl-6-trifluormethyl-2,4(1H,3H)-pyrimidindion, Smp. 164-166°C;
- Chlordimethylsulfid mit Natriumhydrid in Dimethylformamid das 3-[4-Chlor-2-fluor-5-(methylthiomethoxy)-phenyl] -1-methyl-6-trifluormethyl-2,4(1H,3H)-plyrimidindion, $^1$H-NMR ($CDCl_3$, 400 MHz): 7,32 ppm (d,1H), 6,93 ppm (d,1H), 6,36 ppm (s,1H), 5,18 ppm (s,2H), 3,56 pm (d,3H), 2,27 ppm (s,3H);
- 2-Methoxyäthylbromid mit Natriumhydrid in Dimethylformamid das 3-[4-Chlor-2-fluor-5-(2-methoxyäthoxyäthoxy)-phenyl]-1-methyl -6-trifluormethyl-2,4(1H,3H)-pyrimidindion, Smp. 84-87°C;
- 2-Aethoxyäthylbromid mit Natriumhydrid in Dimethylformamid das 3-[5-(2-Aethoxyäthoxy)-4-chlor-2-fluorphenyl]-1-methyl -6-trifluormethyl-2,4(1H,3H)-pyrimidindion, $^1$H-NMR ($CDCl_3$, 400 MHz): 7,30 ppm (d,1H), 6,86 ppm (d,1H), 6,36 ppm (s,1H), 4,14 ppm (t,2H), 3,81 ppm (t,2H), 3,61 ppm (q,2H), 3,55 ppm (d,3H), 1,22 ppm (t,3H);
- 4-Chlorphenoxymethylchlorid mit Natriumhydrid in Dimethylformamid das 3-[4-Chlor-5-(4-chlorphenoxymethoxy)-2-fluorphenyl] -1-methyl-6-trifluormethyl-2,4(1H,3H)-pyrimidindion, Smp. 120-122°C;
- Benzylbromid mit Natriumhydrid in Dimethylformamid das 3-(5-Benzyloxy-4-chlor-2-fluorphenyl)-1-methyl -6-trifluor methyl-2,4(1H,3H)-pyrimidindion, Smp. 129-131°C;
- 4-Phenoxy-2-butinylbromid mit Natriumhydrid in Dimethylformamid das 3-[4-Chlor-2-fluor-5-(4-phenoxy-2-butinyloxy)-phenyl]-1-methyl -6-trifluormethyl-2,4(1H,3H)-pyrimidindion, Smp. 142-144°C;
- N,N-Dimethylchloracetamid mit Natriumhydrid in Dimethylformamid das 3-[4-Chlor-5-(dimethylaminocarbonylmethoxy)-2-fluorphenyl] -1-methyl-6-trifluormethyl-2,4(1H,3H)-pyrimidindion, Smp. 178-180°C;
- Dimethylcarbamoylchlorid und Pyridin in Methylenchlorid das {2-Chlor-5-[3,6-dihydro-3-methyl-4-trifluormethyl-2,6-dioxo -1(2H)-pyrimidinyl]-4-fluorphenyl}dimethylcarbamat. $^1$H-NMR ($CDCl_3$, 400 MHz): 7,34 ppm (d,1H), 7,27 ppm (d,1H), 6,34 ppm (s,1H), 3,54 ppm (d,3H), 3,13 ppm (s,3H), 3,02 ppm (s,3H);

- 4-Chlorbenzolsulfochlorid und Triäthylamin in Methylenchlorid das {2-Chlor-5-[3,6-dihydro-3-methyl-4-trifluormethyl -2,6-dioxo-1(2H)-pyrimidinyl]-4-fluorphenyl}-4-chlorbenzolsulfonat, Smp. 152-154°C;
- Dimethylsulfamoylchlorid und Triäthylamin in Methylenchlorid das {2-Chlor-5-[3,6-dihydro-3-methyl-4-trifluormethyl -2,6-dioxo-1(2H)-pyrimidinyl]-4-fluorphenyl}dimethylsulfamat, Smp. 118-120°C;
- Methansulfonylchlorid und Pyridin in Methylenchlorid das {2-Chlor-5-[3,6-dihydro-3-methyl-4-trifluormethyl -2,6-dioxo-1(2H)-pyrimidinyl]-4-fluorphenyl}methansulfonat, Smp. 176-178°C;
- Chlordifluormethan und Natriumhydrid in Dimethylformamid das 3-(4-Chlor-5-difluormethoxy-2-fluorphenyl)-1-methyl -6-trifluormethyl-2,4(1H,3H)-pyrimidindion, $^1$H-NMR (CDC1$_3$, 400 MHz): 7,39 (d,1H),7,21 ppm (d,1H), 6,52 ppm (t,1H), 6,37 ppm (s,1H), 3,56 ppm (d,3H);
- 5-Chlor-1-methyl-3-trifluormethyl-1H-1,2,4-triazol mit Natriumhydrid in Dimethylformamid das 3-{4-Chlor-2-fluor-5-[(1-methyl-3-trifluormethyl-1H-1,2,4-triazol-5-yl)oxy]phenoxy}-1-methyl-6-trifluormethyl-2,4(1H,3H)-pyrimidindion, Smp. 138-140°C;
- 1,4-Dichlor-2-buten mit Natriumhydrid in Dimethylformamid das 3-{4-Chlor-5-[(E/Z)-4-chlor-2-butenyl)-oxy]-2-fluor-phenyl}-1-methyl-6-trifluormethyl-2,4(1H,3H)-pyrimidindion, $^1$H-NMR ($CDCl_3$, 400 MHz); 7,32 ppm (2d,1H), 6,82 ppm, 6,78 ppm (2d,1H), 6.37 ppm (s,1H), 5,96-6,07 ppm (m,1H), 5,85-5,96 ppm (m,1H), 4,65-4,70 ppm m,1H), 4,54-5,59 ppm (m,1H), 4,07-4,18 ppm (m,2H), 3,56 ppm (m,3H);
- Chlorameisensäure-cyclopropylmethylester mit Pyridin in Methylenchlorid das {2-Chlor-5-[3,6-dihydro-2,6-dioxo-3-methyl-4-trifluormethyl-1(2H)-pyrimidinyl]-4-fluor-phenyl}-cyclopropylmethyl-carbonat, $^1$H-NMR ($CDCl_3$, 400 MHz): 7,38 ppm (d,1H), 7,26 ppm (d,1H), 6,36 ppm (s,1H), 4,12 ppm (m,2H), 3,55 ppm (m,3H, 1,20-1,32 ppm (m,1H), 0,58-0,72 ppm (m,2H), 0,30-0,44 ppm (m,2H);
- Chlorameisensäure-2,2,2-trichloräthylester mit Pyridin in Methylenchlorid das {2-Chlor-5-[3,6-dihydro-2,6-dioxo-3-methyl-4-trifluormethyl-1(2H)-pyrimidinyl]-4-fluorphenyl}-2,2,2-trichloräthylcarbonat, Smp. 140-142°C;
- Chlorameisensäure-2-chloräthylester mit Pyridin in Methylenchlorid das 2-Chloräthyl-{2-Chlor-5-[3,6-dihydro-2,6-dioxo-3-methyl-4-trifluormethyl-1(2H)-pyrimidinyl]-4-fluorphenyl}-carbonat, $^1$H-MNR ($CDCl_3$, 400 MHz): 7,39 ppm (d,1H), 7,26 ppm (d,1H), 6,36 ppm (s,1H), 4,52 ppm (t,2H), 3,78 ppm (t,2H), 3,55 ppm (d,3H);
- Chlorameisensäure-benzylester mit Pyridin in Methylenchlorid das Benzyl-{2-chlor-5-[3,6-dihydro-2,6-dioxo-3-methyl-4-trifluormethyl-1(2H)-pyrimidinyl]-4-fluorphenyl}-carbonat, $^1$H-NMR ($CDCl_3$, 400 MHz): 7,33-7,46 ppm (m,6H), 7,25 ppm (d,1H), 6,35 ppm (s,1H), 5,29 ppm (s,2H), 3,55 ppm (d,3H);
- N-Chlormethylsuccinimid mit Natriumhydrid in Dimethylformamid das N-{[2-Chlor-5-(3,6-dihydro-2,6-dioxo-3-methyl-4-trifluormethyl-1(2H)-pyrimidinyl)-4-fluorphenoxy]methyl}-succinimid, Smp. 212-214°C;
- Chlorameisensäure-cyclohexylmethylester mit Pyridin in Methylenchlorid das {2-Chlor-5-[3,6-dihydro-2,6-dioxo-3-methyl-4-trifluormethyl-1(2H)-pyrimidinyl]-4-fluor-phenyl}-cyclohexylmethyl-carbonat, $^1$H-NMR ($CDCl_3$, 60 MHz): 7,37 ppm (d,1H) 7,24 ppm (d,1H), 6,35 ppm (s,1H), 4,09 ppm (d,2H), 3,57 ppm (d,3H), 0,5-2,2 ppm (m,11H);
- Dithiochlorameisensäure-äthylester mit Natriumhydrid in Dimethylformamid das O-[2-Chlor-5-[3,6-dihydro-2,6-dioxo-3-methyl-4-trifluormethyl-1(2H)-pyrimidinyl]-4-fluorphenyl-S-äthyldithiocarbonat, $^1$H-NMR ($CDCl_3$, 400 MHz): 7,38 ppm (d,1H), 7.13 ppm (d,1H), 6,36 ppm (s,1H), 3,55 ppm (d,3H), 3,24 ppm (q,2H), 1,43 ppm (t,3H);
- 2-Chloräthylcarbaminsäure-äthylester mit Natriumhydrid in Dimethylformamid das Aethyl-[2-{2-chlor-5-[3,6-dihydro-2,6-dioxo-3-methyl-4-trifluormethyl-1(2H)-pyrimidinyl]-4-fluorphenoxy}-äthyl]carbamat, $^1$H-NMR ($CDCl_3$, 400 MHz): 7,31 ppm (d,1H), 6,79 ppm (d,1H) 6,36 ppm (s,1H), 5,20 ppm (t,1H), 4,13 ppm (q,2H), 4,04 ppm (t,2H), 3,60 ppm (q,2H), 3,55 ppm (d,3H), 1,25 ppm (t,3H);
- N-(2-Chloräthyl)-N-methylcarbaminsäure-äthylester mit Natriumhydrid in Dimethylformamid das Aethyl-N-[2-{2-chlor-5-[3,6-dihydro-2,6-dioxo-3-methyl-4-trifluormethyl-1(2H)-pyrimidinyl]-4-fluorphenoxy}-äthyl]-N-methylcarbamat, $^1$H-NMR ($CDCl_3$, 400 MHz): 7,32 ppm (d,1H), 6,77 ppm (d,1H), 6,36 ppm (s,1H), 4,01-4,20 ppm (m,4H), 3,68 ppm (t,2H), 3,56 ppm (d,3H); 3,09 ppm (s,3H), 1,26 ppm (t,3H);
- Chlorschwefelsäure-äthylester in Toluol das {2-Chlor-5-[3,6-dihydro-2,6-dioxo-3-methyl-4-trifluormethyl-1(2H)-pyrimidinyl]-4-fluorphenyl}-äthylsulfat, $^1$H-NMR ($CDCl_3$, 400 MHz): 7,48 ppm (d,1H), 6,36 ppm (s,1H), 4,60 ppm (q,2H), 3,55 ppm (d,3H), 1,47 ppm (t,3H);
- Dimethylthiocarbamoylchlorid mit Natriumhydrid in Dimethylformamid das O-{2-Chlor-5-[3,6-dihydro-2,6-dioxo-3-methyl-4-trifluormethyl-1(2H)-pyrimidinyl]-4-fluorphenyl}-dimethylthioxarbamat, $^1$H-NMR ($CDCl_3$, 400 MHz): 7,35 ppm (d,1H), 7,13 ppm (d,1H), 6,35 ppm (s,1H), 3,56 ppm (d,3H), 3,45 ppm (s,3H), 3,37 ppm (s,3H);
- Thiochlorameisensäure-S-äthylester mit Pyridin in Methylenchlorid das O-{2-Chlor-5-[3,6-dihydro-2,6-dioxo-3-methyl-4-trifluormethyl-1(2H)-pyrimidinyl]-4-fluorphenyl}-S-äthylthiocarbonat, $^1$H-NMR ($CDCl_3$, 400 MHz): 7,38 ppm (d,1H), 7,23 ppm (d,1H), 6.36 ppm (s,1H), 3,56 ppm (d,3H), 2,97 ppm (q,2H), 1,38 ppm

(t,3H);

- Isopropansulfochlorid mit Pyridin in Methylenchlorid das {2-Chlor-5-[3,6-dihydro-2,6-dioxo-3-methyl-4-trifluormethyl-1(2H)-pyrimidinyl]-4-fluorphenyl}isopropansulfonat, $^1$H-NMR ($CDCl_3$, 400 MHz): 7,46 ppm (d,1H), 7,39 ppm (d,1H), 6,36 ppm (s,1H), 3,58 ppm (septet,1H), 3,55 ppm (d,3H), 1,60 ppm (2d,6H);
- Cyclohexancarbonsäurechlorid mit Pyridin in Methylenchlorid das {2-Chlor-5-[3,6-dihydro-2,6-dioxo-3-methyl-4-trifluormethyl-1(2H)-pyrimidinyl]-4-fluorphenyl}-cyclohexancarboxylat, Smp. 97-101°C;
- Thiochlorameisensäure-O-äthylester mit Pyridin in Methylenchlorid das O-{2-Chlor-5-[3,6-dihydro-2,6-dioxo-3-methyl-4-trifluormethyl-1(2H)-pyrimidinyl]-4-fluorphenyl}-O-äthylthiocarbonat, Smp. 101-102°C;
- Chlorameisensäure-2-methoxyäthyl-ester mit Pyridin in Methylenchlorid das {2-Chlor-5-[3,6-dihydro-2,6-dioxo-3-methyl-4-trifluormethyl-1(2H)-pyrimidinyl]-4-fluorphenyl}-2-methoxyäthyl-carbonat, $^1$H-NMR ($CDCl_3$, 250 MHz): 7,38 ppm (d,1H), 7,25 ppm (d,1H), 6,35 ppm (s,1H), 4,42 ppm (quintet,2H), 3,68 ppm (quintet,2H), 3,54 ppm (d,3H), 3,41 ppm (s,3H);
- Cyclopropancarbonsäurechlorid mit Pyridin in Methylenchlorid das {2-Chlor-5-[3,6-dihydro-2,6-dioxo-3-methyl-4-trifluormethyl-1(2H)-pyrimidinyl]-4-fluorphenyl}-cyclopropancarboxylat, $^1$H-NMR ($CDCl_3$, 250 MHz): 7,35 ppm (d,1H), 7,16 ppm (d,1H), 6,35 ppm (s,1H), 3,54 ppm (d,3H), 1,84-1,90 ppm (m,1H), 1,04-1,23 ppm (m,4H);
- Thiophen-2-carbonsäurechlorid mit Pyridin in Methylenchlorid das {2-Chlor-5-[3,6-dihydro-2,6-dioxo-3-methyl-4-trifluormethyl-1(2H)-pyrimidinyl]-4-fluorphenyl}-2-thiophen-carbxylat, $^1$H-NMR ($CDCl_3$, 250 MHz): 8,02 ppm (q,1H), 7,71 ppm (q,1H), 7,41 ppm (d,1H), 7,32 ppm (d,1H), 7,20 ppm (q,1H), 6,36 ppm (s,1H), 3,56 ppm (d,3H);
- Chlorameisensäure-phenylester mit Pyridin in Methylenchlorid das {2-Chlor-5-[3,6-dihydro-2,6-dioxo-3-methyl-4-trifluormethyl-1(2H)-pyrimidinyl]-4-fluorphenyl}-phenyl-carbonat, Smp. 112-115°C;
- Oxalsäure-Monomethylesterchlorid mit Pyridin in Methylenchlorid das {2-Chlor-5-[3,6-dihydro-2,6-dioxo-3-methyl-4-trifluormethyl-1(2H)-pyrimidinyl]-4-fluorphenyl}-methyl-oxalat, Smp. 103-105°C;
- Bromacetaldehyd-dimethylacetal mit Natriumhydrid in Dimethylformamid das 1-[4-Chlor-5-(2,2-dimethoxyäthoxy)-2-fluorphenyl]-3-methyl-4-trifluormethyl-2,6(1H,3H)-pyrimidindion, $^1$H-NMR ($CDCl_3$, 250 MHz): 7,31 ppm (d,1H), 6,84 ppm (d,1H), 3,55 ppm (d,3H), 3,47 ppm (2s,6H);
- 2-Brommethyl-1,3-dioxolan mit Natriumhydrid in Dimethylformamid das 1-{4-Chlor-5-[(1,3-dioxolan-2-yl)-methoxy]-2-fluorphenyl}-3-methyl-4-trifluormethyl-2,6-(1H,3H)-pyrimidindion, $^1$H-NMR ($CDCl_3$, 250 MHz): 7,31 ppm (d,1H), 6,84 ppm (d,1H), 6,36 ppm (s,1H), 5,33 ppm (t,1H), 3,92-4,20 ppm (m,6H), 3,55 ppm (d,3H);
- Benzyl-chlormethyl-äther mit Natriumhydrid in Dimethylformamid das 1-{5-[(Benzyloxy)methoxy]-4-chlor-2-fluorphenyl}-3-methyl-4-trifluormethyl-2,6(1H,3H)-pyrimidindion, $^1$H-NMR ($CDCl_3$, 250 MHz): 7,24-7,40 ppm (m,5H), 7,27 ppm (d,1H), 7,20 ppm (d,1H), 6,34 ppm (s,1H), 5,33 ppm (s,2H), 4,74 ppm (s,1H), 3,54 ppm (d,3H);
- Chlormethyl-propargyl-äther mit Natriumhydrid in Dimethylformamid das 1-{4-Chlor-2-fluor-5-[2-propinyloxy)methyoxy]-phenyl}-3-methyl-4-trifluormethyl-2,6(1H,3H)-pyrimidindion, $^1$H-NMR ($CDCl_3$, 250 MHz): 7,31 ppm (d,1H), 7,13 ppm (d,1H), 6,36 ppm (s,1H), 5,35 ppm (s,2H), 4,36 ppm (d,2H), 3,55 ppm (d,3H), 2,48 ppm (t,1H);
- Brommethylcyclohexan mit Natriumhydrid in Dimethylformamid das 1-(4-Chlor-5-cyclohexylmethoxy-2-fluorphenyl)-3-methyl-4-trifluormethyl-2,6(1H,3H)-pyrimidindion, $^1$H-NMR ($CDCl_3$, 250 MHz): 7,29 ppm (d,1H), 6,75 ppm (d,1H), 6,36 ppm (s,1H), 3,74 ppm (d,2H), 3,55 ppm (d,3H), 1,61-1,91 ppm (m,6H), 0.93-1,42 ppm (m,5H);
- Allyl-chlormethyl-äther mit Natriumhydrid in Dimethylformamid das 1-{5-[(Allyloxyl)methoxy]-4-chlor-2-fluorphenyl}-3-methyl-4-trifluormethyl-2,6(1H,3H)-pyrimidindion, $^1$H-NMR ($CDCl_3$, 250 MHz): 7,30 ppm (d,1H), 7,15 ppm (d,1H), 6,35 ppm (s,1H), 5,79-6,00 ppm (m,1H), 5,27 ppm (s,2H), 5,16-5,32 ppm (m,2H), 4,23 ppm (sextet,2H), 3,55 ppm (t,3H);
- Chlormethyl-4-chlorphenylsulfid mit Natriumhydrid in Dimethylformamid das 1-[4-Chlor-5-{[(p-chlorphenyl)thio]methoxy}-2-fluorphenyl]-3-methyl-4-trifluormethyl-2,6(1H,3H)-pyrimidindion, Smp. 141-142°C;
- Bromcyclopentan mit Natriumhydrid in Dimethylformamid das 1-(4-Chlor-5-cyclopentyloxy-2-fluorphenyl)-3-methyl-4-trifluormethyl-2,6(1H,3H)-pyrimidindion, Smp. 135-137°C;
- 1,4-Dichlor-2-butin mit Natriumhydrid in Dimethylformamid das 3-{4-Chlor-5-[(4-chlor-2-butinyl)oxy]-2-fluorphenyl}-1-methyl-6-trifluormethyl-2,4(1H,3H)-pyrimidindion, Smp. 118-120°C;
- 1-Chlor-4-methoxy-2-buten mit Natriumhydrid in Dimethylformamid das 3-[4-Chlor-2-fluor-5-{[(E/Z)-4-methoxy-2-butenyl]oxy}phenyl}-1-methyl-6-trifluormethyl-2,4(1H,3H)-pyrimidindion, Smp. 78-83°C;
- Brommethylcyclopropan mit Natriumhydrid in Dimethylformamid das 3-(4-Chlor-5-cyclopropylmethoxy-2-fluorphenyl)-1-methyl-6-trifluormethyl-2,4(1H,3H)-pyrimidindion, $^1$H-NMR ($CDCl_3$ 250 MHz): 7,31 ppm (d,1H), 6,77 ppm (d,1H), 6,36 ppm (s,1H), 3,83 ppm (d,2H), 3,55 ppm (d,3H), 1,29 ppm (m,1H), 0,64 ppm

(m,2H), 0,35 ppm (m,2H);

- Chlormethyl-phenylsulfid mit Natriumhydrid in Dimethylformamid das 3-{4-Chlor-2-fluor-5-[(phenylthio)-methoxy]phenyl}-1-methyl-6-trifluormethyl-2,4(1H,3H)-pyrimidindion, Smp. 91-94°C;

- Chlormethyl-cyclopentyläther mit Natriumhydrid in Dimethylformamid das 3-{4-Chlor-5-[-(cyclopentyloxy)methoxy]-2-fluorphenyl}-1-methyl-6-trifluormethyl-2,4(1H,3H)-pyrimidindion, $^1$H-NMR ($CDCl_3$, 250 MHz): 7,30 ppm (d,1H), 7,13 ppm (d,1H), 6,36 ppm (d,1H), 5,27 ppm (s,2H), 4,33 ppm (m,1H), 3,55 ppm (d,3H), 1,41-1,88 ppm (m,8H);

- 2-Brommethyltetrahydropyran mit Natriumhydrid in Dimethylformamid das 3-{4-Chlor-2-fluor-5-[-(tetrahydro-2H-pyran-2-yl)methoxy]phenyl}-1-methyl-6-trifluormethyl)-2,4(1H,3H)-pyrimidindion, $^1$H-NMR ($CDCl_3$, 250 MHz): 7,29 ppm (d,1H), 6,83 ppm (d,1H), 6,36 ppm (s,1H), 3,40-4,10 ppm (m,5H), 3,55 ppm (d,3H), 1,28-2,00 ppm (m,6H);

- Furan-2-carbonsäurechlorid mit Pyridin in Methylenchlorid das {2-Chlor-5-[3,6-dihydro-2,6-dioxo-3-methyl-4-trifluormethyl-1(2H)-pyrimidinyl]-4-fluorphenyl}-2-furoat, $^1$H-NMR ($CDCl_3$, 250 MHz): 7,70 ppm (q,1H), 7,36-7,50 ppm (m,2H), 7,30 ppm (d,1H), 6,61 ppm (q,1H), 7,36 ppm (s,1H), 3,55 ppm (d,3H);

- 2-Chlor-4,6-dimethoxy-s-triazin mit Natriumhydrid in Dimethylformamid das 3-{4-chlor-5-[(4.6-dimethoxy-s-triazin-2-yl)oxy]-2-fluorphenyl}-1-methyl-6-trifluormethyl-2,4(1H,3H)-pyrimidindion, $^1$H-NMR ($CDCl_3$, 250 MHz): 7,40 ppm (d,1H), 7,20 ppm (d,1H), 6,36 ppm (s,1H), 3,99 ppm, (s,6H), 3,56 ppm (d,3H);

- 2-Chlorpyrimidin mit Natriumhydrid in Dimethylformamid das 3-[4-Chlor-2-fluor-5-(2-pyrimidinyloxy)-phenyl]-1 -methyl-6-trifluormethyl-2,4(1H,3H)-pyrimidindion, Smp. 192-194°C;

- 2-Chlor-3-nitropyridin mit Natriumhydrid in Dimethylformamid das 3-{4-Chlor-2-fluor-5-[(3-nitro-2-pyridyl)oxy]-phenyl}-1-methyl-6-trifluormethyl-2,4(1H,3H)-pyrimidindion, Smp. 177-179°C;

- Epibromhydrin mit Natriumhydrid in Dimethylformamid das 3-[4-Chlor-5-(2,3-epoxypropoxy)-2-fluorphenyl]-1-methyl-6-trifluormethyl-2,4(1H,3H)-pyrimidindion, Smp. 93-96°C;

- Chlormethyl-trimethylsilan mit Natriumhydrid in Dimethylformamid das 3-{4-Chlor-2-fluor-5-[-(trimethylsilyl)methoxy]-phenyl}-1-methyl-6-trifluormethyl-24(1H,3H)-pyrimidindion, Smp. 91-93°C;

- 5-Chlormethyl-1-methyltetrazol mit Natriumhydrid in Dimethylformamid das 3-{4-Chlor-2-fluor-5-[(1-methyl-1H-tetrazol-5-yl)methoxy]-phenyl}-1-methyl-6-trifluormethyl-2,4(1H,3H)-pyrimidindion, Smp. 169-171°C;

- Methansulfonsäure-2,2,2-trifluoräthylester mit Natriumhydrid in Dimethylformamid das 3-[4-Chlor-2-fluor-5-(2,2,2-trifluoräthoxy)-phenyl]-1-methyl-6-trifluormethyl-2,4(1H,3H)-pyrimidindion, Smp. 136-138°C;

- 2-Brom-4-methylpentan mit Natriumhydrid in Dimethylformamid das 3-[4-Chlor-5-(1,3-dimethylbutoxy)-2-fluorphenyl]-1-methyl-6-trifluormethyl-2,4(1H,3H)-pyrimidindion, Smp. 169-171°C;

- 1-Jodhexan mit Natriumhydrid in Dimethylformamid das 3-[4-Chlor-5-(n-hexyloxy)-2-fluorphenyl]-1-methyl-6-trifluormethyl-2,4(1H,3H)-pyrimidindion, Smp. 60-63°C;

- 1-Jod-2-methylpropan mit Natriumcarbonat in Acetonitril das 3-(4-Chlor-2-fluor-5-isobutoxyphenyl)-1-methyl-6-trifluormethyl-2,4(1H,3H)-pyrimidindion, $^1$H-NMR ($CDCl_3$, 400 MHz): 7.30 ppm (d, 1H), 6.76 ppm (d, 1H), 6.36 ppm (s, 1H), 3.72 ppm (d, 2H), 3.55 ppm (d, 3H), 2.14 ppm (m, 1H), 1.03 ppm (d, 6H);

- 1-Jod-3-methylbutan mit Natriumhydrid in Dimethylformamid das 3-(4-Chlor-2-fluor-5-(1-isopentoxy)-phenyl]-1-methyl-6-trifluormethyl-2.4(1H, 3H)-pyrimidindion, $^1$H-NMR ($CDCl_3$, 400 MHz): 7,30 ppm (d,1H), 6,77 ppm (d,1H), 6,37 ppm (s,1H), 3,99 ppm (t,2H), 3,56 ppm (d,3H), 1,86 ppm (m,1H), 1,72 ppm (q,2H), 0,96 ppm (d,6H);

- 1-Jod-pentan mit Natriumhydrid in Dimethylformamid das 3-(4-Chlor-2-fluor-5-pentoxy-phenyl)-1-methyl-6-trifluormethyl-2,4(1H,3H)-pyrimidindion, Smp. 64-66°C;

- 2-Brom-pentan mit Natriumhydrid in Dimethylformamid das 2-[4-Chlor-2-fluor-5-(1-methylbutoxy)-phenyl]-1-methyl-6-trifluormethyl-2,4(1H,3H)-pyrimidindion, Smp. 94-97°C;

- 2-Jod-butan mit Natriumcarbonat in Acetonitril das 3-[4-Chlor-2-fluor-5-(1-methylpropoxy)-phenyl]-1-methyl-6-trifluormethyl-2,4(1H,3H)-pyrimidindion, $^1$H-NMR ($CDCl_3$, 400 MHz): 7,29 ppm (d,1H), 6,78 ppm (d,1H), 6,36 ppm (s,1H), 4,22 ppm (sextet,1H), 3,56 ppm (d,3H), 1,60-1,85 ppm (m,2H), 1,31 ppm (d,3H), 0,99 ppm (t,3H);

- 1-Jod-octan-mit Natriumhydrid in Dimethylformamid das 3-(4-Chlor-2-fluor-5-octyloxy-phenyl)-1-methyl-6-trifluormethyl-2,4(1H,3H)-pyrimidindion, Smp. 60-63°C;

- 1-Jod-2,2-dimethylpropan mit Natriumhydrid in Dimethylformamid das 3-[4-Chlor-5-(2,2-dimethylpropoxy)-2-fluor phenyl]-1-methyl-6-trifluormethyl-2,4(1H,3H)-pyrimidindion, Smp. 122-124°C

- Methansulfonsäure-2-chloräthylester mit Natriumhydrid in Dimethylformamid das 3-[4-Chlor-5-(2-chloräthoxy)-2-fluorphenyl]-1-methyl-6-trifluormethyl-2,4(1H,3H)-pyrimidindion, Smp. 100-103°C;

- Diäthyl-(mesyloxy)methanphosphonat und Kaliumcarbonat in Acetonitril das Diäthyl-{2-chlor-5-[3,6-dihydro-2,6-dioxo-3-methyl-4-trifluormethyl-1(2H)-pyrimidinyl]-4-fluorphenoxy}methanphosphonat, Smp. 87-88°C;

- Diäthyl-1-(tosyloxy)äthanphosphonat und Kaliumcarbonat in Acetonitril das Diäthyl-1-{2-chlor-5-[3,6-dihydro-2,6-dioxo-3-methyl-4-trifluormethyl-1(2H)-pyrimidinyl]-4-fluorphenoxy}äthanphosphona, $^1$H-NMR ($CDCl_3$, 60 MHz): 7,36 ppm (d,1H), 7,31 ppm (d,1H), 6,40 ppm (s,1H), 3,90-4,90 ppm (m,5H), 3,60 ppm (s,3H), 1,10-2,00 (m,9H);
- Toluol-4-sulfonsäure-2-[(isopropylidenamino)oxy]äthylester und Kaliumcarbonat in Acetonitril das 3-[4-Chlor-2-fluor-5-{2-[(isopropylidinamino)oxy]äthoxy}-phenyl]-1-methyl-6-trifluormethyl-2,4(1H,3H)-pyrimidindion, Smp. 103°C;
- N,N-Diäthyl-N-[(isopropylidenamino)oxy]methyl-N-methyl-ammoniumhydroxid in Toluol das 3-[4-Chlor-2-fluor-5-{[(isopropylidenamino)oxy]methoxy}-phenyl]-1-methyl-6-trifluormethyl-2,4(1H,3H)-pyrimidindion, Smp. 118°C;
- 3-(4-Brom-2-fluor-5-hydroxyphenyl)-1-methyl-6-trifluormethyl-2,4(1H,3H)-pyrimidindion mit Kaliumcarbonat und Methyljodid in Acetonitril das 3-(4-Brom-2-fluor-5-methoxyphenyl)-1-methyl-6-trifluormethyl-2,4-(1H,3H)-pyrimidindion, Smp. 154°C;
- Aethyljodid und Kaliumcarbonat in Acetonitril das 3-(5-Aethoxy-4-brom-2-fluorphenyl)-1-methyl-6-trifluormethyl-2,4(1H,3H)-pyrimidindion, Smp. 128°C;
- Propyljodid und Kaliumcarbonat in Acetonitril das 3-(4-Brom-2-fluor-5-propoxyphenyl)-1-methyl-6-trifluormethyl-2,4(1H,3H)-pyrimidindion, Smp. 119°C;
- Butyljodid und Kaliumcarbonat in Acetonitril das 3-(4-Brom-5-butoxy-2-fluorphenyl)-1-methyl-6-trifluormethyl-2,4(1H,3H)-pyrimidindion, Smp. 73°C;
- Isobutyljodid und Kaliumcarbonat in Acetonitril das 3-(4-Brom-2-fluor-5-isobutoxyphenyl)-1-methyl-6-trifluormethyl-2,4(1H,3H)-pyrimidindion, $^1$H-NMR ($CDCl_3$, 400 MHz): 7,55 ppm (d,1H), 6,79 ppm (d,1H), 6,43 ppm (s,1H), 3,76 ppm (d,2H), 3,58 ppm (d,3H), 2,17 ppm (m,1H), 1,05 ppm (d,6H);
- Allylbromid und Kaliumcarbonat in Acetonitril das 3-(5-Allyloxy-4-brom-2-fluorphenyl)-1-methyl-6-trifluormethyl-2,4(1H,3H)-pyrimidindion, Smp. 120°C;
- Propargylbromid und Kaliumcarbonat in Acetonitril das 3-[4-Brom-2-fluor-5-(2-propinyloxy)-phenyl]-1-methyl-6-trifluormethyl-2,4(1H,3H)-pyrimidindion, Smp. 134°C;
- N,N-Diäthyl-N-[(isopropylidenamino)oxy]methyl-N-methyl-ammoniumhydroxid in Toluol das 3-[4-Brom-2-fluor-5-{[(isopropylidenamino)oxy]methoxy}-phenyl]-1-methyl-6-trifluormethyl-2,4(1H,3H)-pyrimidindion, Smp. 121°C;
- Chlordimethyläther und Kaliumcarbonat in Acetonitril das 3-[4-Brom-2-fluor-5-(methoxymethoxy)-phenyl]-1-methyl-6-trifluormethyl-2,4(1H,3H)-pyrimidindion, $^1$H-NMR ($CDCl_3$, 400 MHz: 7,46 ppm (d,1H), 7,08 ppm (d,1H), 6,35 ppm (s,1H), 5,22 ppm (s,2H), 3,56 ppm (d,3H), 3,52 ppm (s,3H);
- Toluol-4-sulfonsäure-2-[(isopropylidenamino)oxy]-äthylester und Kaliumcarbonat in Acetonitril das 3-[4-Brom-2-fluor-5-{2-[isopropylidenamino)oxy]äthoxy}-phenyl]-1-methyl-6-trifluormethyl-2,4(1H,3H)-pyrimidindion, Smp. 115°C;
- 3-(4-Chlor-2-fluor-5-hydroxyphenyl)-1-methyl-6-trifluormethyl-2,4(1H,3H)-pyrimidindion mit α-Bromacetophenon und Natriumhydrid in Dimethylformamid das 3-[4-Chlor-2-fluor-5-(phenacyloxy)-phenyl]-1-methyl-6-trifluormethyl-2,4(1H,3H)-pyrimidindion, Smp. 152-153°C;
- 2-Chloroacetamid und Natriumhydrid in Dimethylformamid das 2-{2-Chlor-5-[3,6-dihydro-2,6-dioxo-3-methyl-4-trifluormethyl-1(2H)-pyrimidinyl]-4-fluorphenoxy}-acetamid, Smp. 203-205°C;
- 4-Brombutyronitril und Natriumhydrid in Dimethylformamid das 4-{2-Chlor-5-[3,6-dihydro-2,6-dioxo-3-methyl-4-trifluormethyl-1(2H)-pyrimidinyl]-4-fluorphenoxy}-butyronitril, Smp. 104-106°C;
- N-Methyl-2-chloracetamid und Natriumhydrid in Dimethylformamid das 2-[2-Chlor-5-[3,6-dihydro-2,6-dioxo-3-methyl-4-trifluormethyl-1(2H)-pyrimidinyl]-4-fluorphenoxy]-N-methylacetamid, Smp. 190-192°C;
- 2-Chlormethyl-4,6-dimethylpyrimidin und Natriumhydrid in Dimethylformamid das 3-{4-Chlor-5-[(4,6-dimethyl-2-pyrimidinyl)methoxy]-2-fluorphenyl}-1-methyl-6-trifluormethyl-2,4(1H,3H)-pyrimidindion, $^1$H-NMR ($CDCl_3$, 400 MHz): 7,30 ppm (d,1H), 6,99 ppm (d,1H), 6,96 ppm (s,1H), 6,33 ppm (s,1H), 5,24 ppm (q,2H), 3,54 ppm (d,3H), 2,48 ppm (s,6H);
- 4-Aethoxy-2-chlormethyl-6-methylpyrimidin und Natriumhydrid in Dimethylformamid das 3-{5-[(4-Aethoxy-6-methyl-2-pyrimidinyl)methoxy]-4-chlor-2-fluorphenyl}-1-methyl--6-trifluormethyl-2,4(1H,3H)-pyrimidindion, $^1$H-NMR ($CDCl_3$, 400 MHz): 7,31 ppm (d,1H), 6,92 ppm (d,1H), 6,44 ppm (d,1H), 6,33 ppm (s,1H), 5,20 ppm (q,2H), 4,31 ppm (q,2H), 3,54 ppm (d,3H), 2,42 ppm (s,3H), 1,30 ppm (t,3H);
- 2-Bromäthanol und Natriummethylat in Methanol das 3-[4-Chlor-2-fluor-5-(2-hydroxyäthoxy)-phenyl]-1-methyl-6-trifluormethyl-2,4(1H,3H)-pyrimidindion, $^1$H-NMR ($CDCl_3$, 60 MHz): 7,31 ppm (d,1H), 6,84 ppm (d,1H), 6,37 ppm (s,1H), 3,80-4,25 ppm (m,4H), 3,56 ppm (d,3H), 2,53 ppm (s,1H);
- 2-Chlormethyl-4,7-dimethoxypyrimidin und Natriumhydrid in Dimethylformamid das 3-{4-Chlor-5-[4,6-dimethoxy-2-pyrimidinyl)methoxy]-2-fluorphenyl}-1-methyl-6-trifluor-methyl-2,4(1H,3H)-pyrimidindion, $^1$H-NMR ($CDCl_3$, 400 MHz): 7,33 ppm (d,1H), 6,87 ppm (d,1H), 6,33 ppm (s,1H), 5,93 ppm (s,1H), 5,17 ppm

(q,2H), 3,86 ppm (s,6H), 3,53 ppm (d,3H);

- 2-Chlordiäthyläther und Natriumhydrid in Dimethylformamid das 3-[4-Chlor-5-[2-(2-chloräthoxy)-äthoxy]-2-fluorphenyl]-1-methyl-6-trifluormethyl-2,4(1H,3H)-pyrimidindion, $^1$H-NMR ($CDCl_3$, 400 MHz): 7,31 ppm (d,1H), 6,92 ppm (d,1H), 6,36 ppm (s,1H), 4,18 ppm (m,2H), 3,90 ppm (m,2H), 3,85 ppm (m,2H), 3,65 ppm (m,2H), 3,55 ppm (d,3H);

- 5-Chlor-1,3-dimethyl-4-nitropyrazol und Natriumhydrid in Dimethylformamid das 3-{4-Chlor-5-[(1.3-dimethyl-4-nitropyrazol-5-yl)oxy]-2-fluorphenyl}-1-methyl-6-trifluormethyl-2,4(1H,3H)-pyrimidindion, Smp. 191-193°C;

- Chloracetonitril und Natriumhydrid in Dimethylformamid das {2-Chlor-5-[3,6-dihydro-2,6-dioxo-3-methyl-4-trifluormethyl-1(2H)-pyrimidinyl]-4-fluorphenoxy}acetonitril, Smp. 158-159°C;

- N-Chloracetylpiperidin und Natriumhydrid in Dimethylformamid das N-[{2-Chlor-5-[3,6-dihydro-2,6-dioxo-3-methyl-4-trifluormethyl-1(2H)-pyrimidinyl]-4-fluorphenoxy}acetyl]-piperidin, $^1$H-NMR ($CDCl_3$, 400 MHz): 7,32 ppm (d,1H), 6,98 ppm (d,1H), 6,35 ppm (s,1H), 4,72 ppm (s,2H), 3,48-3,58 ppm (m,7H), 1,46 ppm (m,6H);

- Allyl-chlormethylsulfid und Natriumhydrid in Dimethylformamid das 3-{5-[(Allylthio)methoxy]-4-chlor-2-fluorphenyl}-1-methyl-6-trifluormethyl-2,4(1H,3H)-pyrimidindion, $^1$H-NMR ($CDCl_3$, 400 MHz): 7,32 ppm (d,1H), 6,93 ppm (d,1H), 6,36 ppm (s,1H), 5,73-5,85 ppm (m,1H), 5,13-5,24 ppm (m,4H), 3,55 ppm (d,3H), 3,29 ppm (m,2H);

- Chlormethyl-cyclohexylsulfid und Natriumhydrid in Dimethylformamid das 3-{4-Chlor-5-[(cyclohexylthio)-methoxy]-2-fluorphenyl}-1-methyl-6-trifluormethyl--2,4(1H,3H)-pyrimidindion, $^1$H-NMR ($CDCl_3$, 400 MHz): 7,32 ppm (d,1H), 6,90 ppm (d,1H), 6,36 ppm (s,1H), 5,24 ppm (t,2H), 3,56 ppm (d,3H), 2,89-2,99 ppm (m,1H), 1,99-2,08 ppm (m,2H), 1,71-1,81 ppm (m,2H), 1,56-1,66 ppm (m,1H), 1,18-1,47 ppm (m,5H);

- 3-[4-Chlor-2-fluor-5-(2-hydroxyäthoxy)-phenyl]-1-methyl-6-trifluormethyl-2,4(1H,3H)-pyrimidindion und Acetylchlorid mit Pyridin in Diäthyläther das 2-{2-Chlor-5-[3,6-dioxo-3-methyl-4-trifluormethyl-1(2H)-pyrimidinyl]-4-fluorphenoxy}-äthylacetat, Smp. 88-90°C;

- 3-[4-Chlor-2-fluor-5-(2-hydroxyäthoxy)-phenyl]-1-methyl-6-trifluormethyl-2,4(1H,3H)-pyrimidindion und Chloracetylchlorid mit Pyridin in Diäthyläther das 2-{2-Chlor-5-[3,6-dihydro-2,6-dioxo-3-methyl-4-trifluormethyl-1(2H)-pyrimidinyl]-4-fluorphenoxy}-äthylchlor acetat, Smp. 96-99°C;

- 3-[4-Chlor-2-fluor-5-(2-hydroxyäthoxy)-phenyl]-1-methyl-6-trifluormethyl-2,4(1H,3H)-pyrimidindion und Chlorameisensäure-äthylester mit Pyridin in Diäthyläther das {2-[2-Chlor-5-(3,6-dihydro-2,6-dioxo-3-methyl-4-trifluormethyl-1(2H)-pyrimidinyl]-4-fluorphenoxy]-äthyl}-äthylcarbonat, $^1$H-NMR ($CDCl_3$, 400 MHz): 7,32 ppm (d,1H), 6,81 ppm (d,1H) 6,36 ppm (s,1H), 4,47-4,54 ppm (m,2H), 4,16-4,27 ppm (m,4H), 3,56 ppm (d,3H), 1,32 ppm (t,3H);

- 3-(4-Chlor-2-fluor-5-hydroxyphenyl)-1-methyl-6-trifluormethyl-2,4(1H,3H)-pyrimidindion mit N-Chlorcarbonyl-piperidin und Natriumhydrid in Dimethylformamid das {2-Chlor-5-[3,6-dihydro-2,6-dioxo-3-methyl-4-trifluormethyl-1(2H)-pyrimidinyl]-4-fluorphenyl}-1-piperidincarboxylat, $^1$H-NMR ($CDCl_3$, 400 MHz): 7,33 ppm (d,1H), 7,25 ppm (d,1H), 6,34 ppm, (s,1H), 3,57-3,69 ppm (m,2H), 3,53 ppm (d,3H), 3,44-3,56 ppm, (m,2H), 1,56-1,70 ppm (m,6H);

- 3-[4-Chlor-2-fluor-5-(2-hydroxyäthoxy)-phenyl]-1-methyl-6-trifluormethyl-2,4(1H,3H)-pyrimidindion mit N-Chlorcarbonyl-piperidin und Triäthylamin in Toluol das 2-{2-Chlor-5-[3,6-dihydro-2,6-dioxo-3-methyl-4-trifluormethyl-1(2H)-pyrimidinyl]-4-fluorphenoxy}-äthyl]-1-piperidincarboxylat, $^1$H-NMR ($CDCl_3$, 400 MHz): 7,32 ppm (d,1H), 6,82 ppm (d,1H), 6,37 ppm (s,1H), 4,44 ppm (m,2H), 4,19 ppm (m,2H), 3,56 ppm (d,3H), 3,36-3,46 ppm (m,4H), 1,45-1,63 ppm (m,6H);

- 3-(4-Chlor-2-fluor-5-hydroxyphenyl)-1-methyl-6-trifluormethyl-2,4(1H,3H)-pyrimidindion mit Phenylacetylchlorid und Pyridin in Diäthyläther das {2-Chlor-5-[3,6-dihydro-2,6-dioxo-3-methyl-4-trifluormethyl-1(2H)-pyrimidinyl]-4-fluorphenyl}-phenylacetat, $^1$H-NMR ($CDCl_3$, 400 MHz): 7,27-7,40 ppm (m,6H), 7,10 ppm (d,1H), 6,33 ppm (s,1H), 3,89 ppm (s,2H), 3,53 ppm (d,3H);

- Phenylmethansulfochlorid und Triäthylamin in Diäthyläther das {2-chlor-5-[3,6-dihydro-2,6-dioxo-3-methyl-4-trifluormethyl-1(2H)-pyrimidinyl]-4-fluorphenyl}-phenylmethansulfonat, $^1$H-NMR ($CDCl_3$, 400 MHz): 7,37-7,50 ppm (m,5H), 7,37 ppm (d,1H), 7,23 ppm (d,1H), 6,34 ppm (s,1H), 4,61 ppm (s,2H), 3,54 ppm (d,3H);

- Cyclohexylacetylchlorid und Pyridin in Diäthyläther das {2-Chlor-5-[3,6-dihydro-2,6-dioxo-3-methyl-4-trifluormethyl-1(2H)-pyrimidinyl]-4-fluorphenyl}-cyclohexylacetat, $^1$H-NMR ($CDCl_3$, 400 MHz): 7,36 ppm (d,1H), 7,13 ppm (d,1H), 6,35 ppm (s,1H), 3,55 ppm (d,3H), 2,47 ppm (d,2H), 1,60-2,00 ppm (m,6H), 0,99-1,37 ppm (m,5H);

- Nicotinsäurechlorid-hydrochlorid und Pyridin in Diäthyläther das {2-Chlor-5-[3,6-dihydro-2,6-dioxo-3-methyl-4-trifluormethyl-1(2H)-pyrimidinyl]-4-fluorphenyl}-3-pyridincarboxylat, $^1$H-NMR ($CDCl_3$, 400 MHz):

9,41 ppm (s,1H), 8,89 ppm (d,1H), 8,46 ppm (d,1H), 7,50 ppm (q,1H), 7,44 ppm (d,1H), 7,35 ppm (d,1H), 6,37 ppm (s,1H), 3,57 ppm (d,3H);

- 2-Phenyläthylbromid und Natriumcarbonat in Dimethylformamid das 3-[4-Chlor-2-fluor-5-(2-phenyläthoxy)-phenyl]-1-methyl-6-trifluormethyl-2,4(1H,3H)-pyrimidindion, $^1$H-NMR ($CDCl_3$, 400 MHz): 7,20-7,34 ppm (m,6H), 6,74 ppm (d,1H), 6,34 ppm (s,1H), 4,15 ppm (t,2H), 3,54 ppm (d,3H), 3,14 ppm (t,2H);

- 2-Thienylacetylchlorid und Pyridin in Methylenchlorid das {2-Chlor-5-[3,6-dihydro-2,6-dioxo-3-methyl-4-trifluormethyl-1(2H)-pyrimidinyl]-4-fluorphenyl}-2-thienylacetat, $^1$H-NMR ($CDCl_3$, 400 MHz): 7,36 ppm (d,1H), 7,26 ppm (q,1H), 7,15 ppm (d,1H), 7,03-7,07 ppm (m,1H), 6,99 ppm (q,1H), 6,34 ppm (s,1H), 4,12 ppm (d,2H), 3,54 ppm (d,3H);

- 3-[4-Chlor-2-fluor-5-(2-hydroxyäthoxy)-phenyl]-1-methyl-6-trifluormethyl-2,4(1H,3H)-pyrimidindion mit N,N-Dimethylcarbamoylchlorid und Triäthylamin in Toluol das 2-[2-Chlor-5-[3,6-dihydro-2,6-dioxo-3-methyl-4-trifluormethyl-1(2H)-pyrimidinyl]-4-fluorphenoxy]-äthyl-N,N-dimethylcarbamat, $^1$H-NMR ($CDCl_3$, 400 MHz): 7,31 ppm (d,1H), 6,81 ppm (d,1H), 6,36 ppm (s,1H), 4,40-4,47 ppm (m,2H), 4,16-4,22 ppm (m,2H), 3,56 ppm (d,3H), 2,92 ppm (s,3H), 2,89 ppm (s,3H);

- 3-(4-Chlor-2-fluor-5-hydroxyphenyl)-1-methyl-6-trifluormethyl-2,4(1H,3H)-pyrimidindion mit 3-Phenylpropylbromid und Natriumcarbonat in Dimethylformamid das 3-[4-Chlor-2-fluor-5-(3-phenylpropoxy)-phenyl]-1-methyl-6-trifluormethyl-2,4(1H,3H)-pyrimidindion, $^1$H-NMR ($CDCl_3$, 400 MHz): 7,15-7,34 ppm (m,6H), 6,71 ppm (d,1H), 6,36 ppm (s,1H), 3,96 ppm (t,2H), 3,55 ppm (d,3H), 2,83 ppm (t,2H), 2,10-2,20 ppm (m,2H);

- 1-Chlorsulfonylpiperidin mit Natriumhydrid in Dimethylformamid das {2-Chlor-5-[3,6-dihydro-2,6-dioxo-3-methyl-4-trifluormethyl-1(2H)-pyrimidinyl]-4-fluorphenyl}-1-piperidinsulfonat, $^1$H-NMR ($CDCl_3$, 400 MHz): 7,51 ppm (d,1H), 7,37 ppm (d,1H), 6,35 ppm (s,1H), 3,55 ppm (d,3H), 3,40-3,48 ppm (m,4H), 1,52-1,72 ppm (m,6H);

- N-Aethyl-N-cyclohexylsulfamoylchlorid und Natriumhydrid in Dimethylformamid das {2-Chlor-5-[3,6-dihydro-2,6-dioxo-3-methyl-4-trifluormethyl-1(2H)-pyrimidinyl]-4-fluorphenyl}-N-äthyl-N-cyclohexylsulfamat, $^1$H-NMR ($CDCl_3$, 400 MHz): 7,52 ppm (d,1H), 7,36 ppm (d,1H), 6,36 ppm (s,1H), 3,71-3,81 ppm (m,1H), 3,56 ppm (d,3H), 3,40 ppm (q,2H), 1,94-2,04 ppm (m,2H), 1,79-1,89 ppm (m,2H), 1,61-1,71 ppm (m,1H), 1,43-1,55 ppm (m,2H), 1,28-1,41 ppm (m,2H), 1,29 ppm (t,3H), 1,03-1,16 ppm (m,1H);

- 3-Chlorcarbonyl-1-methylpiperidin-hydrochlorid und Natriumhydrid in Dimethylformamid das {2-Chlor-5-[3,6-dihydro-2,6-dioxo-3-methyl-4-trifluormethyl-1(2H)-pyrimidinyl]-4-fluorphenyl}-1-methyl-3-piperidincarboxylat, $^1$H-NMR ($CDCl_3$, 400 MHz): 7,36 ppm, (d,1H), 7,15 ppm (d,1H), 6,35 ppm (s,1H), 3,55 ppm (d,3H), 3,05-3,13 ppm (m,1H), 2,86-2,96 ppm (m,1H), 2,71-2,80 ppm (m,1H), 2,24-2,36 ppm (m,4H), 1,98-2,15 ppm (m,2H), 1,76-1,86 ppm (m,1H), 1,51-1,74 ppm (m,2H);

- Bromessigsäure-äthylester und Natriumhydrid in Dimethylformamid das Aethyl-{2-chlor-5-[3,6-dihydro-2,6-dioxo-3-methyl-4-trifluormethyl-1(2H)-pyrimidinyl]-4-fluorphenoxy}acetat, Smp. 98-101°C;

- Chloracetylchlorid und Natriumhydrid in Dimethylformamid das {2-Chlor-5-[3,6-dihydro-2,6-dioxo-3-methyl-4-trifluormethyl-1(2H)-pyrimidinyl]-4-fluorphenyl}-chloracetat, Smp. 89-92°C;

- Cyanacetylchlorid in Benzol das {2-Chlor-5-[3,6-dihydro-2,6-dioxo-3-methyl-4-trifluormethyl-1(2H)-pyrimidinyl]-4-fluorphenyl}-cyanacetat, Smp. 157-160°C;

- 4-Chlor-2-oxo-1,3-dioxolan und Natriumhydrid in Dimethylformamid das 3-{4-Chlor-2-fluor-5-[(2-oxo-1,3-dioxolan-4-yl)oxy]-phenyl}-1-methyl-6-trifluormethyl-2,4(1H,3H)-pyrimidindion, Smp. 165-167°C;

- 4-/Chlormethyl-3,5-dimethylisoxazol und Natriumhydrid in Dimethylformamid das 3-{4-Chlor-5-[(3,5-dimethyl-4-isoxazolyl)methoxy-2-fluorphenyl}-1-methyl-6-trifluormethyl-2,4(1H,3H)-pyrimidindion, Smp. 120-123°C;

- 2-Brommethylthiophen und Natriumhydrid in Dimethylformamid das 3-{4-Chlor-2-fluor-5-[(2-thienyl)-methoxy]phenyl}-1-methyl-6-trifluomethyl-2,4(1H,3H)-pyrimidindion, Smp. 156-158°C;

- 4-Chlormethyl-2,6-diäthoxypyrimidin und Natriumhydrid in Dimethylformamid das 3-{4-Chlor-5-[(2,6-diäthoxy-4-pyrimidinyl)methoxy]-2-fluorphenyl}-1-methyl-6-trifluormethyl-2,4(1H,3H)-pyrimidindion, $^1$H-NMR ($CDCl_3$, 400 MHz): 7,36 ppm (d,1H), 6,81 ppm (d,1H), 6,66 ppm (s,1H), 6,36 ppm (s,1H), 4,99 ppm (d,2H), 4,42 ppm (q,2H), 4,38 ppm (q,2H), 3,55 ppm (d,3H), 1,41 ppm (t,3H), 1,40 ppm (t,3H);

- 2-Brommethylfuran und Natriumhydrid in Dimethylformamid das 3-[4-Chlor-2-fluor-5-(2-furfuryl)-phenyl]-1-methyl-6-trifluormethyl-2,4(1H,3H)-pyrimidindion, Smp. 145-147°C;

- 4-Chlormethyl-2-methylthiazol-hydrochlorid und Natriumhydrid in Dimethylformamid das 3-{4-Chlor-2-fluor-5-[(2-methyl-4-thiazolyl)methoxy]-phenyl}-1-methyl-6-trifluormethyl-2,4(1H,3H)-pyrimidindion, Smp. 145-147°C;

- 2-Brom-γ-butyrolacton und Natriumhydrid in Dimethylformamid das 3-{4-Chlor-2-fluor-5-[(tetrahydro-2-oxo-3-furyl)oxy]-phenyl}-1-methyl-6-trifluormethyl-2,4(1H,3H)-pyrimidindion, Smp. 174-176°C,

- 3-(4-Chlor-2-fluor-5-hydroxyphenyl)-1-methyl-6-trifluormethyl-2,4(1H,3H)-pyrimidindion mit Diallylcarbamoylchlorid und Natriumhydrid in Dimethylformamid das 2-Chlor-5-[3,6-dihydro-2,6-dioxo-3-methyl-4-trifluormethyl-1(2H)-pyrimidinyl]-4-fluorphenyl}-N,N-diallylcarbamat, $^1$H-NMR ($CDCl_3$, 400 MHz): 7,34 ppm (d,1H), 7,27 ppm (d,1H), 6,34 ppm (s,1H), 5,75-5,94 ppm (m,2H), 5,17-5,27 ppm (m,4H), 4,04 ppm (d,2H), 3,96 ppm (d,2H), 3,54 pm (d,3H);

- o-Methylbenzylchlorid und Natriumhydrid in Dimethylformamid das 3-{4-Chlor-2-fluor-5-[(o-methylbenzyl)oxy]-phenyl}-1-methyl-6-trifluormethyl-2,4(1H,3H)-pyrimidindion, $^1$H-NMR ($CDCl_3$, 400 MHz): 7,40-7,45 ppm (m,1H), 7,33 ppm (d,1H), 7,19-7,30 ppm (m,3H), 6,90 pm (d,1H), 6,37 ppm (s,1H), 5,03 ppm (s,2H), 3,56 ppm (d,3H), 2,37 ppm (s,3H);
- Bernsteinsäure-monomethylesterchlorid und Pyridin in Methylenchlorid das {2-Chlor-5-[3,6-dihydro-2,6-dioxo-3-methyl-4-trifluormethyl-1(2H)-pyrimidinyl]-4-fluorphenyl}-methyl-succinat, $^1$H-NMR ($CDCl_3$, 400 MHz): 7,36 ppm (d,1H), 7,17 pm (d,1H), 6,35 ppm (s,1H), 3,72 ppm (s,3H), 3,55 ppm (d,3H), 2,94 ppm (q,2H), 2,76 ppm (q,2H);
- p-tert.-Butylbenzylbromid und Natriumhydrid in Dimethlformamid das 3-{5-[(p-tert.-Butylbenzyl)oxy]-4-chlor-2-fluorphenyl}-1-methyl-6-trifluormethyl-2,4(1H,3H)-pyrimidindion, Smp. 156-158°C;
- 2-(2-Bromäthyl)-1,3-dioxolan und Natriumhydrid in Dimethylformamid das 3-{4-Chlor-5-[2-(1,3-dioxolan-2-yl)-äthoxy]-2-fluorphenyl}-1-methyl-6-trifluormethyl-2,4(1H,3H)-pyrimidindion, Smp. 125-127°C;
- 2-(2-Bromäthyl)-1,3-dioxan und Natriumhydrid in Dimethylformamid das 3-{4-Chlor-5-[2-(1,3-dioxan-2-yl)-2,4(1H,3H)-pyrimidindion, Smp. 128-130°C;
- o-Fluorbenzylchlorid und Natriumhydrid in Dimethylformamid das 3-{4-Chlor-5-[(o-fluorbenzyl)oxy]-2-fluorphenyl}-1-methyl-6-trifluormethyl-2,4(1H,3H)-pyrimidindion, Smp. 126-128°C;
- 2,6-Dichlorbenzylbromid und Natriumhydrid in Dimethylformamid das 3-{4-Chlor-5-[(2,6-dichlorbenzyl)-oxy]-2-fluorphenyl}-1-methyl-6-trifluormethyl-2,4(1H,3H)-pyrimidindion, $^1$H-NMR ($CDCl_3$, 400 MHz): 7,23-7,40 ppm (m,4H), 7,01 ppm (d,1H), 6,38 ppm (s,1H), 5,29 ppm (s,2H), 3,58 ppm (d,3H);
- 2,3,4,5,6-Pentafluorbenzylbromid und Natriumhydrid in Dimethylformamid das 3-{4-Chlor-2-fluor-5-[-(2,3,4,5,6-pentafluorbenzyl)oxy]phenyl}-1-methyl-6-trifluormethyl-2,4(1H,3H)-pyrimidindion, $^1$H-NMR ($CDCl_3$, 400 MHz): 7,33 ppm (d,1H), 6,94 ppm (d,1H), 6,38 ppm (s,1H), 5,14 ppm (t,2H), 3,57 ppm (d,3H);
- 3,4-Dichlorbenzylchlorid und Natriumhydrid in Dimethylformamid das 3-{4-Chlor-5-[(3,4-dichlorbenzyl)-oxy]-2-fluorphenyl}-1-methyl-6-trifluormethyl-2,4(1H,3H)-pyrimidindion, Smp. 173-175°C;
- o-Chlorbenzylchlorid und Natriumhydrid in Dimethylformamid das 3-{4-Chlor-5-[(o-chlorbenzyl)oxy]-2-fluorphenyl}-1-methyl-6-trifluormethyl-2,4(1H,3H)-pyrimidindion, Smp. 126-129°C;
- p-Nitrobenzylbromid und Natriumhydrid in Dimethylformamid das 3-{4-Chlor-2-fluor-5-[(4-nitrobenzyl)-oxy]-phenyl}-1-methyl-6-trifluormethyl-2,4(1H,3H)-pyrimidindion, Smp. 147-149°C;
- 2,4-Dichlorbenzylchlorid und Natriumhydrid in Dimethylformamid das 3-{4-Chlor-5-[(2,4-dichlorbenzyl)-oxy]-2-fluorphenyl}-1-methyl-6-trifluormethyl-2,4(1H,3H)-pyrimidindion, Smp. 112-120°C;
- m-Fluorbenzylchlorid und Natriumhydrid in Dimethylformamid das 3-{4-Chlor-5-[(m-fluorbenzyl)oxy]-2-fluorphenyl}-1-methyl-6-trifluormethyl-2,4(1H,3H)-pyrimidindion, Smp. 135-137°C;
- p-Fluorbenzylchlorid und Natriumhydrid in Dimethylformamid das 3-{4-Chlor-5-[(p-fluorbenzyl)oxy]-2-fluorphenyl}-1-methyl-6-trifluormethyl-2,4(1H,3H)-pyrimidindion, Smp. 174-177°C;
- p-Trifluormethyl-benzylbromid und Natriumhydrid in Dimethylformamid das 3-[4-Chlor-2-fluor-5-(p-trifluormethyl-benzyloxy)-phenyl]-1-methyl-6-trifluormethyl-2,4(1H,3H)-pyrimidindion, Smp. 158-159°C;
- 1-Brom-2-[2-(2-methoxyäthoxy)-äthoxy]-äthan und Natriumhydrid in Dimethylformamid das 3-[4-Chlor-2-fluor-5-{2-[2-(2-methoxyäthoxy)-äthoxy]-äthoxy}-phenyl]-1-methyl-6-trifluormethyl-2,4(1H,3H)-pyrimidindion, $^1$H-NMR ($CDCl_3$, 400 MHz): 7,30 ppm (d,1H), 6,84 ppm (d,1H), 6,36 ppm (s,1H), 4,12-4,17 ppm (m,2H), 3,85-3,90 ppm (m,2H), 3,73-3,78 ppm (m, 2H), 3,62-3,69 ppm (m,4H), 3,52-3,58 ppm (m,5H), 3,37 ppm (s,3H);
- 1-Brom-2-isopropoxyäthan und Natriumhydrid in Dimethylformamid das 3-{4-Chlor-2-fluor-5-[2-(isopropoxy)-äthoxy]-phenyl}-1-methyl-6-trifluormethyl-2,4(1H,3H)-pyrimidindion, $^1$H-NMR ($CDCl_3$, 400 MHz): 7,30 ppm (d,1H), 6,86 ppm (d,1H), 6,36 ppm (s,1H), 4,12 ppm (t,2H), 3,80 ppm (m,2H), 3,71 ppm (m,1H), 3,56 ppm (d,3H), 1,18 ppm (d,6H);
- 2-Brom-1-methoxypropan und Natriumhydrid in Dimethylformamid das 3-[4-Chlor-2-fluor-5-(2-methoxy-1-methyläthoxy)-phenyl]-1-methyl-6-trifluormethyl-2,4(1H,3H)-pyrimidindion, Smp. 80-82°C;
- Methoxyacetylchlorid und Pyridin in Methylenchlorid das {2-Chlor-5-[3,6-dihydro-2,6-dioxo-3-methyl-4-trifluormethyl-1(2H)-pyrimidinyl]-4-fluorphenyl}-methoxyacetat, Smp. 101-103°C.

Beispiel 9

Zu einer Lösung von 2,18 g N,N'-Dicyclohexylcarbodiimid in 10 ml Methylenchlorid wird unter Rühren bei 0°C während 5 Minuten eine Lösung von 0,68 g Propiolsäure in 5 ml Methylenchlorid zugetropft, und das Gemisch wird 15 Minuten bei 0°C nachgerührt. Anschliessend wird eine Lösung von 1,5 g 3-(4-chlor-2-fluor-5-hydroxyphenyl)-1-methyl -6-trifluormethyl-2,4(1H,3H)-pyrimidindion in 10 ml Methylenchlorid unter Rühren während 20 Minuten zugetropft und 2 Stunden bei 0°C nachgerührt. Unlösliche Anteile werden abgenutscht, und man dampft das Filtrat unter vermindertem Druck zur Trockene ein. Der Rückstand wird an einer Kieselgel-Säule unter Verwendung von Essigsäure-äthylester/n-Hexan (1:7) als Laufmittel chromatographisch gereinigt. Man erhält das {2-Chlor-5-[3,6-dihydro-2,6-dioxo-3-methyl-4-trifluormethyl-1-(2H)-pyrimidinyl]-4-fluorphenyl}propiolat, $^1$H-NMR ($CDCl_3$, 400 MHz): 7,40 ppm (d,1H), 7,21 ppm (d,1H), 6,36 ppm (s,1H), 3,56 ppm (d,3H), 3,15 ppm (s,1H).

In analoger Weise erhält man beim Einsatz von:

- 3-(4-Chlor-2-fluor-5-hydroxyphenyl)-1-methyl-6-trifluormethyl-2,4(1H,3H)-pyrimidindion und 3-[Piperidino-(carbonyl)]-propionsäure mit N,N'-Dicyclohexylcarbodiimid und 4-Dimethylaminopyridin als Katalysator in Methylenchlorid das {2-Chlor-5-[3,6-dihydro-2,6-dioxo-3-methyl-4-trifluormethyl-1(2H)-pyrimidinyl]-4-fluorphenyl}-3-[piperidino(carbonyl)]propionat, $^1$H-NMR ($CDCl_3$, 400 MHz): 7,35 ppm (d,1H), 7,20 ppm (d,1H), 6,35 ppm (s,1H), 3,50-3,60 ppm (m,5H), 3,42 ppm (t,2H), 2,94 ppm (t,2H), 2,74 ppm (t,2H), 1,49-1,70 ppm (m,6H);
- 3-(4-Chlor-2-fluor-5-hydroxyphenyl)-1-methyl-6-trifluormethyl-2,4(1H,3H)-pyrimidindion und Dithiopropionsäure mit N,N'-Dicyclohexylcarbodiimid und 4-Dimethylaminopyridin als Katalysator in Methylenchlorid das 3-[4-Chlor-2-fluor-5-(thiopropionyloxy)-phenyl]-1-methyl-6-trifluormethyl-2,4(1H,3H)-pyrimidindion, $^1$H-NMR ($CDCl_3$, 400 MHz): 7,39 ppm (d,1H), 7,05 ppm (d,1H), 6,36 ppm (s,1H), 3,56 ppm (d,3H), 2,95 ppm (q,2H), 1,40 ppm (t,3H);
- 3-(4-Chlor-2-fluor-5-hydroxyphenyl)-1-methyl-6-trifluormethyl-2,4(1H,3H)-pyrimidindion und Bernsteinsäure-mono-2-chloräthylester mit N,N'-Dicyclohexylcarbodiimid und 4-Dimethylaminopyridin als Katalysator in Methylenchlorid das {2-Chlor-5-[3,6-dihycro-2,6-dioxo-3-methyl-4-trifluormethyl-1(2H)-pyrimidinyl]-4-fluorphenyl}-(2-chloräthyl)-succinat, Smp. 94-96°C;
- 3-(4-Chlor-2-fluor-5-hydroxyphenyl)-1-methyl-6-trifluormethyl-2,4(1H,3H)-pyrimidindion und 3-(diäthylcarbamoyl)-propionsäure mit N,N'-Dicyclohexylcarbodiimid und 4-Pyrrolidino-pyridin als Katalysator in Methylenchlorid das {2-Chlor-5-[3,6-dihydro-2,6-dioxo-3-methyl-4-trifluormethyl-1(2H)-pyrimidinyl]-4-fluorphenyl}-3-(diäthylcarbamoyl)propionat, Smp. 91-93°C.

Beispiel 10

Zu einer Lösung von 2,00 g 3-(4-Chlor-2-fluor-5-hydroxyphenyl)-1-methyl-6-trifluormethyl -2,4(1H,3H)-pyrimidindion in 20 ml Methylenchlorid wird unter Rühren bei Raumtemperatur 0,51 g Methylisocyanat und anschliessend ein Tropfen Triäthylamin zugefügt. Das Reaktionsgemisch wird eine Stunde nachgerührt und unter vermindertem Druck zur Trockene eingedampft. Der Rückstand wird an einer Kieselgel-Säule unter Verwendung von Diäthyläther/n-Hexan (2:1) als Laufmittel chromatographisch gereinigt. Man erhält das {2-Chlor-5-[3,6-dihydro-2,6-dioxo-3-methyl-4-trifluormethyl-1(2H)-pyrimidinyl]-4-fluorphenyl}-methylcarbamat, $^1$H-NMR ($CDCl_3$, 400 MHz): 7,33 ppm (d,1H), 7,24 ppm (d,1H), 6,34 pm (s,1H), 5,21 ppm (q,1H), 3,54 ppm (d,3H), 2,88 ppm (d,3H).

In analoger Weise erhält man beim Einsatz von:

- Isopropylisocyanat mit Triäthylamin in Methylenchlorid das {2-Chlor-5-[3,6-dihydro-2,6-dioxo-3-methyl-4-tri fluormethyl -1(2H)-pyrimidinyl]-4-fluorphenyl}-isopropylcarbamat, $^1$H-NMR ($CDCl_3$, 400 MHz): 7,33 ppm (d,1H), 7,25 ppm (d,1H), 6,35 ppm (s,1H), 5,04 ppm (d,1H), 3,88 pm (m,1H), 3,54 ppm (d,3H), 1,23 ppm (d,6H);
- tert.-Butylisocyanat mit Triäthylamin in Methylenchlorid das {2-Chlor-5-[3,6-dihydro-2,6-dioxo-3-methyl-4-trifluormethyl -1(2H)-pyrimidinyl]-4-fluorphenyl}-tert.-butylcarbamat, $^1$H-NMR ($CDCl_3$, MHz): 7,33 ppm (d,1H), 7,25 ppm (d,1H), 6,34 ppm (s,1H), 5,16 ppm (s,1H), 3,54 ppm (d,3H), 1,38 ppm (s,9H);
- Cyclohexylisocyanat mit Triäthylamin in Methylenchlorid das {2-Chlor-5-[3,6-dihydro-2,6-dioxo-3-methyl-4-trifluormethyl -1(2H)-pyrimidinyl]-4-fluorphenyl}-cyclohexylcarbamat, Smp. 171-173°C;
- Allylisocyanat mit Triäthylamin in Methylenchlorid das {2-Chlor-5-[3,6-dihydro-2,6-dioxo-3-methyl-4-trifluormethyl-1(2H)-pyrimidinyl]-4-fluorphenyl}allylcarbamat, Smp. 98-100°C;
- 3-Trifluormethylphenylisocyanat mit Triäthylamin in Methylenchlorid das {2-Chlor-5-[3,6-dihydro-2,6-dioxo-3-methyl-4-trifluormethyl -1(2H)-pyrimidinyl]-4-fluorphenyl} -3-trifluormethylphenylcarbamat, Smp.

153-155°C;

- Propargylisocyanat mit Triäthylamin in Methylenchlorid das {2-Chlor-4-fluor-5-[3,6-dihydro-2,6-dioxo-3-methyl-4-trifluormethyl-1(2H)-pyrimidinyl]phenyl}-2-propinylcarbamat, Smp. 144-146°C;
- 3-[4-Chlor-2-fluor-5-(2-hydroxyäthoxy)-phenyl]-1-methyl-6-trifluormethyl-2,4(1H,3H)-pyrimidindion und Methylisocyanat mit Pyridin in Diäthyläther das {2-[2-Chlor-5-(3,6-dihydro-2,6-dioxo-3-methyl-4-trifluormethyl-1(2H)-pyrimi dinyl]-4-fluorphenoxy]-äthyl}-methylcarbamat, Smp. 173-175°C;
- 3-(4-Chlor-2-fluor-5-hydroxyphenyl)-1-methyl-6-trifluormethyl-2,4(1H,3H)-pyrimidindion mit Cyclohexylmethylisocyanat und Triäthylamin in Methylenchlorid das {2-Chlor-5-[3,6-dihydro-2,6-dioxo-3-methyl-4-trifluormethyl-1(2H)-pyrimidinyl]-4-fluorphenyl}-cyclohexylmethylcarbamat, Smp. 132-135°C;
- Benzylisocyanat und Triäthylamin in Diäthyläther das {2-Chlor-5-[3,6-dihydro-2,6-dioxo-3-methyl-2-trifluormethyl-1(2H)-pyrimidinyl]-4-fluorphenyl}-benzylcarbamat, Smp. 147-150°C.

Beispiel 11

Analog Beispiel 1 kann 3-Amino-2-chlorcrotonsäure-äthylester und 4-Chlor-2-fluor-5-isopropoxyphenylisocyanat zu dem 5-Chlor-3-(4-chlor-2-fluor-5-isopropoxyphenyl)-6-methyl -2,4(1H,3H)-pyrimidindion umgesetzt werden.

Durch Alkylierung analog Beispiel 6 erhält man aus obigem Produkt mit Natriumhydrid und Dimethylsufat in Dimethylformamid das 5-chlor-3-(4-chlor-2-fluor-5-isopropoxyphenyl)-1,6 -dimethyl-2,4(1H,3H)-pyrimidindion, Smp. 182-184°C.

Dasselbe Produkt erhält man auch aus 3-(4-Chlor-2-fluor-5-isopropoxyphenyl)-1,6-dimethyl -2,4(1H,3H)-pyrimidindion durch Chlorierung mit Sulfurylchlorid in Essigsäure.

Beispiel 12

Eine Lösung von 10,5 g 3-Amino-4,4,4-trifluorcrotonsäure-äthylester in 25 ml absolutem Dimethylformamid wird unter Rühren bei 0°C während 5 Mintuen zu einer Suspension von 2,5 g einer 55%igen Natriumhydrid-Dispersion in 25 ml absoluten Dimethylformamid zugetropft. Die Temperatur steigt auf 20°C an, anschliessend wird 30 Minuten bei dieser Temperatur nachgerührt. Danach wird 14,4 g Aethyl-N-(4-chlor-2-fluor-5-isopropoxyphenyl)-carbamat zugefügt, 30 Minuten bei Raumtemperatur nachgerührt und 2,5 Stunden auf 150°C (Badtemperatur) erhitzt. Das Reaktionsgemisch wird auf 50°C abgekühlt, mit 3,5 ml konzentrierter Essigsäure neutralisiert und unter vermindertem Druck bei 60°C weitgehend eingeengt. Der Rückstand wird auf eine Lösung von 200 ml Wasser und 30 ml 2N Salzsäure gegossen und das wässrige Gemisch zweimal mit je 150 ml Diäthyläther extrahiert, und die organischen Phasen werden zweimal mit je 50 ml Wasser gewaschen, über wasserfreiem Natriumsulfat getrocknet und unter vermindertem Druck zur Trockene eingedampft. Der Rückstand wird aus Diäthyläther/n-Hexan umkristallisiert. Man erhält das 3-(4-Chlor-2-fluor-5-isopropoxyphenyl)-6-trifluormethyl-2,4(1H,3H)-pyrimidindion, Smp. 195-197°C.

Beispiel 13

Eine Lösung von 1,0 g 3-Chlor-perbenzoesäure in 15 ml Methylenchlorid wird unter Rühren bei 0°C während 1 Stunde zu einer Lösung von 2,3 g 1-[4-Chlor-2-fluor-5-(methylthio)-methoxy-phenyl]-3-methyl-4-trifluormethyl-2,6(1H,3H)-pyrimidindion in 30 ml Methylenchlorid zugetropft. Anschliessend wird das Reaktionsgemisch 15 Stunden bei Raumtemperatur nachgerührt, mit verdünnter wässriger Natriumhydrogencarbonatlösung, danach mit Wasser, gewaschen und über wasserfreiem Natriumsulfat getrocknet. Die organische Phase wird unter vermindergem Druck zur Trockene eingedampft und der Rückstand an einer Kieselgel-Säule unter Verwendung von Diäthyläther/Essigsäure-äthylester, danach Essigsäure-äthylester allein, als Laufmittel chromatographisch gereinigt. Man erhält das 1-[4-Chlor-2-fluor-5-(methylsulfinyl)-methoxy-phenyl]-3-methyl-4-trifluormethyl-2,6(1H,3H)-pyrimidindion, $^1$H-NMR ($CDCl_3$, 400 MHz): 7,33 ppm -(d,1H), 7,27 ppm (2d,1H), 6,34 ppm (2s,1H), 4,91-5,05 ppm (m,2H), 3,54 ppm (d,3H), 2,72 ppm (s,3H).

In analoger Weise erhält man beim Einsatz von:

- 1-[4-Chlor-2-fluor-5-(methylthio)methoxy-phenyl]-3-methyl-4-trifluormethyl-2,6(1H,3H)-pyrimidindion mit einem 0,5 molaren Ueberschuss an 3-Chlor-perbenzoesäure das 1-[4-Chlor-2-fluor-5-(methylsulfonyl)-methoxy-phenyl]-3-methyl-4-trifluormethyl-2,6(1H,3H)-pyrimidindion, Smp. 178-179°C;
- 3-[4-Chlor-2-fluor-5-(phenylthio)methoxy-phenyl]-1-methyl-6-trifluormethyl-2,4(1H,3H)-pyrimidindion mit

einem 0,15 molaren Ueberschuss an 3-Chlor-perbenzoesäure das 3-[4-Chlor-2-fluor-5-(phenylsulfinyl)-methoxy-phenyl]-1-methyl-6-trifluormethyl-2,4(1H,3H)-pyrimidindion, ¹H-NMR ($CDCl_3$, 400 MHz): 7,63-7,79 ppm (m,2H), 7,47-7,63 ppm (m,3H), 7,20-7,38 ppm (m,2H), 6,34 ppm (2s,1H), 4,84-5,05 ppm (m,2H), 3,55 ppm (d,3H);

- 3-[4-Chlor-2-fluor-5-(phenylthio)methoxy-phenyl]-1-methyl-6-trifluormethyl-2,4(1H,3H)-pyrimidindion mit einem 0,3 molaren Ueberschuss an 3-Chlor-perbenzoesäure das 3-[4-Chlor-2-fluor-5-(phenylsulfonyl)-methoxy-phenyl]-1-methyl-6-trifluormethyl-2,4(1H,3H)-pyrimidindion, ¹H-NMR ($CDCl_3$, 400 MHz): 7,95-8,06 ppm (m,2H), 7,65-7,72 ppm (m,1H), 7,52-7,65 ppm (m,2H), 7,27 ppm (d,1H), 7,16 ppm (d,1H), 6,33 ppm (s,1H), 5,06 ppm (s,2H), 3,54 ppm (d,3H).

Beispiel 14

Eine Lösimg von 0,51 g Essigsäureanhydrid und 0,23 g Ameisensäure in 10 ml Methylenchlorid wird 2 Stunden auf Rückflusstemperatur erhitzt. Anschliessend wird 2 µl Pyridin zugefügt und das Reaktionsgemisch mit 1,5 g 3-(4-Chlor-2-fluor-5-hydroxyphenyl)-1-methyl-6-trifluor methyl-2,4(1H,3H)-pyrimidindion 1,5 Stunden bei Raumtemperatur gerührt. Das Reakfionsgemisch wird unter vermindertem Druck zur Trockene eingedampft und der Rückstand aus Diäthyläther/n-Hexan umkristallisiert. Man erhält das 2-Chlor-5-[3,6-dihydro-2,6-dioxo-3-methyl-4-trifluormethyl-1(2H)-pyrimidinyl]-4-fluor-phenylformiat, Smp. 97-99°C.

In analoger Weise erhält man beim Einsatz von:

- 3-[4-Chlor-2-fluor-5-(2-hydroxyäthoxy)-phenyl]-1-methyl-6-trifluormethyl-2,4(1H,3H)-pyrimidindion mit Pyridin in Diäthyläther das 2-{2-Chlor-5-[3,6-dihydro-2,6-dioxo-3-methyl-4-trifluormethyl-1(2H)-pyrimidinyl]-4-fluorphenoxy}-äthylformiat, ¹H-NMR ($CDCl_3$, 400 MHz): 8,11 ppm (t,1H), 7,33 ppm (d,1H), 6,82 ppm (d,1H), 6,35 ppm (s,1H), 4,54 ppm (m,2H), 4,22 ppm (t,2H), 3,56 ppm (d,3H).

Beispiel 15

Eine Lösung von 10,16 g 3-(4-Chlor-2-fluor-5-hydroxyphenyl)-1-methyl-6-trifluormethyl-2,4(1H,3H)-pyrimidindion und 20,20 g Sulfurylchlorid in 100 ml Methylenchlorid wird unter Rühren und Kühlen während 5 Minuten mit einer Lösung von 3,56 g Pyridin in 10 ml Methylenchlorid versetzt. Die Temperatur steigt dabei auf 20°C an. Das Reaktionsgemisch wird 15 Minuten bei 20-25°C nachgerührt, anschliessend auf Eis gegossen und kräftig gerührt. Die organische Phase wird mit Wasser gründlich gewaschen, über wasserfreiem Natriumsulfat getrocknet und unter vermindertem Druck zur Trockene eingedampft. Der Rückstand wird aus Diäthyläther/n-Hexan umkristallisiert. Man erhält das {2-Chlor-5-[3,6-dihydro-2,6-dioxo-3-methyl-4-trifluormethyl-1(2H)-pyrimidinyl]-4-fluorphenyl}-chlorsulfat, Smp. 133-135°C.

Eine Lösung von 1,50 des Produktes der vorhergehenden Stufe in 20 ml Methylenchlorid wird unter Rühren auf -70°C abgekühlt und eine Lösung von 0,41 g Isopropylamin in 10 ml Methylenchlorid während 30 Minuten zugetropft und anschliessend 2 Stunden bei -70°C, danach 16 Stunden bei 20°C, nachgerührt. Das Reaktionsgemisch wird unter vermindertem Druck zur Trockene eingedampft und der Rückstand an einer Kieselgel-Säule unter Verwendung von Diäthyläther/n-Hexan (1:1) als Laufmittel chromatographisch gereinigt. Anschliessend wird das Produkt aus Diäthyläther/n-Hexan umkristallisiert. Man erhält das {2-Chlor-5-[3,6-dihydro-2,6-dioxo-3-methyl-4-trifluormethyl-1(2H)-pyrimidinyl]-4-fluorphenyl}-isopropylsulfamat, Smp. 145-146°C.

Beispiel 16

Ein Gemisch von 1,70 g 3-[5-(2-Aminoäthoxy)-4-chlor-2-fluorphenyl]-1-methyl-6-trifluormethyl-2,4-(1H,3H)-pyrimidindion, 0,98 g einer 36%-igen wässrigen Formaldehydlösung und 0,92 g Ameisensäure wird unter Rühren 3 Stunden auf 100°C erhitzt. Das Reaktionsgemisch wird auf 100 ml 2%-ige wässrige Natriumhydrogencarbonatlösung gegossen, die ölige Fällung in Essigsäure-äthylester gelöst und die Lösung mit Wasser gewaschen und über wasserfreiem Natriumsulfat getrocknet. Die Lösung wird unter vermindertem Druck zur Trockene eingedampft und der harzige Rückstand an einer Aluminiumoxid-Säule (neutral, Brockmann I) unter Verwendung von Essigsäure-äthylester als Laufmittel chromatographisch gereinigt. Man erhält das 3-{4-Chlor-5-[2-(dimethylamino)-äthoxy]-2-fluorphenyl}-1-methyl-6-trifluormethyl-2,4(1H,3H)-pyrimidindion, ¹H-NMR ($CDCl_3$, 400 MHz): 7,31 ppm (d,1H), 6,80 ppm (d,1H), 6,36 ppm (s,1H), 4,09 ppm (t,2H), 3,56 ppm (d,3H), 2,79 ppm (t,2H), 2,35 ppm (s,6H).

Beispiel 17

Ein Gemisch von 20,3 g 3-(4-Chlor-2-fluor-5-hydroxyphenyl)-1-methyl-6-trifluormethyl-2,4(1H,3H)-pyrimidindion und 8,9 g 2-Aethyl-Δ2-oxazolin wird 3 Stunden auf 140°C erhitzt. Das Reaktionsgemisch wird in 50 ml Essigsäure-äthylester gelöst, 200 ml Diäthyläther werden zugefügt, das Gemisch wird mit Wasser ausgeschüttelt und die organische Phase über wasserfreiem Natriumsulfat getrocknet. Die Lösung wird unter vermindertem Druck zur Trockene eingedampft und der harzige Rückstand an einer Kieselgel-Säule unter Verwendung von Diäthyläther/Essigsäure-äthylester (3:1) als Laufmittel chromatographisch gereinigt. Anschliessend wird das Produkt aus Diäthyläther umkristallisiert. Man erhält das N-[2-{2-Chlor-5-[3,6-dihydro-2,6-dioxo-3-methyl-4-trifluormethyl-1(2H)-pyrimidinyl]-4-fluorphenoxy}-äthyl]-propionamid, Smp. 100-103°C.

Beispiel 18

Ein Gemisch von 2,0 g 3-[5-(2-Aminoäthoxy)-4-chlor-2-fluorphenyl]-1-methyl-6-trifluormethyl-2,4(1H,3H)-pyrimidindion-hydrochloridin 20 ml Ameisensäure wird mit 0,47 g Triäthylamin 20 Stunden auf Siedetemperatur erhitzt. Anschliessend wird das Reaktionsgemisch unter vermindertem Druck zur Trockene eingedampft, der Rückstand in Essigsäure-äthylester gelöst und mit Wasser, danach mit wässriger Natriumhydrogencarbonatlösung, ausgeschüttelt und die organische Phase über wasserfreiem Natriumsulfat getrocknet. die Lösung wird danach unter vermindertem Druck zur Trockene eingedampft, der harzige Rückstand in Essigsäure-äthylester gelöst und weitgehend eingedampft. Der noch flüssige Rückstand wird bis zur leichten Trübung mit Diäthyläther versetzt und geimpft. Die Kristalle werden abgenutscht, mit Diäthyläther gewaschen und getrocknet. Man erhält das N-[2-{2-Chlor-5-[3,6-dihydro-2,6-dioxo-3-methyl-4-trifluormethyl-1(2H)-pyrimidinyl]-4-fluorphenoxy}-äthyl]-formamid, Smp. 128-131°C.

Beispiel 19

Eine Lösung von 17,0 g N-[2-{2-Chlor-5-[3,6-dihydro-2,6-dioxo-3-methyl-4-trifluormethyl-1(2H)-pyrimidinyl]-4-fluorphenoxy}-äthyl]-propionamid in 100 ml Aethanol wird mit einer Lösung von 75 ml konzentrierter Salzsäure in 75 ml Wasser 5 Stunden auf Rückflusstemperatur erhitzt. Anschliessend wird das Aethanol bei 50°C abdestilliert und die klare, wässrige Lösung mit Natriumhydrogencarbonat neutralisiert. Die ölige Fällung wird in Essigsäure-äthylester gelöst und die Lösung mit Wasser gewaschen, über wasserfreiem Natriumsulfat getrocknet und unter vermindertem Druck zur Trockene eingedampft. Der harzige Rückstand wird in 200 ml 2N Salzsäure bei 50°C aufgenommen, das Gemisch mit wenig Essigsäure-äthylester ausgeschüttelt und die wässrige Phase unter vermindertem Druck bis zur Kristallisation eingedampft. Anschliessend werden 50 ml Diäthyläther zugefügt, und das Gemisch wird gerührt. Die Kristalle werden abgenutscht, mit Diäthyläther gewaschen und im Vakuum bei 50°C getrocknet. Man erhält das 3-[5-(2-Aminoäthoxy)-4-chlor-2-fluorphenyl]-1-methyl-6-trifluormethyl-2,4(1H,3H)-pyrimidindion-hydrochlorid, Smp. 217-227°C (Zersetzung).

Beispiel 20

Eine Lösung von 2,0 g 3-(4-Chlor-2-fluor-5-hydroxyphenyl)-1-methyl-6-trifluormethyl-2,4(1H,3H)-pyrimidindion wird mit 0,25 g Kaliumcarbonat versetzt und die Suspension unter Rühren während 10 Minuten mit Chlortrifluoräthylen gesättigt. Anschliessend wird das Reaktionsgemisch 10 Minuten auf 50°C erhitzt, währenddessen man einen schwachen Strom Chlortrifluoräthylen einleitet. Danach wird das Reaktionsgemisch auf eine Lösung von 3 ml 2N Salzsäure in 250 ml Wasser gegossen und das Ganze mit 100 ml Essigsäure-äthylester ausgeschüttelt. Die organische Phase wird mit Wasser neutral gewaschen, über wasserfreiem Natriumsulfat getrocknet und unter vermindertem Druck zur Trockene eingedampft. Der harzige Rückstand wird an einer Kieselgel-Säule unter Verwendung von Diäthyläther/n-Hexan (1:3) als Laufmittel chromatographisch gereinigt. Man erhält das 3-[4-Chlor-5-(2-chlor-1,1,2-trifluoräthoxy)-2-fluorphenyl]-1-methyl-6-trifluormethyl-2,4(1H,3H)-pyrimidindion, $^{1}$H-NMR ($CDCl_3$, 400 MHz): 7,40 ppm (d,1H), 7,32 ppm (m,1H), 6,37 ppm (s,1H), 6,33 ppm (m,1H), 3,57 ppm (d,3H).

II. Herstellung der Verbindungen der Formeln III, XI, XII, XIII, XIV, XV und XVI:

Beispiel 21

Zu einer Lösung von 22,4 g 3-(4-Chlor-2-fluor-5-isopropoxyphenyl)-1-methyl -6-trifluormethyl-2,4-(1H,3H)-pyrimidindion in 50 ml Methylenchlorid werden unter Rühren und Kühlen bei Raumtemperatur 25 ml konzentrierte Schwefelsäure während 1 Minute zugetropft. Das Reaktionsgemisch wird 2 Stunden bei Raumtemperatur nachgerührt und auf 100 g Eis gegossen. Die organische Phase wird abgetrennt, zweimal mit je 50 ml Wasser gewaschen und über wasserfreiem Natriumsulfat getrocknet und unter vermindertem Druck zur Trockene eingedampft. Der Rückstand wird aus Diäthyläther/n-Hexan umkristallisiert. Man erhält das 3-(4-Chlor-2-fluor-5-hydroxyphenyl)-1-methyl -6-trifluormethyl-2,4(1H,3H)-pyrimidindion, Smp. 169-171°C.

In analoger Weise erhält man beim Einsatz von:

- 3-(4-Brom-2-fluor-5-isopropoxyphenyl)-1-methyl-6-trifluormethyl-2,4(1H,3H)-pyrimidindion das 3-(4-Brom-2-fluor-5-hydroxyphenyl)-1-methyl-6-trifluormethyl-2,4(1H,3H)-pyrimidindion, Smp. 192°C.

Beispiel 22

Eine Lösung von 4,67 g 3-Amino-4,4,4-trifluorcrotonsäure-äthylester in 15 ml Toluol wird unter Rühren bei 0°C während 20 Minuten zu einer Suspension von 1,11 g einer 55%igen Natriumhydrid-Dispersion in 30 ml absolutem Dimethylformamid zugetropft, und das Gemisch wird 15 Minuten bei 0°C nachgerührt. Anschliessend wird das Reaktionsgemisch auf -40°C abgekühlt und eine Lösung von 6,24 g (2-Chlor-4-fluor-5-isocyanatophenyl)methylcarbonat in 30 ml Toluol während 5 Minuten zugetropft. Das Reaktionsgemisch wird 1 Stunde bei Raumtemperatur nachgerührt und mit einer Lösung von 5,40 g Natriumcarbonat in 100 ml Wasser 30 Minuten gerührt. Die wässrige Phase wird abgetrennt, zweimal mit je 50 ml Diäthyläther ausgeschüttelt und mit 80 ml 2N Salzsäure auf pH 1 gestellt. Das Gemisch wird zweimal mit je 100 ml Diäthyläther ausgeschüttelt, und die organischen Phasen werden mit Wasser gewaschen, über wasserfreiem Natriumsulfat getrocknet und unter vermindertem Druck zur Trockene eingedampft. Der Rückstand wird aus Diäthyläther/n-Hexan umkristallisiert. Man erhält das 3-(4-Chlor-2-fluor-5-hydroxyphenyl)-6-trifluormethyl -2,4(1H,3H)-pyrimidindion, Smp. 223-225°C.

Beispiel 23

Einer Lösung von 22,2 g o-Fluoranilin in 25 ml Pyridin und 80 ml n-Hexan tropft man bei 0°C 22,8 g Chlorameisensäure-äthylester so zu, dass die Temperatur des Reaktionsgemisches 5°C nicht übersteigt. Nach Abfiltrieren des gebildeten Pyridinhydrochlorids wird das Filtrat eingeengt und der Rückstand unter vermindertem Druck getrocknet. Man erhält auf diese Weise den o-Fluorcarbanilsäure-äthylester, der ohne Reinigung in der nächsten Reaktionsstufe verwendet wird.

Mikroanalyse:

| | C% | H% | N% | F% |
|---|---|---|---|---|
| berechnet | 59,01 | 5,50 | 7,65 | 10,37 |
| gefunden | 59,12 | 5,67 | 7,55 | 10,30. |

5,5 g des obigen Zwischenproduktes sowie 5,4 g Natriumacetat werden in 25 ml Eisessig suspendiert. Die Suspension wird bei 50°C mit 4,8 ml Sulfurylchlorid versetzt und anschliessend das Reaktionsgemisch 30 Minuten auf dieser Temperatur gehalten. Dann giesst man das Gemisch auf 200 ml Eiswasser, wobei der gewünschte 4-Chlor-2-fluorcarbanilsäure-äthylester auskristallisiert. $^1$H-NMR ($CDCl_3$, 60 MHz: 8,1 ppm (t,1H), 7,1 ppm (d,2H), 6,8 ppm (breites s,1H), 4,25 ppm (q,2H), 1,3 ppm (t,3H).

Einer Suspension von 15,6 g Natriummethylat in 150 ml Dimethylformamid gibt man 51,0 g 3-amino-4,4,4-trifluorcrotonsäure-äthylester so zu, dass die Temperatur des Reaktionsgemisches 30°C nicht übersteigt. Man gibt der resultierenden gelben Suspension 60,9 g 4-Chlor-2-fluorcarbanilsäure-äthylester zu und erhitzt das Gemisch 2 Stunden auf 130°C unter ständigem Abdestillieren von Methanol und Aethanol. Dann wird das Gemisch auf 800 ml Eiswasser gegossen und dreimal mit je 100 ml Diäthyläther gewaschen.

Die wässrige Phase wird mit konzentrierter Salzsäure angesäuert und dreimal mit je 250 ml Essigester extrahiert, und anschliessend werden die vereinigten organischen Phasen der Reihe nach mit 5%iger wässriger Kochsalzlösung und Aethanol gewaschen, über wasserfreiem Natriumsulfat getrocknet und eingedampft. Das ölige Rohprodukt wird aus Diäthyläther/n-Hexan kristallisiert. Man erhält das 3-(4-Chlor-2-fluorphenyl)-6-trifluormethyl-2,4(1H,3H)-pyrimidindion, Smp. 189°C.

In analoger Weise erhält man beim Einsatz von o-Fluorcarbanilsäure-äthylester und Brom den 4-Brom-2-fluorcarbanilsäure-äthylester, Smp. 72-73°C, und aus diesem und 3-Amino-4,4,4-trifluorcrotonsäure-äthylester das 3-(4-Brom-2-fluorphenyl)-6-trifluormethyl-2,4(1H,3H)-pyrimidindion, Smp. 205°C.

In analoger Weise erhält man ausgehend von 4-Chlor-2-fluorcarbanilsäure-äthylester und 3-Aminocrotonsäure-äthylester das 3-(4-Chlor-2-fluorphenyl)-6-methyl-2,4(1H,3H)-pyrimidindion, Smp. >220°C; Massensspektrum (m/e): 254 ($M^+$), 235, 219, 171, 143, 108, 83, 68, 42;
Mikroanalyse:

| | C% | H% | N% | F% | Cl% |
|---|---|---|---|---|---|
| berechnet | 51,69 | 3,31 | 11,01 | 7,34 | 13,76 |
| gefunden | 51,88 | 3,17 | 11,00 | 7,46 | 13,92. |

Beispiel 24

Eine Suspension von 6,17 g 3-(4-Chlor-2-fluorphenyl)-6-trifluormethyl-2,4(1H,3H)-pyrimidindion, 5,5 g Kaliumcarbonat und 2,9 ml Dimethylsulfat in 40 ml Acetonitril wird 60 Minuten bei 55°C gerührt und anschliessend auf 150 ml Wasser gegossen. Die wässrige Lösung wird dreimal mit je 50 ml Aethylacetat extrahiert, und die vereinigten organischen Phasen werden über wasserfreiem Natriumsulfat getrocknet und eingedampft. Der resultierende ölige Rückstand wird aus Diäthyläther/n-Hexan kristallisiert. Man erhält so das 3-(4-Chlor-2-fluorphenyl)-methyl-6-trifluormethyl2,4(1H,3H)-pyrimidindion, Smp. 108°C.

9,7 g des Produktes der vorhergehenden Reaktionsstufe werden portionenweise zu einer auf -15°C gekühlten Lösung von 2,2 ml Salpetersäure (60%ig) und 25 ml Schwefelsäure gegeben. Man rührt das Reaktionsgemisch 300 Minuten bei Raumtemperatur und giesst es anschliessend auf 150 ml Eiswasser. Das Produkt, 3-(4-Chlor-2-fluor-5-nitrophenyl)-1-methyl-6-trifluormethyl-2,4(1H,3H)-pyrimidindion, kristallisiert aus und wird abfiltriert. Smp. 124°C.

Anstelle dieser Methylierung, gefolgt von der Nitrierung kann man die Nitrierung als erste Reaktionsstufe vornehmen, und zwar wie folgt:

Eine Nitrierlösung bestehend aus 2,2 ml Salpetersäure und 24 ml Schwefelsäure wird auf -15°C vorgekühlt. Zur Lösung gibt man portionenweise und unter Rühren 9,26 g 3-(4-Chlor-2-fluorphenyl)-6-trifluormethyl-2,4(1H,3H) -pyrimidindion zu. Es wird 30 Minuten bei 15°C, anschliessend 1 Stunde bei Raumtemperatur, nachgerührt. Danach giesst man das Gemisch auf 150 ml Eiswasser und filtriert den resultierenden kristallinen Niederschlag ab. Auf diese Weise erhält man das 3-(4-Chlor-2-fluor-5-nitrophenyl)-6-trifluormethyl-22,4(1H,3H)-pyrimidindion, Smp. 204°C.

49,5 g des Produktes der vorhergehenden Reaktionsstufe werden in 300 ml Acetonitril gelöst. Man gibt dann 38,7 g Kaliumcarbonat und 15,3 ml Dimethylsulfat zu und rührt das Reaktionsgemisch 2,5 Stunden bei 55°C. Anschliessend wird das Lösungsmittel abdestilliert und das zurückbleibende Gemisch dreimal mit je 100 ml Aethylacetat extrahiert. Man trocknet die vereinigten organischen Phasen über wasserfreiem Natriumsulfat, destilliert das Lösungsmittel ab und unterwirft den öligen Rückstand einer chromatographischen Reinigung unter Verwendung von 300 g Kieselgel sowie n-Hexan/Aethylacetat (4:1). Das Produkt, 3-(4-Chlor-2-fluor-5-nitrophenyl)-1-methyl-6-trifluormethyl-2,4(1H,3H)-pyrimidindion, kristallisiert aus n-Hexan/Aethylacetat.

In analoger Weise erhält man beim Einsatz von:

- 3-(4-Brom-2-fluorphenyl)-6-trifluormethyl-2,4(1H,3H)-pyrimidindion durch Methylierung das 3-(4Brom-2-fluorphenyl)-1-methyl-6-trifluormethyl-2,4(1H,3H)-pyrimidindion, und aus diesem durch Nitrierung das 3-(4-Brom-2-fluor-5-nitrophenyl)-1-methyl-6-trifluormethyl-2,4(1H,3H)-pyrimidindion, Smp. 124-125°C;
- 3-(4-Brom-2-fluorphenyl)-6-trifluormethyl-2,4(1H,3H)-pyrimidindion durch Nitrierung das 3-(4-Brom-2-fluor-5-nitrophenyl)-6-trifluormethyl-2,4(1H,3H)-pyrimidindion, und aus diesem durch Methylierung das 3-(4-Brom-2-fluor-5-nitrophenyl)-1-methyl-6-trifluormethyl-2,4(1H,3H)-pyrimidindion, Smp. 124-125°C;
- 3-(4-Chlor-2-fluorphenyl)-6-methyl-2,4(1H,3H)-pyrimidindion durch Methylierung das 3-(4-Chlor-2-fluor-

phenyl)-1,6-dimethyl-2,4(1H,3H)-pyrimidindion, Smp. 145°C, und aus diesem durch Nitrierung das 3-(4-Chlor-2-fluor-5-nitrophenyl)-1,6-dimethyl-2,4(1H,3H)-pyrimidindion, Smp. 165-172°C; $^1$H-NMR ($d_6$-DMSO, 60 MHz): 8,55 ppm (d,J-7Hz, 1H), 8,20 ppm (d,J = 10Hz,1H), 3,51 pm (s,3H), 2,52 ppm (s,3H);

- 3-(4-Chlor-2-fluorphenyl)-6-methyl-2,4(1H,3H)-pyrimidindion durch Nitrierung das 3-(4-Chlor-2-fluor-5-nitrophenyl)-6-methyl-2,4(1H,3H)-pyrimidindion, und aus diesem durch Methylierung das 3-(4-Chlor-2-fluor-5-nitrophenyl)-1,6-dimethyl-2,4(1H,3H)-pyrimidindion (physikalische Daten wie oben).

Beispiel 25

Ein Gemisch bestehend aus 2,45 g Eisenpulver, 8 ml Wasser und 16 ml Aethanol wird mittels eines Oelbads auf 80°C erhitzt. Nach Entfernung des Oelbads werden portionenweise 3,14 g 3-(4-Chlor-2-fluor-5-nitrophenyl)-1,6-dimethyl-2,4(1H,3H)-pyrimidindion so zugegeben, dass die Reaktionslösung auf Rückflusstemperatur gehalten wird. Nach erfolgter Zugabe wird eine Spatelspitze Aktivkohle hinzugefügt und das Reaktionsgemisch noch wenige Minuten auf Rückflusstemperatur gehalten. Dann wird abfiltriert und das farblose Filtrat eingeengt. Das Produkt kristallisiert beim Abkühlen des resultierenden Oels aus. Man erhält das 3-(5-Amino-4-chlor-2-fluorphenyl)-1,6-dimethyl-2,4(1H,3H)-pyrimidindion, Smp. > 220°C; Massenspektrum (m/e): 283 ($M^+$), 186, 98; Mikroanalyse:

| | C% | H% | N% | F% | Cl% |
|---|---|---|---|---|---|
| berechnet | 50,81 | 3,91 | 14,81 | 6,70 | 12,50 |
| gefunden | 50,60 | 4,16 | 14,64 | 6,50 | 12,39. |

In analoger Weise erhält man beim Einsatz von:

- 3-(4-Chlor-2-fluor-5-nitrophenyl)-1-methyl-6-trifluormethyl)-2,4(1H,3H)-pyrimidindion das 3-(5-Amino-4-chlor-2-fluorphenyl)-1-methyl-6-trifluormethyl-2,4(1H,3H)-pyrimidindion, Smp. 129°C;
- 3-(4-Brom-2-fluor-5-nitrophenyl)-1-methyl-6-trifluormethyl-2,4(1H,3H)-pyrimidindion das 3-(5-Amino-4-brom-2-fluorphenyl)-1-methyl-6-trifluormethyl-2,4(1H,3H)-pyrimidindion, Smp. 145°C.

Beispiel 26

4,24 g Bortrifluorid-ätherat und 6,75 g 3-(5-Amino-4-chlor-2-fluorphenyl)-1-methyl-6-trifluormethyl-2,4-(1H,3H)-pyrimidindion werden bei -15°C in 50 ml Methylenchlorid vorgelegt. Zum Gemisch tropft man innerhalb von 20 Minuten und unter Kühlen eine Lösung von 3,2 ml Isopentylnitrit in 20 ml Methylenchlorid zu. Anschliessend rührt man das Gemisch 45 Minuten bei 5°C nach, versetzt es mit 50 ml n-Pentan und filtriert die resultierenden Kristalle ab. Man erhält das 2-Chlor-5-[3,6-dihydro-2,6-dioxo-3-methyl-4-trifluormethyl-1(2H)-pyrimidinanyl]-4-fluorbenzoldiazonium-tetrafluorborat, Smp. 191°C (Zersetzung).

In analoger Weise erhält man beim Einsatz von:

- 3-(5-Amino-4-brom-2-fluorphenyl)-1-methyl-6-trifluormethyl-2,4(1H,3H)-pyrimidindion das 2-Brom-5[3,6-dihydro-2,6-dioxo-3-methyl-4-trifluormethyl-1(2H)-pyrimidinyl]-4-fluorbenzoldiazonium-tetrafluorborat, Smp. 218-220°C (Zersetzung);
- 3-(5-Amino-4-chlor-2-fluorphenyl)-1,6-dimethyl-2,4(1H,3H) -pyrimidindion das 2-Chlor-5-[3,6-dihydro-2,6-dioxo-3,4-dimethyl-1(2H)-pyrimidinyl]-4-fluorbenzoldiazonium-tetrafluorat, Smp. 248-2543°C (Zersetzung).

Beispiel 27

0,23 g 2-Chlor-5-[3,6-dihydro-2,6-dioxo-3-methyl-4-trifluormethyl-1-(2H)-pyrimidinyl]-4-fluorbenzoldiazonium-tetrafluorborat wird zu einer Lösung von 75 g Kupfer (II)-nitrat-trihydrat in 50 ml Wasser gegeben. Man fügt 67 mg Kupfer (I)-oxid hinzu und rührt das Reaktionsgemisch 10 Minuten bei Raumtemperatur. Anschliessend wird das Gemisch filtriert, das Filtrat mit 50 ml Aethylacetat extrahiert und die organische Phase gewaschen und unter verminderten Druck eingedampft. Das resultierende rohe Produkt wird aus Diäthyläther/n-Hexan kristallisiert. Man erhält das 3-(4-Chlor-2-fluor-5-hydroxyphenyl)-1-methyl-6-trifluormethyl-2,4(1H,3H)-pyrimidindion.

In analoger Weise erhält man beim Einsatz von :

- 2-Brom-5-[3,6-dihydro-2,6-dihydro-2,6-dioxo-3-methyl-4-trifluormethyl-1(2H)-pyrimidinyl]-4-fluorbenzoldiazonium-tetrafluorborat das 3-(4-Brom-2-fluor-5-hydroxyphenyl)-1-methyl-6-trifluormethyl-2,4-(1H,3H)-pyrimidindion;
- 2-Chlor-5-[3,6-dihydro-2,6-dioxo-3,4-dimethyl-1(2H)-pyrimidinyl]-4-fluorbenzoldiazonium-tetrafluorborat das 3-(4-Chlor-2-fluor-5-hydroxyphenyl)-1-6-dimethyl-2,4(1H,3H)-pyrimidindion.

III. Herstellung der Verbindungen der Formel IX:

Beispiel 28

Das im Beispiel 12 verwendete, neue Aethyl-N-(4-chlor-2-fluor-5-isopropoxyphenyl)-carbamat kann wie folgt hergestellt werden:

12,8 g Chlorameisensäure-äthylester, werden unter Rühren zu einer Lösung von 20 g 4-Chlor-2-fluor-5-isopropoxyanilin in 100 ml Methylenchlorid bei Raumtemperatur gegeben. Anschliessend werden 9,4 g Pyridin während 20 Minuten bei 20°C unter Rühren und Kühlen zugetropft. Das Reaktionsgemisch wird 20 Minuten nachgerührt, dreimal mit je 200 ml einer Lösung von 20 ml 2N Salzsäure in 200 ml Wasser gewaschen, über wasserfreiem Natriumsulfat getrocknet und unter vermindertem Druck zur Trockene eingedampft. Man erhält das Aethyl-N-(4-chlor-2-fluor-5-isopropoxyphenyl)-carbamat, Smp. 89-91°C.

IV. Formulierungsbeispiele:

Beispiel 29

Ein emulgierbares Konzentrat enthält folgende Bestandteile:

Verbindung der Formel I bzw. III, XI-XVI (Wirkstoff) 50 g/l
N-Methylpyrrolidon (Hilfslösungsmittel) 200 g/l
Nonylphenol-(10)äthoxylat (nichtionogener Emulgator) 50 g/l
Calcium-dodecylbenzolsulfonat (anionischer Emulgator) 25 g/l
Gemisch von Alkylbenzolen (Lösungsmittel) ad 1000 ml

Der Wirkstoff und die Emulgatoren werden unter Rühren im Hilfslösungsmittel gelöst, und die Lösung wird mit dem Lösungsmittel auf 1 Liter ergänzt.

Das resultierende emulgierbare Konzentrat lässt sich in Wasser emulgieren und ergibt so eine gebrauchsfertige Spritzbrühe mit der gewünschten Konzentration.

Beispiel 30

Zur Herstellung eines 25% Spritzpulvers werden die nachstehend aufgeführten Bestandteile miteinander vermischt:

| | |
|---|---|
| Verbindung der Formel I bzw. III, XI-XVI (Wirkstoff) | 25 g |
| Kieselsäure, hydratisiert | 5 g |
| Natrium-laurylsulfat | 1 g |
| Natrium-lignosulfonat | 2 g |
| Kaolin | 67 g |
| | 100 g |

Anschliessend wird das Gemisch unter Verwendung einer Stiftenmühle oder vergleichbaren Mahleinrichtung fein gemahlen.

Das resultierende Spritzpulver ergibt beim Einrühren in Wasser eine feine Suspension, die sich als gebrauchsfertige Spritzbrühe eignet.

## Ansprüche

1. Verbindungen der allgemeinen Formel

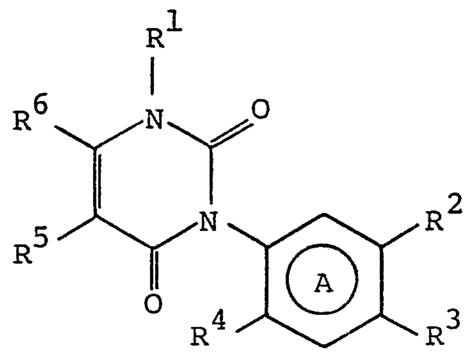

I

worin
$R^1$ Waserstoff, $C_{1-4}$-Alkyl, $C_{1-4}$-Halogenalkyl, Formyl oder $C_{2-6}$-Alkanoyl bedeutet,
$R^2$ eine Aethergruppe oder einen eine (Thio) Carbonyloxygruppe oder Sulfonyloxygruppe enthaltenden Rest bedeutet, wobei dieser Rest unmittelbar über das Sauerstoffatom an den Benzolkern A geknüpft ist,
und
$R^3$ Halogen oder Cyano,
$R^4$ Wasserstoff oder Halogen,
$R^5$ Wasserstoff, Halogen oder $C_{1-4}$ -Alkyl,
$R^6$ $C_{1-4}$-Alkyl oder $C_{1-4}$-Halogenalkyl,
oder
$R^5$ und $R^6$ zusammen Tri-oder Tetramethylen bedeuten,
sowie Salze derjenigen Verbindungen der Formel I, in denen $R^1$ Wasserstoff bedeutet.

2. Verbindungen nach Anspruch 1 oder 2, worin $R^1$ verschieden von Wasserstoff ist.

3. Verbindungen nach Anspruch 1 oder 2, worin $R^2$ $C_{1-8}$-Alkoxy, $C_{3-7}$-Cycloalkoxy, $C_{3-8}$-Alkenoxy, $C_{3-6}$-Alkinoxy, C $_{4-8}$-Cycloalkylalkoxy, gegebenenfalls substituiertes Phenyl-C $_{1-4}$-alkoxy, $C_{1-8}$-Halogenalkoxy, $C_{3-9}$ -Halogenalkenoxy, $C_{2-8}$-Alkoxyalkoxy, gegebenenfalls sub stituiertes Phenoxy-$C_{1-4}$-alkoxy, $C_{4-8}$ -Halogenalkenoxyalkoxy, $C_{2-8}$-Alkylthioalkoxy, N-Alkyl-oder N,N-Dialkylcarbamoylalkoxy, worin die Summe der Kohlenstoffatome 2 bis 10 beträgt, $C_{3-8}$-Alkinoyloxy, N-Alkyl-oder N,N-Dialkylcarbamoyloxy, worin die Summe der Kohlenstoffatome 2 bis 10 beträgt, $C_{1-8}$-Alkylsulfonyloxy, gegebenenfalls substituiertes Phenylsulfonyloxy, oder N-Alkyl-oder N,N-Dialkylsulfamoyloxy, worin die Summe der Kohlenstoffatome 1 bis 8 beträgt, bedeutet.

4. Verbindungen nach Anspruch 3, worin $R^2$ $C_{1-3}$-Alkoxy, Allyloxy oder Propargyloxy ist.

5. Verbindungen nach einem der Ansprüche 1 bis 4, worin $R^3$ Chlor oder Brom bedeutet.

6. Verbindungen nach einem der Ansprüche 1 bis 5, worin $R^4$ Fluor bedeutet.

7. Verbindungen nach einem der Ansprüche 1 bis 6, worin $R^5$ Wasserstoff, Fluor oder Methyl bedeutet.

8. Verbindungen nach einem der Ansprüche 1 bis 7, worin $R^6$ Methyl oder Trifluormethyl bedeutet.

9. Verbindungen nach Anspruch 1, worin $R^1$ verschieden von Wasserstoff ist und $R^5$ Wasserstoff bedeutet.

10. Verbindungen nach Anspruch 1, worin $R^1$ verschieden von Wasserstoff ist und $R^4$ Fluor und $R^5$ Wasserstoff bedeuten.

11. Verbindungen nach Anspruch 1, worin $R^1$ verschieden von Wasserstoff ist und $R^5$ Wasserstoff und $R^6$ Trifluormethyl bedeuten.

12. Verbindungen nach Anspruch 1, worin $R^1$ Methyl, $R^4$ Fluor, $R^5$ Wasserstoff und $R^6$ Trifluormethyl bedeuten.

13. Verbindungen nach Anspruch 1, worin $R^1$ Methyl, $R^3$ Chlor, $R^4$ Fluor, $R^5$ Wasserstoff und $R^6$ Trifluormethyl bedeuten.

14. Eine Verbindung nach Anspruch 1, ausgewählt aus:
3-(4-Chlor-2-fluor-5-propargyloxyphenyl)-1-methyl-6-trifluormethyl-2,4(1H,3H)-pyrimidindion,
3-(4-Chlor-2-fluor-5-isopropoxyphenyl)-1-methyl-6-trifluormethyl-2,4(1H,3H)-pyrimidindion,
3-(5-Aethoxy-4-chlor-2-fluorphenyl)-1-methyl-6-trifluormethyl-2,4(1H,3H)-pyrimidindion,
3-(4-Chlor-2-fluor-5-methoxyphenyl)-1-methyl-6-trifluormethyl-2,4(1H,3H)-pyrimidindion,

3-(5-Butoxy-4-chlor-2-fluorphenyl)-1-methyl-6-trifluormethyl-2,4(1H,3H)-pyrimidindion,
3-(4-Chlor-2-fluor-5-propoxyphenyl)-1-methyl-6-trifluormethyl-2,4(1H,3H)-pyrimidindion,
3-(4-Allyloxy-4-chlor-2-fluorphenyl)-1-methyl-6-trifluormethyl-2,4(1H,3H)-pyrimidindion,
3-(4-Chlor-2-fluor-5-isopropoxyphenyl)-1-methyl-6-pentafluoräthyl-2.4(1H,3H)-pyrimidindion,
3-(4-Chlor-2-fluor-5-isopropoxyphenyl)-5-fluor-1-methyl-6-pentafluoräthyl-2,4(1H,3H)-pyrimidindion,
3-(4-Chlor-2-fluor-5-methoxymethoxy-phenyl)-1-methyl-6-trifluormethyl-2,4(1H,3H)-pyrimidindion,
2-Chlor-5-[3,6-dihydro-2,6-dioxo-3-methyl-4-trifluormethyl-1(2H)-pyrimidinyl]-4-fluorphenyl-N-tert.-butylcarbamat,
3-[5-(2-Bromäthoxy)-4-chlor-2-fluorphenyl]-1-methyl-6-trifluormethyl-2,4(1H,3H)-pyrimidindion,
{2-Chlor-5-[3,6-dihydro-2,6-dioxo-3-methyl-4-trifluormethyl-1(2H)-pyrimidinyl]-4-fluorphenyl}-p-chlorbenzolsulfonat,
3-[4-Chlor-5-(3,3-dichlorallyloxy)-2-fluorphenyl]-1-methyl-6-trifluormethyl-2,4(1H,3H)-pyrimidindion,
3-{4-Chlor-5-[2-(2,2-dichlorvinyloxy)-äthoxy]-2-fluorphenyl}-1-methyl-6-trifluormethyl-2,4(1H,3H)-pyrimidindion,
3-{4-Chlor-2-fluor-5-[2-(2-methyloxyäthoxy)-äthoxy]phenyl}-1-methyl-6-trifluormethyl-2,4(1H,3H)-pyrimidindion,
{2-Chlor-5-[3,6-dihydro-2,6-dioxo-3-methyl-4-trifluormethyl-1(2H)-pyrimidinyl]-4-fluorphenyl}-dimethylsulfamat,
{2-Chlor-5-[3,6-dihydro-2,6-dioxo-3-methyl-4-trifluormethyl-1(2H)-pyrimidinyl]-4-fluorphenyl}-methansulfonat,
3-(4-Chlor-2-fluor-5-methylthiomethoxy-phenyl)-1-methyl-6-trifluormethyl-2,4(1H,3H)-pyrimidindion,
3-(4-Chlor-5-difluormethoxy-2-fluorphenyl)-1-methyl-6-trifluormethyl-2,4(1H,3H)-pyrimidindion,
2-Chlor-5-[3,6-dihydro-2,6-dioxo-3-methyl-4-trifluormethyl-1(2H)-pyrimidinyl]-4-fluorphenyl-2-propiolat,
3-[4-Chlor-2-fluor-5-(2-methoxyäthoxy)-phenyl]-1-methyl-6-trifluormethyl-2,4(1H,3H)-pyrimidindion,
3-[5-(2-Aethoxyäthoxy)-4-chlor-2-fluorphenyl]-1-methyl-6-trifluormethyl-2,4(1H,3H)-pyrimidindion,
3-{4-Chlor-5-[(p-chlorphenoxy)methoxy]-2-fluorphenyl}-1-methyl-6-trifluormethyl-2,4(1H,3H)-pyrimidindion,
3-(5-Benzyloxy-4-chlor-2-fluorphenyl)-1-methyl-6-trifluormethyl-2,4(1H,3H)-pyrimidindion,
2-{2-Chlor-5-[3,6-dihydro-2,6-dioxo-3-methyl-4-trifluormethyl-1(2H)-pyrimidinyl]-4-fluorphenoxy}-N,N-dimethylacetamid,
3-(4-Chlor-2-fluor-5-isopropoxyphenyl)-1-difluormethyl-6-methyl-2,4(1H,3H)-pyrimidindion und
3-(4-Chlor-5-cyclopentoxy-2-fluorphenyl)-1-methyl-6-trifluormethyl-2,4(1H,3H)-pyrimidindion.

15. 3-(4-Chlor-5-cyclohexylmethoxy-2-fluorphenyl)-1-methyl-6-trifluormethyl-2,4(1H,3H)-pyrimidindion.

16. 2-Chlor-5-[3,6-dihydro-2,6-dioxo-3-methyl-4-trifluormethyl-1(2H)-pyrimidinyl]-4-fluorphenyl-isopropansulfonat.

17. Verbindungen der allgemeinen Formel

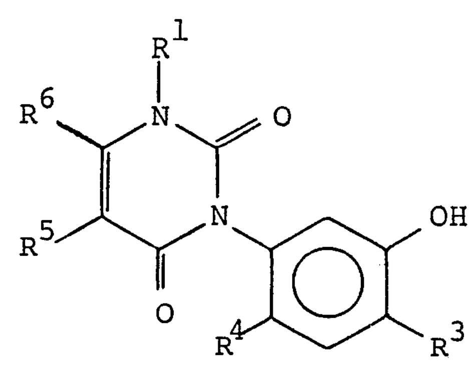

III

worin
$R^1$ Wasserstoff, $C_{1-4}$-Alkyl, $C_{1-4}$-Halogenalkyl, Formyl oder $C_{2-6}$-Alkanoyl,
$R^3$ Halogen oder Cyano,
$R^4$ Wasserstoff oder Halogen,
$R^5$ Wasserstoff, Halogen oder $C_{1-4}$-Alkyl,
$R^6$ $C_{1-4}$-Alkyl oder $C_{1-4}$-Halogenalkyl
oder
$R^5$ und $R^6$ zusammen Tri-oder Tetramethylen bedeuten,

sowie Salze dieser Verbindungen.

18. Verbindungen der allgemeinen Formel

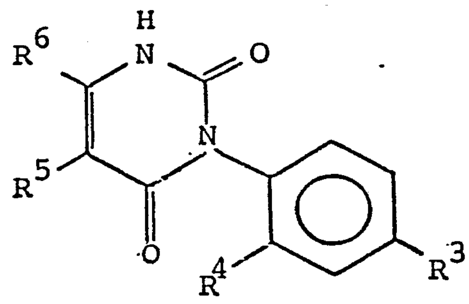

XI

worin
$R^3$ Halogen oder Cyano,
$R^4$ Wasserstoff oder Halogen,
$R^5$ Wasserstoff, Halogen oder $C_{1-4}$-Alkyl,
$R^6$ $C_{1-4}$-Alkyl oder $C_{1-4}$-Halogenalkyl oder
$R^5$ und $R^6$ zusammen Tri-oder Tetramethylen bedeuten.

19. Verbindungen der allgemeinen Formel

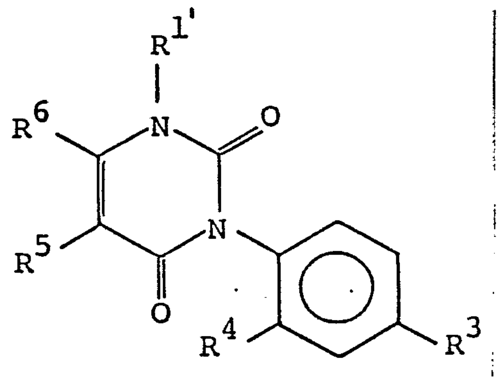

XII

worin
$R^{1'}$ $C_{1-4}$-Alkyl oder $C_{1-4}$-Halogenalkyl,
$R^3$ Halogen oder Cyano,
$R^4$ Wasserstoff oder Halogen,
$R^5$ Wasserstoff, Halogen oder $C_{1-4}$-Alkyl,
$R^6$ $C_{1-4}$-Alkyl oder $C_{1-4}$-Halogenalkyl, oder
$R^5$ und $R^6$ zusammen Tri-oder Tetramethylen bedeuten.

20. Verbindungen der allgemeinen Formel

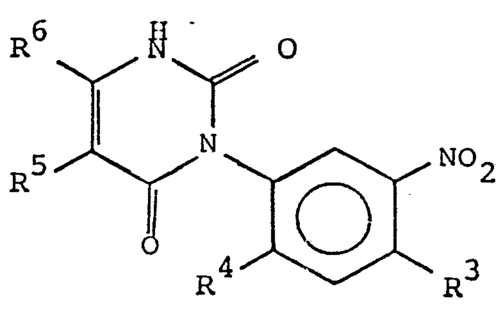

XIII

worin

$R^3$ Halogen oder Cyano,
$R^4$ Wasserstoff oder Halogen,
$R^3$ Wasserstoff, Halogen oder $C_{1-4}$-Alkyl,
$R^6$ $C_{1-4}$-Alkyl oder $C_{1-4}$ -Halogenalkyl, oder
$R^5$ und $R^6$ zzusammen Tri-oder Tetramethylen bedeuten.

Verbindungen der allgemeinen Formel

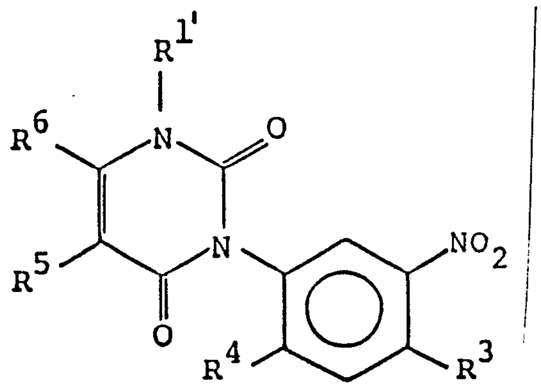

XIV

worin
$R^{1'}$ $C_{1-4}$-Alkyl oder $C_{1-4}$-Halogenalkyl,
$R^3$ Halogen oder Cyano,
$R^4$ Wasserstoff oder Halogen,
$R^5$ Wasserstoff, Halogen oder $C_{1-4}$-Alkyl,
$R^6$ $C_{1-4}$ -Alkyl oder $C_{1-4}$-Halogenalkyl, oder
$R^5$ und $R^6$ zusammen Tri-oder Tetramethylen bedeuten.

22. Verbindungen der allgemeinen Formel

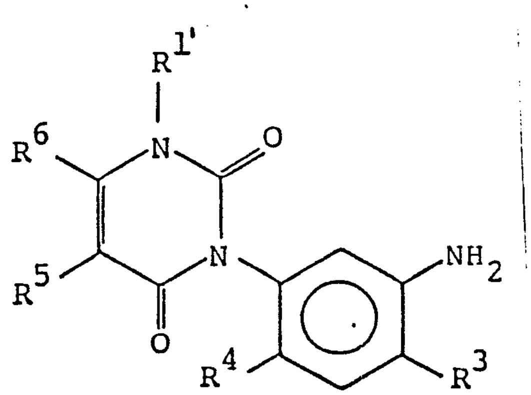

XV

worin
$R^{1'}$ $C_{1-4}$-Alkyl oder $C_{1-4}$ -Halogenalkyl,
$R^3$ Halogen oder Cyano,
$R^4$ Wasserstoff oder Halogen,
$R^5$ Wasserstoff, Halogen oder $C_{1-4}$-Alkyl,
$R^6$ $C_{1-4}$-Alkyl oder $C_{1-4}$-Halogenalkyl, oder
$R^5$ und $R^6$ zusammen Tri-oder Tetramethylen bedeuten.

23. Verbindungen der allgemeinen Formel

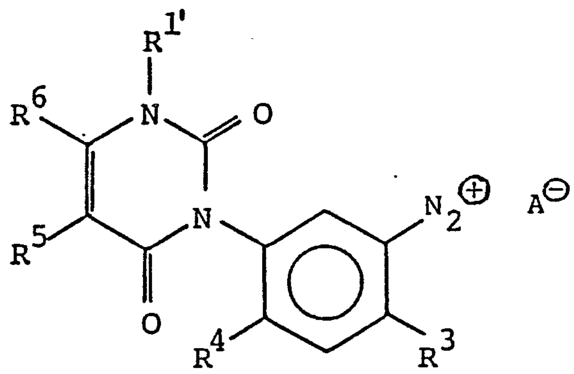

XVI

worin
$R^{1'}$ $C_{1-4}$-Alkyl oder $C_{1-4}$-Halogenalkyl,
$R^3$ Halogen oder Cyano,
$R^4$ Wasserstoff oder Halogen,
$R^5$ Wasserstoff, Halogen oder $C_{1-4}$-Alkyl,
$R^6$ $C_{1-4}$ -Alkyl oder $C_{1-4}$-Halogenalkyl, oder
$R^5$ und $R^6$ zusammen Tri-oder Tetramethylen und
$A^{\ominus}$ eine Anion bedeuten.

24. Unkrautbekämpfungsmittel, dadurch gekennzeichnet, dass es eine wirksame Menge mindestens einer Verbindung der allgemeinen Formel

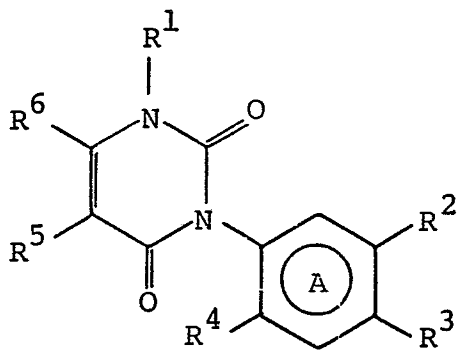

I

worin
$R^1$ Wasserstoff, $C_{1-4}$-Alkyl, $C_{1-4}$-Halogenalky, Formyl oder $C_{2-6}$-Alkanoyl bedeutet,
$R^2$ eine Aethergruppe oder einen eine (Thio)Carbonyloxygruppe oder Sulfonyloxygruppe enthaltenden Rest bedeu tet, wobei dieser Rest unmittelbar über das Sauerstoffatom an den Benzolkern A geknüpft ist,
und
$R^3$ Halogen oder Cyano,
$R^4$ Wasserstoff oder Halogen,
$R^5$ Wasserstoff, Halogen oder $C_{1-4}$-Alkyl,
$R^6$ $C_{1-4}$-Halogenalkyl,
oder
$R^5$ und $R^6$ zusammen Tri-oder Tetramethylen bedeuten,
oder eines Salzes einer solchen Verbindung, in der $R^1$ Wasserstoff bedeutet,
sowie Formulierungshilfsstoffe enthält.

25. Unkrautbekämpfungsmittel nach Anspruch 24, dadurch gekennzeichnet, dass es eine wirksame Menge mindestens einer aus der Gruppe
3-(4-Chlor-2-fluor-5-propargyloxyphenyl)-1-methyl-6-trifluormethyl-2,4(1H,3H)-pyrimidindion,
3-(4-Chlor-2-fluor-5-isopropoxyphenyl)-1-methyl-6-trifluormethyl-2,4(1H,3H)-pyrimidindion,
3-(5-Aethoxy-4-chlor-2-fluorphenyl)-1-methyl-6-trifluormethyl-2,4(1H,3H)-pyrimidindion,

3-(4-Chlor-2-fluor-5-methoxyphenyl)-1-methyl-6-trifluormethyl-2,4(1H,3H)-pyrimidindion,
3-(5-Butoxy-4-chlor-2-fluorphenyl)-1-methyl-6-trifluormethyl-2,4(1H,3H)-pyrimidindion,
3-(4-Chlor-2-fluor-5-propoxyphenyl)-1-methyl-6-trifluormethyl-2,4(1H,3H)-pyrimidindion,
3-(5-Allyloxy-4-chlor-2-fluorphenyl)-1-methyl-6-trifluormethyl-2,4(1H,3H)-pyrimidindion,
3-(4-Chlor-2-fluor-5-isopropoxyphenyl)-1-methyl-6-pentafluoräthyl-2,4(1H,3H)-pyrimidindion,
3-(4-Chlor-2-fluor-5-isopropoxyphenyl)-5-fluor-1-methyl-6-pentafluoräthyl-2,4(1H,3H)-pyrimidindion,
3-(4-Chlor-2-fluor-5-methoxymethoxy-phenyl)-1-methyl-6-trifluormethyl-2,4(1H,3H)-pyrimidindion,
2-Chlor-5-[3,6-dihydro-2,6-dioxo-3-methyl-4-trifluormethyl-1(2H)-pyrimidinyl]-4-fluorphenyl-N-tert.-butylcarbamat,
3-[5-(2-Bromäthoxy)-4-chlor-2-fluorphenyl]-1-methyl-6-trifluormethyl-2,4(1H,3H)-pyrimidindion,
{2-Chlor-5-[3,6-dihydro-2,6-dioxo-3-methyl-4-trifluormethyl-1(2H)-pyrimidinyl]-4-fluorphenyl}-p-chlorbenzolsulfonat,
3-[4-Chlor-5-(3,3-dichlorallyloxy)-2-fluorphenyl]-1-methyl-6-trifluormethyl-2,4(1H,3H)-pyrimidindion,
3-{4-Chlor-5-[2-(2,2-dichlorvinyloxy)-äthoxy]-2-fluorphenyl}-1-methyl-6-trifluormethyl-2,4(1H,3H)-pyrimidindion,
3-{4-Chlor-2-fluor-5-[2-(2-methoxyäthoxy)-äthoxy]phenyl}-1-methyl-6-trifluormethyl-2,4(1H,3H)-pyrimidindion,
{2-Chlor-5-[3,6-dihydro-2,6-dioxo-3-methyl-4-trifluormethyl-1(2H)-pyrimidinyl]-4-fluorphenyl}-dimethylsulfamat,
{2-Chlor-5-[3,6-dihydro-2,6-dioxo-3-methyl-4-trifluormethyl-1(2H)-pyrimidinyl]-4-fluorphenyl}-methansulfonat,
3-(4-Chlor-2-fluor-5-methylthiomethoxy-phenyl)-1-methyl-6-trifluormethyl-2,4(1H,3H)-pyrimidindion,
3-(4-Chlor-5-difluormethoxy-2-fluorphenyl)-1-methyl-6-trifluormethyl-2,4(1H,3H)-pyrimidindion,
2-Chlor-5-[3,6-dihydro-2,6-dioxo-3-methyl-4-trifluormethyl-1(2H)-pyrimidinyl]-4-fluorphenyl-2-propiolat,
3-[4-Chlor-2-fluor-5-(2-methoxyäthoxy)-phenyl]-1-methyl-6-trifluormethyl-2,4(1H,3H)-pyrimidindion,
3-[5-(2-Aethoxyäthoxy)-4-chlor-2-fluorphenyl]-1-methyl-6-trifluormethyl-2,4(1H,3H)-pyrimidindion,
3-{4-Chlor-5-[(p-chlorphenoxy)methoxy]-2-fluorphenyl}-1-methyl-6-trifluormethyl-2,4(1H,3H)-pyrimidindion,
3-(5-Benzyloxy-4-chlor-2-fluorphenyl)-1-methyl-6-trifluormethyl-2,4(1H,3H)-pyrimidindion,
2-{2-Chlor-5-[3,6-dihydro-2,6-dioxo-3-methyl-4-trifluormethyl-1(2H)-pyrimidinyl]-4-fluorphenoxy}-N,N-dimethylacetamid,
3-(4-Chlor-2-fluor-5-isopropoxyphenyl)-1-difluormethyl-6-methyl-2,4(1H,3H)-pyrimidindion und
3-(4-Chlor-5-cyclopentoxy-2-fluorphenyl)-1-methyl-6-trifluormethyl-2,4(1H,3H)-pyrimidindion,
ausgewählten Verbindung sowie Formulierungshilfsstoffe enthält.

26. Unkrautbekämpfungsmittel nach Anspruch 24, dadurch gekennzeichnet, dass es eine wirksame Menge von 3-(4-Chlor-5-cyclohexylmethoxy-2-fluorphenyl)-1-methyl-6-trifluormethyl-2,4(1H,3H)-pyrimidindion oder 2-Chlor-5-[3,6-dihydro-2,6-dioxo-3-methyl-4-trifluormethyl-1(2H)-pyrimidinyl]-4-fluorphenyl-isopopansulfonat sowie Formulierungshilfsstoffe enthält.

27. Unkrautbekämpfungsmittel, dadurch gekennzeichnet, dass es eine wirksame Menge einer Verbindung gemäss Anspruch 17 sowie Formulierungshilfsstoffe enthält.

28. Unkrautbekämpfungsmittel, dadurch gekennzeichnet, dass es ein wirksame Menge einer Verbindung gemäss einem der Ansprüche 18 bis 23 sowie Formulierungshilfsstoffe enthält.

29. Verfahren zur Herstellung von Verbindungen der allgemeinen Formel

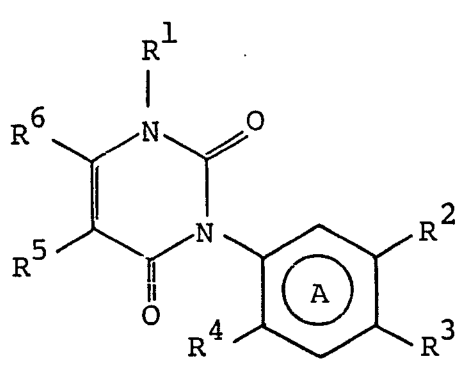

I

worin

$R^1$ Wasserstoff, $C_{1-4}$-Alkyl, $C_{1-4}$-Halogenalkyl, Formyl oder $C_{2-6}$-Alkanoyl bedeutet,
$R^2$ eine Aethergruppe oder einen eine (Thio)Carbonyloxygruppe oder Sulfonyloxygruppe enthaltenden Rest bedeutet, wobei dieser Rest unmittelbar über das Sauerstoffatom an den Benzolkern A geknüpft ist,
und
$R^3$ Halogen oder Cyano,
$R^4$ Wasserstoff oder Halogen,
$R^5$ Wasserstoff, Halogen oder $C_{1-4}$-Alkyl,
$R^6$ $C_{1-4}$-Alkyl oder $C_{1-4}$-Halogenalkyl,
oder
$R^5$ und $R^6$ zusammen Tri-oder Tetramethylen bedeuten,
und von Salzen derjenigen Verbindungen der Formel I, in denen $R^1$ Wasserstoff bedeutet, dadurch gekennzeichnet, dass man

a) eine Verbindung der allgemeinen Formel

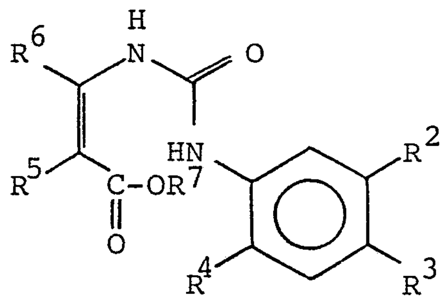

II

worin
$R^2$, $R^3$, $R^4$, $R^5$ und $R^6$ die oben angegebenen Bedeutungen besitzen,
und
$R^7$ nieder alkyl bedeutet,
einer Cyclisierung unter basischen Bedingungen unterwirft, und gegebenenfalls das so erhaltene Salz der algemeinen Formel

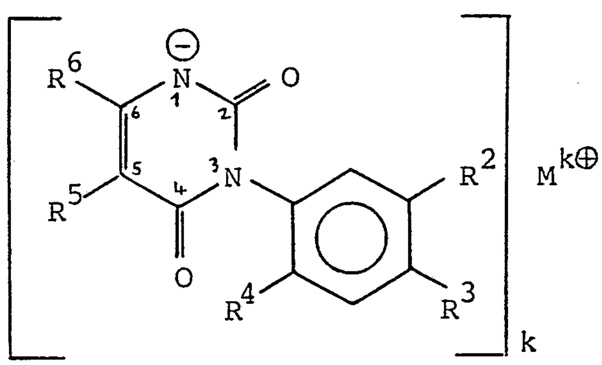

I'

worin
$R^2$, $R^3$, $R^4$, $R^5$ und $R^6$ die oben angegebenen Bedeutungen besitzen,
und
$M^{k\oplus}$ ein Kation
und
k die Wertigkeit des Kations bedeuten,
entweder in die entsprechende saure Form ($R^1$ = Wasserstoff) überführt oder mit dem gewünschten Rest $R^1$ N-substituiert, und eine so erhaltene, 5-unsubstituierte, Verbindung der Formel I ($R^5$= Wasserstoff) gegebenenfalls halogeniert, oder

b) in einer Verbindung der allgemeinen Formel

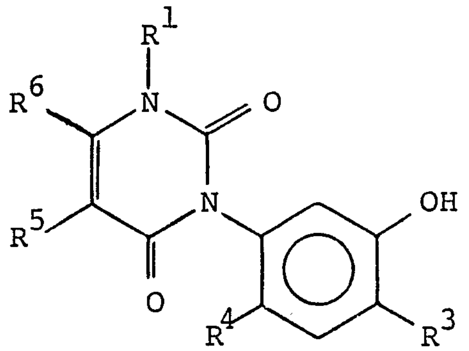

III

worin

$R^1$, $R^3$, $R^4$, $R^5$ und $R^6$ die oben angegebenen Bedeutungen besitzen,

die Hydroxygruppe in an sich bekannter Weise durch den Substituenten $R^2$ ersetzt und eine so erhaltene, 5-unsubstituierte Verbindung der Formel I ($R^5$ = Wasserstoff) gegebenfalls halogeniert.

30. Verfahren zur Bekämpfung von Unkräutern, dadurch gekennzeichnet, dass man das gegen Unkräuter zu schützende Gut und/oder die Unkräuter mit einer wirksamen Menge einer Verbindung gemäss einem der Ansprüche 1 bis 14 und 17 bzw. eines Mittels gemäss einem der Ansprüche 24, 25 und 27 behandelt.

31. Verfahren zur Bekämpfung von Unkräutern, dadurch gekennzeichnet, dass man das gegen Unkräuter zu schützende Gut und/oder die Unkräuter mit einer wirksamen Menge einer Verbindung gemäss einem der Ansprüche 15, 16 und 18 bis 23 bzw. eines Mittels gemäss Anspruch 26 oder 28 behandelt.

32. Verwendung einer Verbindung gemäss einem der Ansprüche 1 bis 14 und 17 bzw. eines Mittels gemäss einem der Ansprüche 24, 25 und 27 zur Bekämpfung von Unkräutern.

33. Verwendung einer Verbindung gemäss einem der Ansprüche 15, 16 und 18 bis 23 bzw. eines Mittels gemäss Anspruch 26 oder 28 zur Bekämpfung von Unkräutern.

Patentansprüche für folgenden Vertragsstaat: AT

1. Unkrautbekämpfungsmittel, dadurch gekennzeichnet, dass es eine wirksame Menge mindestens einer Verbindung der allgemeinen Formel

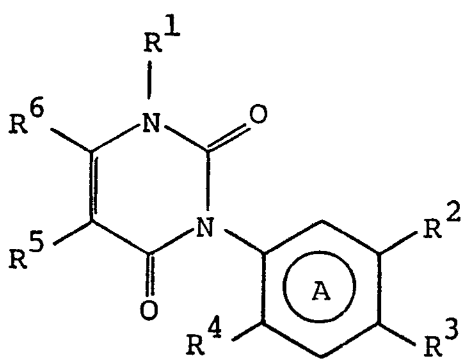

I

worin

$R^1$ Wasserstoff, $C_{1-4}$-Alkyl, $C_{1-4}$-Halogenalkyl, Formyl oder $C_{2-6}$-Alkanoyl bedeutet,

$R^2$ eine Aethergruppe oder einen eine (Thio)Carbonyloxygruppe oder Sulfonyloxygruppe enthaltenden Rest bedeutet, wobei dieser Rest unmittelbar über das Sauerstoffatom an den Benzolkern A geknüpft ist,

und

$R^3$ Halogen oder Cyano,

$R^4$ Wasserstoff oder Halogen,

$R^5$ Wasserstoff, Halogen oder $C_{1-4}$-Alkyl,

$R^6$ $C_{1-4}$-Alkyl oder $C_{1-4}$-Halogenalkyl,

oder

$R^5$ und $R^6$ zusammen Tri-oder Tetramethylen bedeuten,

oder eines Salzes einer solchen Verbindung, in der $R^1$ Wasserstoff bedeutet, sowie Formulierungshilfsstoffe enthält.

2. Unkrautbekämpfungsmittel nach Anspruch 1, worin $R^1$ der Formel I verschieden von Wasserstoff ist.

3. Unkrautbekämpfungsmittel nach Anspruch 1 oder 2, worin $R^2$ in Formel I $C_{1-8}$-Alkoxy, $C_{3-7}$-Cycloalkoxy, $C_{3-8}$-Alkenoxy, $C_{3-6}$-Alkinoxy, $C_{4-8}$-Cycloalkylalkoxy gegebenenfalls substituiertes Phenyl-$C_{1-4}$-alkoxy, $C_{1-8}$ -Halogenalkoxy, $C_{3-9}$-Halogenalkenoxy, $C_{2-8}$-Alkoxyalkoxy, gegebenenfalls substituiertes Phenoxy-$C_{1-4}$-alkoxy, $C_{4-8}$-Halogenalkenoxyalkoxy, $C_{2-8}$-Alkylthioalkoxy, N-Alkyl-oder N,N-Dialkylcarbamoylalkoxy, worin die Summe der Kohlenstoffatome 2 bis 10 beträgt, $C_{3-8}$-Alkinoyloxy, N-Alkyl-oder N,N-Dialkylcarbomoyloxy, worin die Summe der Kohlenstoffatome 2 bis 10 beträgt, $C_{1-8}$-Alkylsulfonyloxy, gegebenenfalls substituiertes Phenylsulfonyloxy, oder N-Alkyl-oder N,N-Dialkylsulfamoyloxy, worin die Summe der Kohlenstoffatome 1 bis 8 beträgt, bedeutet.

4. Unkrautbekämpfungsmittel nach Anspruch 3, worin $R^2$ in Formel I $C_{1-3}$ -Alkoxy, Allyloxy oder Propargyloxy ist.

5. Unkrautbekämpfungsmittel nach einem der Ansprüche 1 bis 4, worin $R^3$ in Formel I Chlor oder Brom bedeutet.

6. Unkrautbekämpfungsmittel nach einem der Ansprüche 1 bis 5, worin $R^4$ in Formel I Fluor bedeutet.

7. Unkrautbekämpfungsmittel nach einem der Ansprüche 1 bis 6, worin $R^5$ in Formel I Wasserstoff, Fluor oder Methyl bedeutet.

Unkrautbekämpfungsmittel nach einem der Ansprüche 1 bis 7, worin $R^6$ in Formel I Methyl oder Trifluormethyl bedeutet.

9. Unkrautbekämpfungsmittel nach Anspruch 1, worin $R^1$ verschieden von Wasserstoff ist und $R^5$ Wasserstoff bedeutet.

10. Unkrautbekämpfungsmittel nach Anspruch 1, worin $R^1$ verschieden von Wasserstoff ist und $R^4$ Fluor und $R^5$ Wasserstoff bedeuten.

11. Unkrautbekämpfungsmittel nach Anspruch 1, worin $R^1$ verschieden von Wasserstoff ist und $R^5$ Wasserstoff und $R^6$ Trifluormethyl bedeuten.

12. Unkrautbekämpfungsmittel nach Anspruch 1, worin $R^1$ Methyl, $R^4$ Fluor, $R^5$ Wasserstoff und $R^6$ Trifluormethyl bedeuten.

13. Unkrautbekämpfungsmittel nach Anspruch 1, worin $R^1$ Methyl, $R^3$ Chlor, $R^4$ Fluor, $R^5$ Wasserstoff und $R^6$ Trifluormethyl bedeuten.

14. Unkrautbekämpfungsmittel nach Anspruch 1, dadurch gekennzeichnet, dass es eine wirksame Menge mindestens einer aus der Gruppe:

3-(4-Chlor-2-fluor-5-propargyloxyphenyl)-1-methyl-6-trifluormethyl-2,4(1H,3H) = pyrimidindion,
3-(4-Chlor-2-fluor-5-isopropoxyphenyl)-1-methyl-6-trifluormethyl-2,4(1H,3H)-pyrimidindion,
3-(5-Aethoxy-4-chlor-2-fluorphenyl)-1-methyl-6-trifluormethyl-2,4(1H,3H)-pyrimidindion,
3-(4-Chlor-2-fluor-5-methoxyphenyl)-1-methyl-6-trifluormethyl-2,4(1H,3H)-pyrimidindion,
3-(5-Butoxy-4-chlor-2-fluorphenyl)-1-methyl-6-trifluormethyl-2,4(1H,3H)-pyrimidindion,
3-(4-Chlor-2-fluor-5-propoxyphenyl)-1-methyl-6-trifluormethyl-2,4(1H,3H)-pyrimidindion,
3-(5-allyloxy-4-chlor-2-fluorphenyl)-1-methyl-6-trifluormethyl-2,4(1H,3H)-pyrimidindion,
3-(4-Chlor-2-fluor-5-isopropoxyphenyl)-1-methyl-6-pentafluoräthyl-2,4(1H,3H)-pyrimidindion,
3-(4-Chlor-2-fluor-5-isopropoxyphenyl)-5-fluor-1-methyl-6-pentafluoräthyl-2,4(1H,3H)-pyrimidindion,
3-(4-Chlor-2-fluor-5-methoxymethoxy-phenyl)-1-methyl-6-trifluormethyl-2,4(1H,3H)-pyrimidindion,
2-Chlor-5-[3,6-dihydro-2,6-dioxo-3-methyl-4-trifluormethyl-1(2H)-pyrimidinyl]-4-fluorphenyl-N-tert.-butylcarbamat,
3-[5-(2-Bromäthoxy)-4-chlor-2-fluorphenyl]-1-methyl-6-trifluormethyl-2,4(1H,3H)-pyrimidindion,
{2-Chlor-5-[3,6-dihydro-2,6-dioxo-3-methyl-4-trifluormethyl-1(2H)-pyrimidinyl]-4-fluorphenyl}-p-chlorbenzolsulfonat,
3-[4-Chlor-5-(3,3-dichlorallyloxy)-2-fluorphenyl]-1-methyl-6-trifluormethyl-2,4(1H,3H)-pyrimidindion,
3-{4-Chlor-5-[2-(2,2-dichlorvinyloxy)-äthoxy]-2-fluorphenyl}-1-methyl-6-trifluormethyl-2,4(1H,3H)-pyrimidindion,
3-{4-Chlor-2-fluor-5-[2-(2-methoxyäthoxy)-äthoxy]phenyl}-1-methyl-6-trifluormethyl-2,4(1H,3H)-pyrimidindion,
{2-Chlor-5-[3,6-dihydro-2,6-dioxo-3-methyl-4-trifluormethyl-1(2H)-pyrimidinyl]-4-fluorphenyl}-dimethylsulfamat,
{2-Chlor-5-[3,6-dihydro-2,6-dioxo-3-methyl-4-trifluormethyl-1(2H)-pyrimidinyl]-4-fluorphenyl}-methansulfonat,
3-(4-Chlor-2-fluor-5-methylthiomethoxy-phenyl)-1-methyl-6-trifluormethyl-2,4(1H,3H)-pyrimidindion,
3-(4-Chlor-5-difluormethoxy-2-fluorphenyl)-1-methyl-6-trifluormethyl-2,4(1H,3H)-pyrimidindion,

2-Chlor-5-[3,6-dihydro-2,6-dioxo-3-methyl-4-trifluormethyl-1(2H)-pyrimidinyl]-4-fluorphenyl-2-propiolat,
3-[4-Chlor-2-fluor-5-(2-methoxyäthoxy)-phenyl]-1-methyl-6-trifluormethyl-2,4(1H,3H)-pyrimidindion,
3-[5-(2-Aethoxyäthoxy-4-chlor-2-fluorphenyl]-1-methyl-6-trifluormethyl-2,4(1H,3H)-pyrimidindion,
2-{4-Chlor-5-[(p-chlorphenopxy)methoxyl]-2-fluorphenyl}-1-methyl-6-trifluormethyl-2,4(1H,3H)-pyrimidindion,
3-(5-Benzyloxy-4-chlor-2-fluorphenyl)-1-methyl-6-trifluormethyl-2,4(1H,3H)-pyrimidindion,
2-{2-Chlor-5-[3,6-dihydro-2,6-dioxo-3-methyl-4-trifluormethyl-1(2H)-pyrimidinyl]-4-fluorphenoxy}-N,N-dimethylacetamid,
3-(4-Chlor-2-fluor-5-isopropoxyphenyl)-1-difluormethyl-6-methyl-2,4(1H,3H)-pyrimidindion und
3-(4-Chlor-5-cyclopentoxy-2-fluorphenyl)-1-methyl-6-trifluormethyl-2,5(1H,3H)-pyrimidindion
ausgewählten Verbindung sowie Formulierungshilfsstoffe enthält.

15. Unkrautbekämpfungsmittel nach Anspruch 1, dadurch gekennzeichnet, dass es eine wirksame Menge von
3-(4-Chlor-5-cyclohexylmethoxy-2-fluorphenyl)-1-methyl-6-trifluormethyl-2,4(1H,3H)-pyrimidindion oder
2-Chlor-5-[3,6-dihydro-2,6-dioxo-3-methyl-4-trifluormethyl-1(2H)-pyrimidinyl]-4-fluorphenyl-isopropansulfonat
sowie Formulierungshilfsstoffe enthält.

16. Unkrautbekämpfungsmittel, dadurch gekennzeichnet, dass es eine wirksame Menge mindestens einer Verbindung der allgemeinen Formel

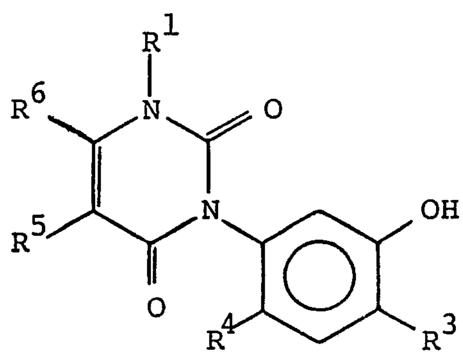

III

worin
$R^1$ Wasserstoff, $C_{1-4}$-Alkyl, $C_{1-4}$-Halogenalky, Formyl oder $C_{2-6}$-Alkanoyl,
$R^3$ Halogen oder Cyano,
$R^4$ Wasserstoff oder Halogen,
$R^5$ Wasserstoff, Halogen oder $C_{1-4}$-Alkyl,
$R^6$ $C_{1-4}$-alkyl oder $C_{1-4}$-Halogenalkyl
oder
$R^5$ und $R^6$ zusammen Tri-oder Tetramethylen bedeuten,
oder eines Salzes einer solchen Verbindung sowie Formulierungshilfsstoffe enthält.

17. Unkrautbekämpfungsmittel, dadurch gekennzeichnet, dass es eine wirksame Menge mindestens einer Verbindung der allgemeinen Formel

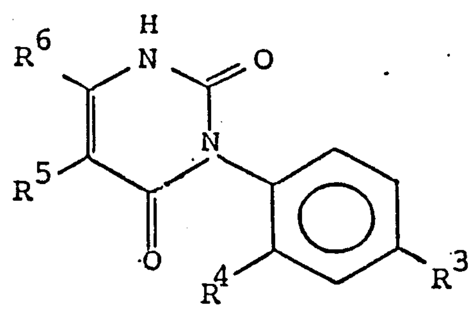

XI

worin
$R^3$ Halogen oder Cycano,

$R^4$ Wasserstoff oder Halogen,
$R^5$ Wasserstoff, Halogen oder $C_{1-4}$-Alkyl,
$R^6$ $C_{1-4}$ -Alkyl oder $C_{1-4}$-Halogenalkyl oder
$R^5$ und $R^6$ zusammen Tri-oder Tetramethylen bedeuten,
sowie Formulierungshilfsstoffe enthält.

18. Unkrautbekämpfungsmittel, dadurch gekennzeichnet, dass es eine wirksame Menge mindestens einer Verbindung der allgemeinen Formel

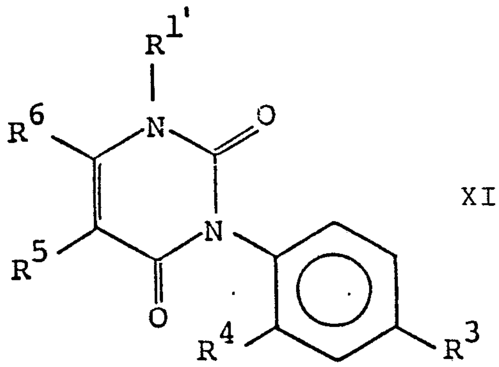

XII

worin
$R^{1'}$ $C_{1-4}$-Alkyl oder $C_{1-4}$ -Halogenalkyl,
$R^3$ Halogen oder Cyano,
$R^4$ Wasserstoff oder Halogen,
$R^5$ Wasserstoff, Halogen oder $C_{1-4}$-Alkyl,
$R^6$ $C_{1-4}$-Alkyl oder $C_{1-4}$-Halogenalkyl, oder
$R^5$ und $R^6$ zusammen Tri-oder Tetramethylen bedeuten,
sowie Formulierungshilfsstoffe enthält.

19. Unkrautbekämpfungsmittel, dadurch gekennzeichnet, dass es eine wirksame Menge mindestens einer Verbindung der allgemeinen Formel

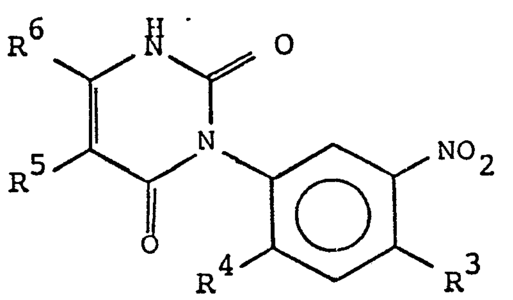

XIII

worin
$R^3$ Halogen oder Cyano,
$R^4$ Wasserstoff oder Halogen,
$R^5$ Wasserstoff, Halogen oder $C_{1-4}$-Alkyl,
$R^6$ $C_{1-4}$-Alkyl oder $C_{1-4}$ -Halogenalkyl, oder
$R^5$ und $R^6$ zusammen Tri-oder Tetramethylen bedeuten,
sowie Formulierungshilfsstoffe enthält.

20. Unkrautbekämpfungsmittel, dadurch gekennzeichnet, dass es eine wirksame Menge mindestens einer Verbindung der allgemeinen Formel

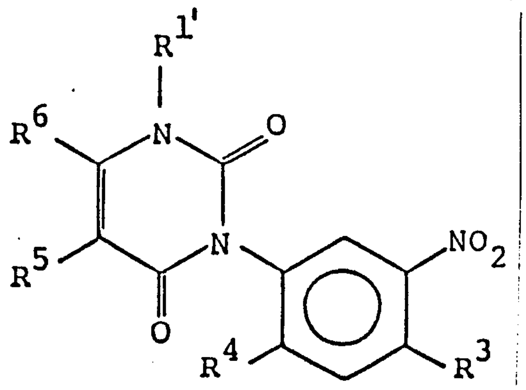

XIV

worin
$R^{1'}$ $C_{1-4}$-Alkyl oder $C_{1-4}$-Halogenalkyl,
$R^3$ Halogen oder Cyano,
$R^4$ Wasserstoff oder Halogen,
$R^5$ Wasserstoff, Halogen oder $C_{1-4}$-Alkyl,
$R^6$ $C_{1-4}$ -Alkyl oder $C_{1-4}$-Halogenalkyl oder
$R^5$ und $R^6$ zusammen Tri-oder Tetramethylen bedeuten,
sowie Formulierungshilfsstoffe enthält.

21. Unkrautbekämpfungsmittel, dadurch gekennzeichnet, dass es eine wirksame Menge mindestens einer Verbindung der allgemeinen Formel

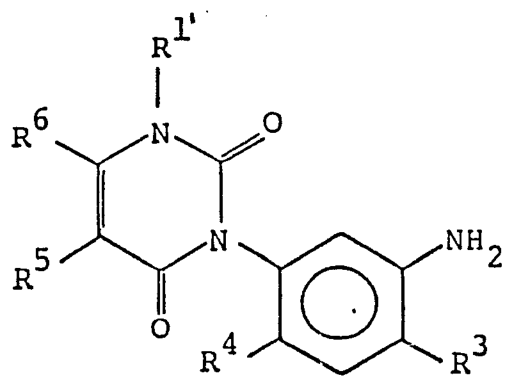

XV

worin
$R^{1'}$ $C_{1-4}$-Alkyl oder $C_{1-4}$-Halogenalkyl,
$R^3$ Halogen oder Cyano,
$R^4$ Wasserstoff oder Halogen,
$R^5$ Wasserstoff, Halogen oder $C_{1-4}$-Alkyl,
$R^6$ $C_{1-4}$-Alkyl oder $C_{1-4}$-Halogenalkyl, oder
$R^5$ und $R^6$ zusammen Tri-oder Tetramethylen bedeuten,
sowie Formulierungshilfsstoffe enthält.

22. Unkrautbekämpfungsmittel, dadurch gekennzeichnet, dass es eine wirksame Menge mindestens einer Verbindung der allgemeinen Formel

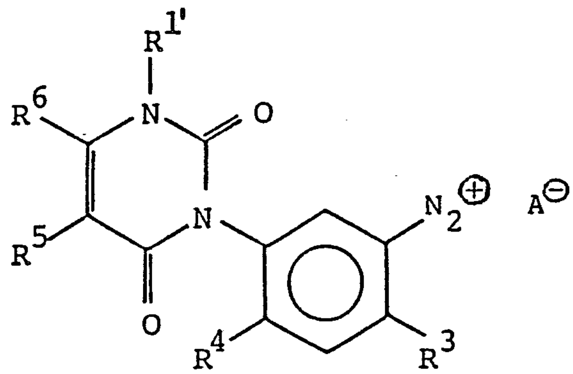

XVI

worin
$R^{1'}$ $C_{1-4}$-Alkyl oder $C_{1-4}$-Halogenalkyl,
$R^3$ Halogen oder Cyano,
$R^4$ Wasserstoff oder Halogen,
$R^5$ Wasserstoff, Halogen oder $C_{1-4}$-Alkyl,
$R^6$ $C_{1-4}$-Alkyl oder $C_{1-4}$-Halogenalkyl, oder
$R^5$ und $R^6$ zusammen Tri-oder Tetramethylen und
$A^{\ominus}$ ein Anion bedeuten,
sowie Formulierungshilfsstoffe enthält.

23. Verfahren zur Herstellung von Verbindungen der allgemeinen Formel

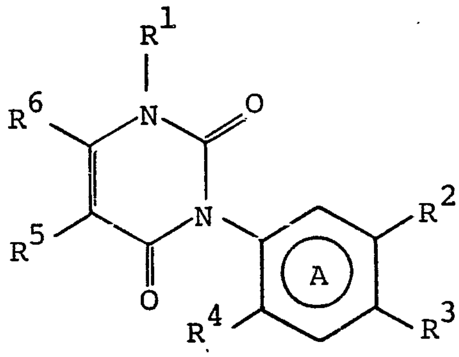

I

worin
$R^1$ Wasserstoff, $C_{1-4}$-Alkyl, $C_{1-4}$-Halogenalkyl, Formyl oder $C_{2-6}$-Alkanoyl bedeutet,
$R^2$ eine Aethergruppe oder einen eine (Thio)Carbonyloxygruppe oder Sulfonyloxygruppe enthaltenden Rest bedeutet, wobei dieser Rest unmittelbar über das Sauerstoffatom an den Benzolkern A geknüpft ist und
$R^3$ Halogen oder Cyano,
$R^4$ Wasserstoff oder Halogen,
$R^5$ Wasserstoff, Halogen oder $C_{1-4}$-Alkyl,
$R^6$ $C_{1-4}$-Alkyl oder $C_{1-4}$-Halogenalkyl,
oder
$R^5$ und $R^6$ zusammen Tri-oder Tetremethylen bedeuten, und von Salzen derjenigen Verbindungen der Formel I, in denen $R^1$ Wasserstoff bedeutet, dadurch gekennzeichnet, dass man

a) eine Verbindung der allgemeinen Formel

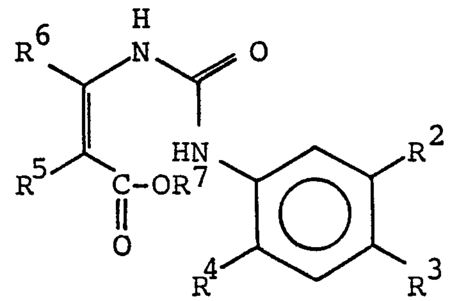

II

worin
$R^2$, $R^3$, $R^4$, $R^5$ und $R^6$ die oben angegebenen Bedeutungen besitzen,
und
$R^7$ nieder Alkyl bedeutet,
einer Cyclisierung unter basischen Bedingungen unterwirft, und gegebenenfalls das so erhaltene Salz der allgemeinen Formel

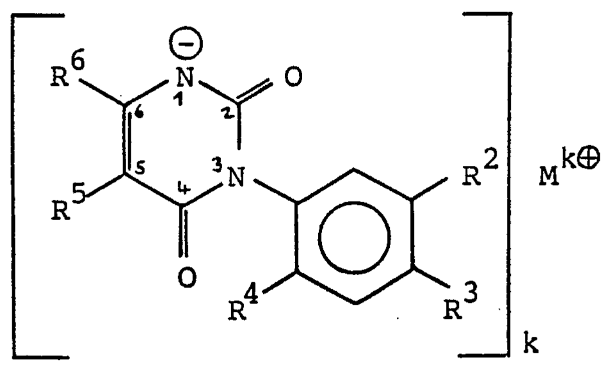

I'

worin
$R^2$,$R^3$, $R^4$, $R^5$ und $R^6$ die oben angegebenen Bedeutungen besitzen,
und
$M^{k\oplus}$ ein Kation
und
k die Wertigkeit des Kations bedeuten,
entweder in die entsprechende saure Form ($R^1$ = Wasserstoff) überführt oder mit dem gewünschen Rest $R^1$ N-substituiert, und eine so erhaltene, 5-unsubstituierte Verbindung der Formel I ($R^5$ = Wasserstoff) gegebenenfalls halogeniert, oder

b) in einer Verbindung der allgemeinen Formel

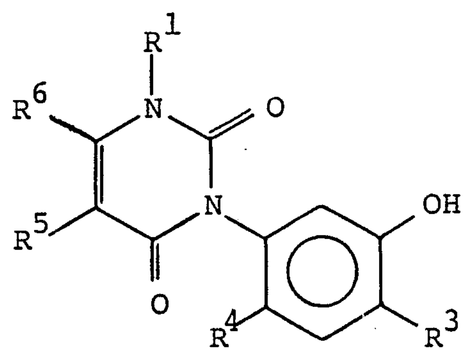

III

worin
$R^1$, $R^3$, $R^4$, $R^5$ und $R^6$ die oben angegebenen Bedeutungen besitzen,
die Hydroxygruppe in an sich bekannter Weise durch den Substituenten $R^2$ ersetzt und eine so erhaltene,

5-unsubsitituierte Verbindung der Formel I ($R^5$ = Wasserstoff) gegebenenfalls halogeniert.

24. Verfahren zur Herstellung eines Unkrautbekämpfungsmittels, dadurch gekennzeichnet, dass man mindestens eine der in Anspruch 2 genannten Verbindungen mit Formulierungshilfsstoffen vermischt.

25. Verfahren zur Bekämpfung von Unkräutern, dadurch gekennzeichnet, dass man das gegen Unkräuter zu schützende Gut und/oder die Unkräuter mit einer wirksamen Menge eines Mittels gemäss einem der Ansprüche 1 bis 14 und 16 behandelt.

26. Verfahren zur Bekämpfung von Unkräutern, dadurch gekennzeichnet, dass man dass gegen Unkräuter zu schützende Gut und/oder die Unkräuter mit einer wirksamen Menge eines Mittels gemäss einem der Ansprüche 15 und 17 bis 22 behandelt.

27. Verwendung eines Mittels gemäss einem der Ansprüche 1 bis 4 und 16 zur Bekämpfung von Unkräutern.

28. Verwendung eines Mittels gemäss einem der Ansprüche 15 und 17 bis 22 zur Bekämpfung von Unkräutern.

Patentansprüche für folgenden Vertragsstaat: ES

1. Verfahren zur Herstellung von Verbindungen der allgemeinen Formel

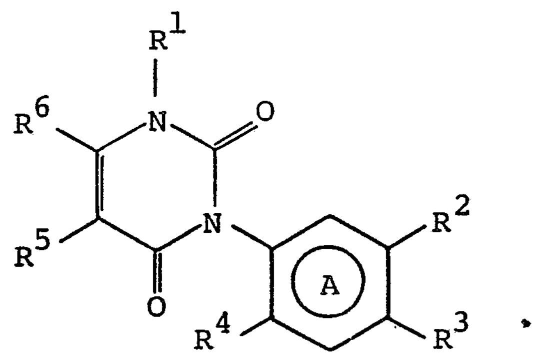

I

worin

$R^1$ Wasserstoff, $C_{1-4}$-Alkyl, $C_{1-4}$-Halogenalkyl, Formyl oder $C_{2-6}$-Alkyanoyl bedeutet,

$R^2$ eine Aethergruppe oder einen eine (Thio)Carbonyloxygruppe oder Sulfonyloxygruppe enthaltenden Rest bedeutet, wobei dieser Rest unmittelbar über das Sauerstoffatom an den Benzolkern A geknüpft ist,

und

$R^3$ Halogen oder Cyano,

$R^4$ Wasserstoff oder Halogen,

$R^5$ Wasserstoff, Halogen oder $C_{1-4}$-Alkyl,

$R^6$ $C_{1-4}$-Alkyl oder $C_{1-4}$-Halogenalkyl,

oder

$R^5$ und $R^6$ zusammen Tri-oder Tetramethylen bedeuten,

und von Salzen derjenigen Verbindungen der Formel I, in denen $R^1$ Wasserstoff bedeutet, dadurch gekennzeichnet, dass man

a) eine Verbindung der allgemeinen Formel

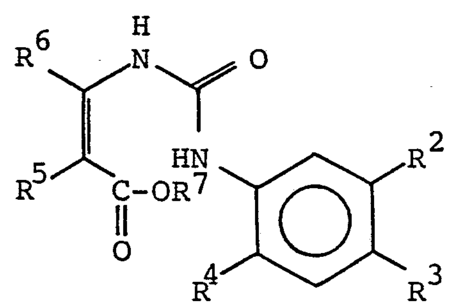

II

worin
$R^2$, $R^3$, $R^4$, $R^5$ und $R^6$ die oben angegebenen Bedeutungen besitzen,
und
$R^7$ nieder Alkyl bedeutet,
einer Cyclisierung unter basischen Bedingungen unterwirft, und gegebenenfalls das so erhaltene Salz der allgemeinen Formel

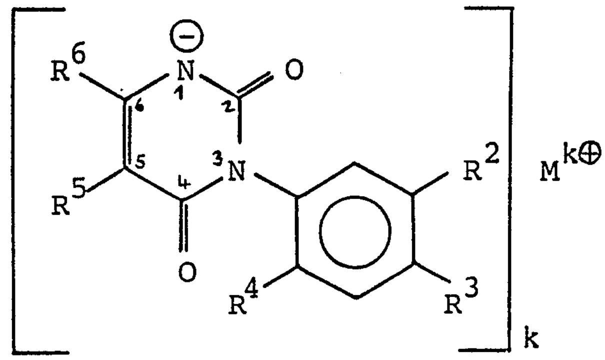

I'

worin
$R^2$, $R^3$, $R^4$, $R^5$ und $R^6$ die oben angegebenen Bedeutungen besitzen,
und
$M^{k\oplus}$ ein Kation
und
k die Wertigkeit des Kations bedeuten,
entweder in die entsprechende saure Form ($R^1$ = Wasserstoff) überführt oder mit dem gewünschten Rest $R^1$ N-substituiert, und eine so erhaltene, 5-unsubstituierte Verbindung der Formel I ($R^5$ = Wasserstoff) gegebenenfalls halogeniert, oder

b) in einer Verbindung der allgemeinen Formel

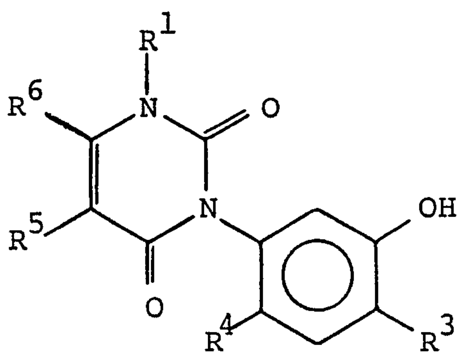

III

worin
$R^1$, $R^3$, $R^4$, $R^5$ und $R^6$ die oben angegebenen Bedeutungen besitzen,
die Hydroxygruppe in an sich bekannter Weise durch den Substituenten $R^2$ ersetzt und eine so erhaltene, 5-unsubstituierte Verbindung der Formel I ($R^5$ = Wasserstoff) gegebenenfalls halogeniert.

2. Verfahren nach Anspruch 1, worin $R^1$ verschieden von Wasserstoff ist.

3. Verfahren nach Anspruch 1 oder 2, worin $R^2$ $C_{1-8}$-Alkoxy, $C_{3-7}$-Cycloalkoxy, $C_{3-8}$-Alkenoxy, $C_{3-6}$-Alkinoxy, $C_{4-8}$-Cycloalkylalkoxy, gegebenenfalls substituiertes Phenyl-$C_{1-4}$-alkoxy, $C_{1-8}$-Halogenalkoxy, $C_{3-9}$-Halogenalkenoxy, $C_{2-8}$-Alkoxyalkoxy, gegebenenfalls substituiertes Phenoxy-$C_{1-4}$-alkoxy, $C_{4-8}$-Halogenalkenoxyalkoxy, $C_{2-8}$-Alkylthioalkoxy, N-Alkyl-oder N,N-Dialkylcarbamoylalkoxy, worin die Summe der Kohlenstoffatome 2 bis 10 beträgt, $C_{3-8}$-Alkinoyloxy, N-Alkyl-oder N,N-Dialkylcarbamoyloxy, worin die Summe der Kohlenstoffatome 2 bis 10 beträgt, $C_{1-8}$-Alkylsulfonyloxy, gegebenenfalls substituiertes Phenylsulfonyloxy, oder N-Alkyl-oder N,N-Dialkylsulfamoyloxy, worin die Summe der Kohlenstoffatome 1 bis 8 beträgt, bedeutet.

4. Verfahren nach Anspruch 3, worin $R^2$ $C_{1-3}$-Alkoxy, Allyloxy oder Propargyloxy ist.

5. Verfahren nach einem der Ansprüche 1 bis 4, worin $R^3$ Chlor oder Brom bedeutet.

6. Verfahren nach einem der Ansprüche 1 bis 5, worin $R^4$ Fluor bedeutet.

7. Verfahren nach einem der Ansprüche 1 bis 6, worin $R^5$ Wasserstoff, Fluor oder Methyl bedeutet.

8. Verfahren nach einem der Ansprüche 1 bis 7, worin $R^6$ Methyl oder Trifluormethyl bedeutet.

9. Verfahren nach Anspruch 1, worin $R^1$ verschieden von Wasserstoff ist und $R^5$ Wasserstoff bedeutet.

10. Verfahren nach Anspruch 1, worin $R^1$ verschieden von Wasserstoff ist und $R^4$ Fluor und $R^5$ Wasserstoff bedeuten.

11. Verfahren nach Anspruch 1, worin $R^1$ vrschieden von Wasserstoff ist und $R^5$ Wasserstoff und $R^6$ Trifluormethyl bedeuten.

12. Verfahren nach Anspruch 1, worin $R^1$ Methyl, $R^4$ Fluor, $R^5$ Wasserstoff und $R^6$ Trifluormethyl bedeuten.

13. Verfahren nach Anspruch 1, worin $R^1$ Methyl, $R^3$ Chlor, $R^4$ Fluor, $R^5$ Wasserstoff und $R^6$ Trifluormethyl bedeuten.

14. Verfahren nach Anspruch 1 zur Herstellung einer Verbindung ausgewählt aus:

3-(4-Chlor-2-fluor-5-propargyloxyphenyl)-1-methyl-6-trifluormethyl-2,4(1H,3H)-pyrimidindion,
3-(4-Chlor-2-fluor-5-isopropoxyphenyl)-1-methyl-6-trifluormethyl-2,4(1H,3H)-pyrimidindion,
3-(5-Aethoxy-4-chlor-2-fluorphenyl)-1-methyl-6-trifluormethyl-2,4(1H,3H)-pyrimidindion,
3-(4-Chlor-2-fluor-5-methoxyphenyl)-1-methyl-6-trifluormethyl-2,4(1H,3H)-pyrimidindion,
3-(5-Butoxy-4-chlor-2-fluorphenyl)-1-methyl-6-trifluormethyl-2,4(1H,3H)-pyrimidindion,
3-(4-Chlor-2-fluor-5-propoxyphenyl)-1-methyl-6-trifluormethyl-2,4(1H,3H)-pyrimidindion,
3-(5-Allyloxy-4-chlor-2-fluorphenyl)-1-methyl-6-trifluormethyl-2,4(1H,3H)-pyrimidindion,
3-(4-Chlor-2-fluor-5-isopropoxyphenyl)-1-methyl-6-pentafluoräthyl-2,4(1H,3H)-pyrimidindion,
3-(4-Chlor-2-fluor-5-isopropoxyphenyl)-5-fluor-1-methyl-6-pentafluoräthyl-2,4(1H,3H)-pyrimidindion,
3-(4-chlor-2-fluor-5-methoxymethoxy-phenyl)-1-methyl-6-trifluormethyl-2,4(1H,3H)-pyrimidindion,
2-Chlor-5-[3,6-dihydro-2,6-dioxo-3-methyl-4-trifluormethyl-1(2H)-pyrimidinyl]-4-fluorphenyl-N-tert.-butylcarbamat,
3-[5-(2-Bromäthoxy)-4-chlor-2-fluorphenyl]-1-methyl-6-trifluormethyl-2,4(1H,3H)-pyrimidindion,
{2-Chlor-5-[3,6-dihydro-2,6-dioxo-3-methyl-4-trifluormethyl-1(2H)-pyrimidinyl]-4-fluorphenyl}-p-chlorbenzolsulfonat,
3-[4-Chlor-5-(3,3-dichlorallyloxy)-2-fluorphenyl]-1-methyl-6-trifluormethyl-2,4(1H,3H)-pyrimidindion,
3-{4-Chlor-5-[2,2-dichlorvinyloxy)-äthoxy]-2-fluorphenyl}-1-methyl-6-trifluormethyl-2,4(1H,3H)-pyrimidindion,
3-{4-Chlor-2-fluor-5-[2-(2-methoxyäthoxy)-äthoxy]-phenyl}-1-methyl-6-trifluormethyl-2,4(1H,3H)-pyrimidindion,
{2-Chlor-5-[3,6-dihydro-2,6-dioxo-3-methyl-4-trifluormethyl-1(2H)-pyrimidinyl]-4-fluorphenyl}-dimethylsulfamat,
{2-Chlor-5-{3,6-dihydro-2,6-dioxo-3-methyl-4-trifluormethyl-1(2H)-pyrimidinyl]-4-fluorphenyl}-methansulfonat,
3-(4-Chlor-2-fluor-5-methylthiomethoxy-phenyl)-1-methyl-6-trifluormethyl-2,4(1H,3H)-pyrimidindion,
3-(4-Chlor-5-difluormethoxy-2-fluorphenyl)-1-methyl-6-trifluormethyl-2,4(1H,3H)-pyrimidindion,
2-Chlor-5-[3,6-dihydro-2,6-dioxo-3-methyl-4-trifluormethyl-1(2H)-pyrimidinyl]-4-fluorphenyl-2-propiolat,
3-[4-Chlor-2-fluor-5-(2-methoxyäthoxy)-phenyl]-1-methyl-6-trifluormethyl-2,4(1H,3H)-pyrimidindion,
3-[5-(2-Aethoxyäthoxy)-4-chlor-2-fluorphenyl]-1-methyl-6-trifluormethyl-2,4(1H,3H)-pyrimidindion,
3-{4-Chlor-5-[(p-chlorphenoxy)methoxy]-2-fluorphenyl}-1-methyl-6-trifluormethyl-2,4(1H,3H)-pyrimidindion,
3-(5-Benzyloxy-4-chlor-2-fluorphenyl)-1-methyl-6-trifluormethyl-2,4(1H,3H)-pyrimidindion,
2-{2-Chlor-5-[3,6-dihydro-2,6-dioxo-3-methyl-4-trifluormethyl-1(2H)-pyrimidinyl]-4-fluorphenoxy}-N,N-dimethylacetamid,
3-(4-Chlor-2-fluor-5-isopropoxyphenyl)-1-difluormethyl-6-methyl-2,4(1H,3H)-pyrimidindion und
3-(4-Chlor-5-cyclopentoxy-2-fluorphenyl)-1-methyl-6-trifluormethyl-2,4(1H,3H)-pyrimidindion.

15. Verfahren nach Anspruch 1 zur Herstellung von 3-(4-Chlor-5-cyclohexylmethoxy-2-fluorphenyl)-1-methyl-6-trifluormethyl-2,4(1H,3H)-pyrimidindion.

16. Verfahren nach Anspruch 1 zur Herstellung von 2-Chlor-5-[3,6-dihydro-2,6-dioxo-3-methyl-4-trifluormethyl-1(2H)-pyrimidinyl]-4-fluorphenyl-isopropansulfonat.

17. Verfahren zur Herstellung von Verbindungen der allgemeinen Formel

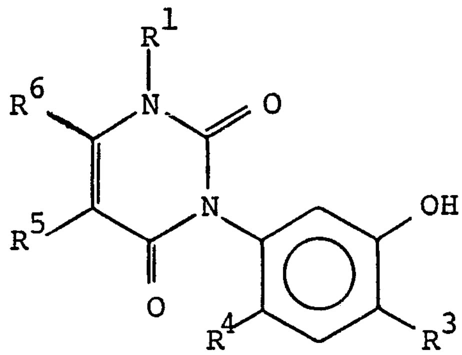

III

worin
$R^1$ Wasserstoff, $C_{1-4}$-Alkyl, $C_{1-4}$-Halogenalkyl, Formyl oder $C_{2-6}$-Alkanoyl,
$R^3$ Halogen oder Cyano,
$R^4$ Wasserstoff oder Halogen,
$R^5$ Wasserstoff, Halogen oder $C_{1-4}$-Alkyl,
$R^6$ $C_{1-4}$-Alkyl oder $C_{1-4}$ -Halogenalkyl
oder
$R^5$ und $R^6$ zusammen Tri-oder Tetramethylen bedeuten,
dadurch gekennzeichnet, dass man das entsprechende geschützte Phenol der allgemeinen Formel

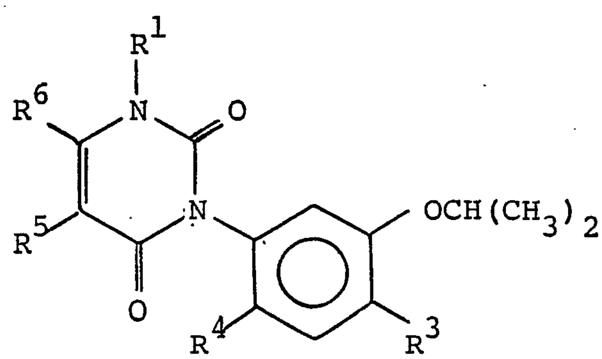

I'''''

worin $R^1$, $R^3$, $R^4$, $R^5$ und $R^6$ die oben angegebenen Bedeutungen besitzen, einer säurekatalysierten Hydrolyse unterwirft.

18. Verfahren zur Herstellung von Verbindungen der allgemeinen Formel

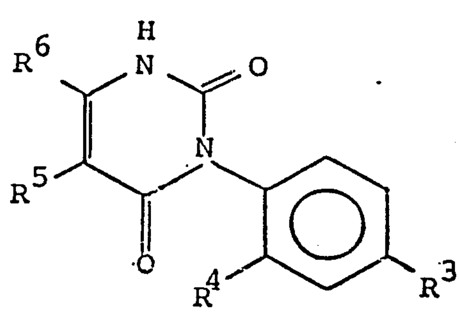

XI

worin
$R^3$ Halogen oder Cyano,
$R^4$ Wasserstoff oder Halogen,
$R^5$ Wasserstoff, Halogen oder $C_{1-4}$-Alkyl,
$R^6$ $C_{1-4}$-Alkyl oder $C_{1-4}$-Halogenalkyl oder
$R^5$ und $R^6$ zusammen Tri-oder Tetramethylen bedeuten,

dadurch gekennzeichnet, dass man eine Verbindung der allgemeinen Formel

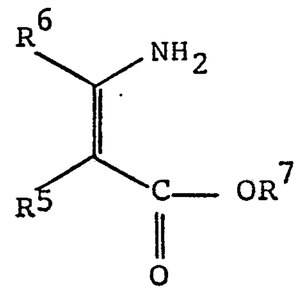

VI

worin $R^5$, $R^6$ und $R^7$ die oben angegebenen Bedeutungen besitzen, mit einer Verbindung der allgemeinen Formel

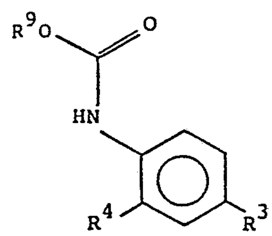

X

worin $R^3$ und $R^4$ die oben angegebenen Bedeutungen besitzen und $R^9$ nieder Alkyl bedeutet, umsetzt.

19. Verfahren zur Herstellung von Verbindungen der allgemeinen Formel

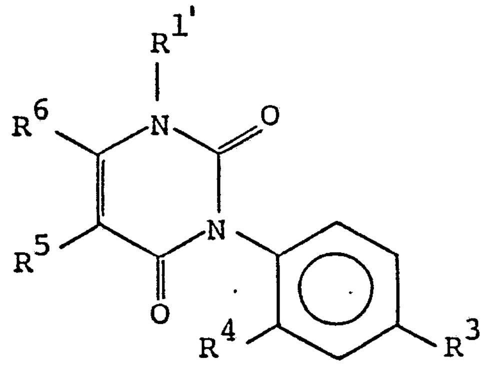

XII

worin
$R^{1'}$ $C_{1-4}$-Alkyl oder $C_{1-4}$-Halogenalkyl,
$R^3$ Halogen oder Cyano,
$R^4$ Wasserstoff oder Halogen,
$R^5$ Wasserstoff, Halogen oder $C_{1-4}$-Alkyl,
$R^6$ $C_{1-4}$-Alkyl oder $C_{1-4}$-Halogenalkyl, oder
$R^5$ und $R^6$ zusammen Tri-oder Tetramethylen bedeuten,
dadurch gekennzeichnet, dass man eine Verbindung der allgemeinen Formel

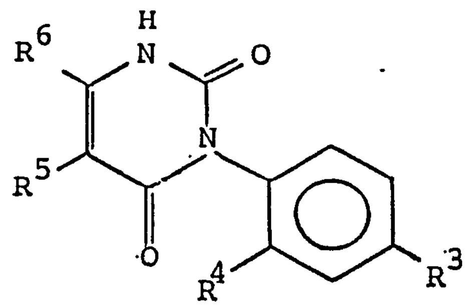

XI

worin $R^3$, $R^4$, $R^5$ und $R^6$ die oben angegebenen Bedeutungen besitzen, oder ein Metallsalz davon mit dem gewünschten $C_{1-4}$-Alkyl-bzw. $C_{1-4}$-Halogenalkylrest $R^{1'}$ N-substituiert.

20. Verfahren zur Herstellung von Verbindungen der allgemeinen Formel

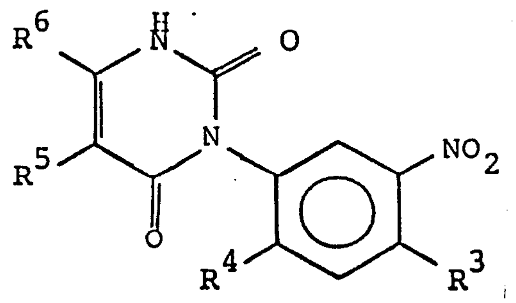

XIII

worin
$R^3$ Halogen oder Cyano,
$R^4$ Wasserstoff oder Halogen,
$R^5$ Wasserstoff, Halogen oder $C_{1-4}$-Alkyl,
$R^6$ $C_{1-4}$-Alkyl oder C $_{1-4}$-Halogenalkyl, oder
$R^5$ und $R^6$ zusammen Tri-oder Tetremethylen bedeuten,
dadurch gekennzeichnet, dass man eine Verbindung der allgemeinen Formel

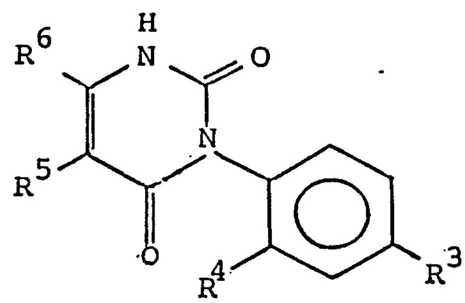

XI

worin $R^3$, $R^4$, $R^5$ und $R^6$ die oben angegebenen Bedeutungen besitzen, nitriert.

21. Verfahren zur Herstellung von Verbindungen der allgemeinen Formel

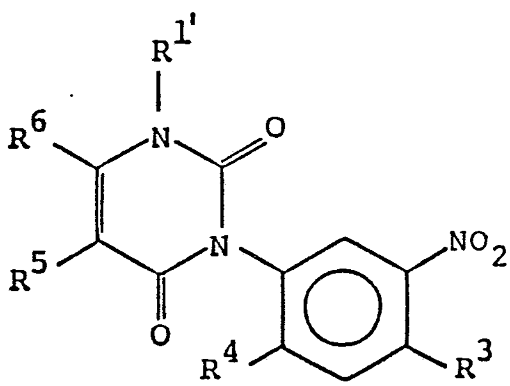

XIV

worin
$R^{1'}$ $C_{1-4}$-Alkyl oder $C_{1-4}$-Halogenalkyl,
$R^3$ Halogen oder Cyano,
$R^4$ Wasserstoff oder Halogen,
$R^5$ Wasserstoff, Halogen oder $C_{1-4}$-Alkyl,
$R^6$ $C_{1-4}$-Alkyl oder $C_{1-4}$-Halogenalkyl, oder
$R^5$ und $R^6$ zusammen Tri-oder Tetramethylen bedeuten,
dadurch gekennzeichnet, dass man eine Verbindung der allgemeinen Formel

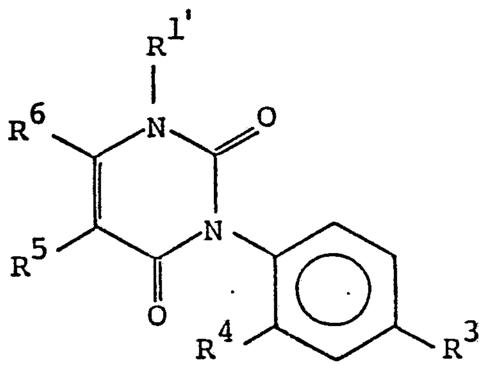

XII

worin $R^{1'}$, $R^3$, $R^4$, $R^5$ und $R^6$ die oben angegebenen Bedeutungen besitzen,
nitriert oder eine Verbindung der allgemeinen Formel

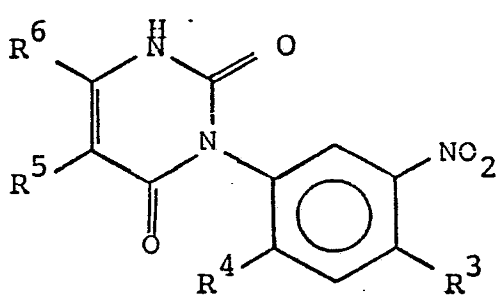

XIII

worin $R^3$, $R^4$, $R^5$ und $R^6$ die oben angegebenen Bedeutungen besitzen,
mit dem gewünschten $C_{1-4}$-alkyl-bzw. $C_{1-4}$-Halogenalkyl-rest $R^{1'}$ N-substituiert.

22. Verfahren zur Herstellung von Verbindungen der allgemeinen Formel

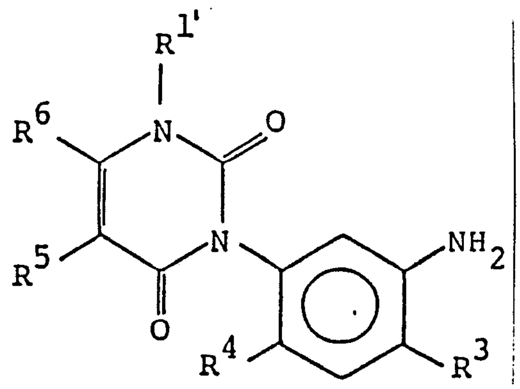

XV

worin
$R^{1'}$ $C_{1-4}$-Alkyl oder $C_{1-4}$-Halogenalkyl,
$R^3$ Halogen oder Cyano,
$R^4$ Wasserstoff oder Halogen,
$R^5$ Wasserstoff, Halogen oder $C_{1-4}$-Alkyl,
$R^6$ $C_{1-4}$ -Alkyl oder $C_{1-4}$-Halogenalkyl, oder
$R^5$ und $R^6$ zusammen Tri-oder Tetramethylen bedeuten,
dadurch gekennzeichnet, dass man in einer Verbindung der allgemeinen Formel

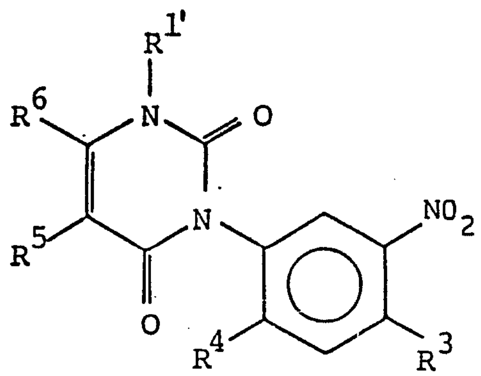

XIV

worin $R^{1'}$, $R^3$, $R^4$, $R^5$ und $R^6$ die oben angegebenen Bedeutungen besitzen, die Nitrogruppe zur Aminogruppe reduziert.

23. Verfahren zur Herstellung von Verbindungen der allgemeinen Formel

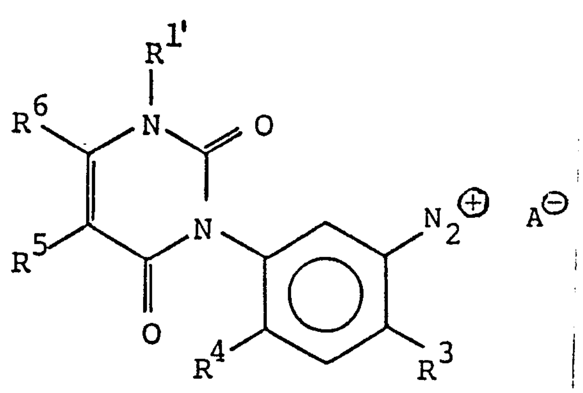

XVI

worin
$R^{1'}$ $C_{1-4}$-Alkyl oder $C_{1-4}$-Halogenalkyl,
$R^3$ Halogen oder Cyano,

$R^4$ Wasserstoff oder Halogen,
$R^5$ $C_{1-4}$-Alkyl oder $C_{1-4}$-Alkyl,
$R^6$ $C_{1-4}$ -Alkyl oder $C_{1-4}$-Halogenalkyl, oder
$R^5$ und $R^6$ zusammen Tri-oder Tetramethylen und
$A^{\ominus}$ ein Anion bedeuten,
dadurch gekennzeichnet, dass man eine Verbindung der allgemeinen Formel

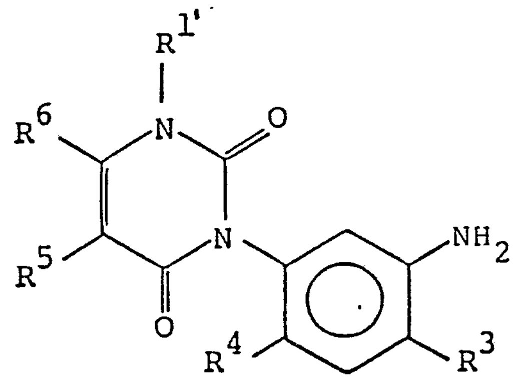

XV

worin $R^{1'}$, $R^3$, $R^4$, $R^5$ und $R^6$ die oben angegebenen Bedeutungen besitzen, einer Diazotierung unterwirft.

24. Unkrautbekämpfungsmittel, dadurch gekennzeichnet, dass es eine wirksame Menge mindestens einer Verbindung der allgemeinen Formel

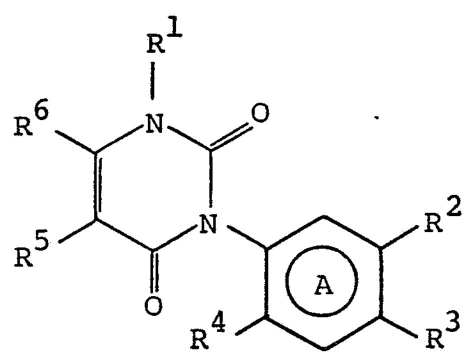

I

worin
$R^1$ Wasserstoff, $C_{1-4}$ -Alkyl, $C_{1-4}$-Halogenalkyl, Formyl oder $C_{2-6}$ -Alkanoyl bedeutet,
$R^2$ eine Aethergruppe oder einen eine (Thio)Carbonyloxygruppe oder Sulfonyloxygruppe enthaltenden Rest bedeutet, wobei dieser Rest unmittelbar über das Sauerstoffatom an den Benzolkern A geknüpft ist,
und
$R^3$ Halogen oder Cyano,
$R^4$ Wasserstoff oder Halogen,
$R^5$ Wasserstoff, Halogen oder $C_{1-4}$-Alkyl,
$R^6$ $C_{1-4}$-Alkyl oder $C_{1-4}$-Halogenalkyl,
oder
$R^5$ und $R^6$ zusammen Tri-oder Tetramethylen bedeuten,
oder eines Salzes einer solchen Verbindung, in der $R^1$ Wasserstoff bedeutet,
sowie Formulierungshilfsstoffe enthält.

25. Unkrautbekämpfungsmittel nach Anspruch 24, dadurch gekennzeichnet, dass es eine wirksame Menge mindestens eienr aus der Gruppe

3-(4-Chlor-2-fluor-5-propargyloxyphenyl)-1-methyl-6-trifluormethyl-2,4(1H,3H)-pyrimidindion,
3-(4-Chlor-2-fluor-5-isopropoxyphenyl)-1-methyl-6-trifluormethyl-2,4(1H,3H)-pyrimidindion,
3-(5-Aethoxy-4-chlor-2-fluorphenyl)-1-methyl-6-trifluormethyl-2,4(1H,3H)-pyrimidindion,
3-(4-Chlor-2-fluor-5-methoxyphenyl)-1-methyl-6-trifluormethyl-2,4(1H,3H)-pyrimidindion,

3-(5-Butoxy-4-chlor-2-fluorphenyl)-1-methyl-6-trifluormethyl-2,4(1H,3H)-pyrimidindion,
3-(4-Chlor-2-fluor-5-propoxyphenyl)-1-methyl-6-trifluormethyl-2,4(1H,3H)-pyrimidindion,
3-(5-Allyloxy-4-chlor-2-fluorphenyl)-1-methyl-6-trifluormethyl-2,4(1H,3H)-pyrimidindion,
3-(4-Chlor-2-fluor-5-isopropoxyphenyl)-1-methyl-6-pentafluoräthyl-2,4(1H,3H)-pyrimidindion,
3-(4-Chlor-2-fluor-5-isopropoxyphenyl)-5-fluor-1-methyl-6-pentafluoräthyl-2,4(1H,3H)-pyrimidindion,
3-(4-Chlor-2-fluor-5-methoxymethoxy-phenyl)-1-methyl-6-trifluormethyl-2,4(1H,3H)-pyrimidindion,
2-Chlor-5-[3,6-dihydro-2,6-dioxo-3-methyl-4-trifluormethyl-1(2H)-pyrimidinyl]-4-fluorphenyl-N-tert.-butylcarbamat,
3-[5-(2-Bromäthoxy)-4-chlor-2-fluorphenyl]-1-methyl-6-trifluormethyl-2,4(1H,3H)-pyrimidindion,
{2-Chlor-5-[3,6-dihydro-2,6-dioxo-3-methyl-4-trifluormethyl-1(2H)-pyrimidinyl]-4-fluorphenyl}-p-chlorbenzolsulfonat,
3-[4-Chlor-5-(3,3-dichlorallyloxy)-2-fluorphenyl]-1-methyl-6-trifluormethyl-2,4(1H,3H)-pyrimidindion,
3-{4-Chlor-5-[2-(2,2-dichlorvinyloxy)-äthoxy]-2-fluorphenyl}-1-methyl-6-trifluormethyl-2,4(1H,3H)-pyrimidindion,
3-{4-Chlor-2-fluor-5-[2-(2-methoxyäthoxy)-äthoxy]-phenyl}-1-methyl-6-trifluormethyl-2,4(1H,3H)-pyrimidindion,
{2-Chlor-5-[3,6-dihydro-2,6-dioxo-3-methyl-4-trifluormethyl-1(2H)-pyrimidinyl]-4-fluorphenyl}-dimethylsulfamat,
{2-Chlor-5-[3,6-dihydro-2,6-dioxo-3-methyl-4-trifluormethyl-1(2H)-pyrimidinyl]-4-fluorphenyl}-methansulfonat,
3-(4-Chlor-2-fluor-5-methylthiomethoxy-phenyl)-1-methyl-6-trifluormethyl-2,4(1H,3H)-pyrimidindion,
3-(4-Chlor-5-difluormethoxy-2-fluorphenyl)-1-methyl-6-trifluormethyl-2,4(1H,3H)-pyrimidindion,
2-Chlor-5-[3,6-dihydro-2,6-dioxo-3-methyl-4-trifluormethyl-1(2H)-pyrimidinyl]-4-fluorphenyl-2-propiolat,
3-[4-Chlor-2-fluor-5-(2-methoxyäthoxy)-phenyl]-1-methyl-6-trifluormethyl-2,4(1H,3H)-pyrimidindion,
3-[5-(2-Aethoxyäthoxy)-4-chlor-2-fluorphenyl[-1-methyl-6-trifluormethyl-2,4(1H,3H)-pyrimidindion,
3-{4-Chlor-5-[(p-chlorphenoxy)methoxy]-2-fluorphenyl}-1-methyl-6-trifluormethyl-2,4(1H,3H)-pyrimidindion,
3-(5-Benzyloxy-4-chlor-2-fluorphenyl)-1-methyl-6-trifluormethyl-2,4(1H,3H)-pyrimidindion,
2-{2-Chlor-5-[3,6-dihydro-2,6-dioxo-3-methyl-4-trifluormethyl-1(2H)-pyrimidinyl]-4-fluorphenoxy}-N,N-dimethylacetamid,
3-(4-Chlor-2-fluor-5-isopropoxyphenyl)-1-difluormethyl-6-methyl-2,4(1H,3H)-pyrimidindion und
3-(4-Chlor-5-cyclopentoxy-2-fluorphenyl)-1-methyl-6-trifluormethyl-2,4(1H,3H)-pyrimidindion
ausgewählten Verbindung sowie Formulierungshilfsstoffe enthält.

26. Unkrautbekämpfungsmittel nach Anspruch 24, dadurch gekennzeichnet, dass es eine wirksame Menge von 3-(4-Chlor-5-cyclohexylmethoxy-2-fluorphenyl)-1-methyl-6-trifluormethyl-2,4(1H,3H)-pyrimidindion oder 2-Chlor-5-[3,6-dihydro-2,6-dioxo-3-methyl-4-trifluormethyl-1(2H)-pyrimidinyl]-4-fluorphenyl-isopropansulfonat sowie Formulierungshilfsstoffe enthält.

27. Unkrautbekämpfungsmittel, dadurch gekennzeichnet, dass es eine wirksame Menge einer Verbindung der Formel III gemäss Anspruch 17 sowie Formulierungshilfsstoffe enthält.

28. Unkrautbekämpfungsmittel, dadurch gekennzeichnet, dass es eine wirksame Menge einer Verbindung der Formel XI, XII, XIII, XIV, XV oder XVI gemäss Anspruch 18, 19, 20, 21, 22 bzw. 23 sowie Formulierungshilfsstoffe enthält.

29. Verfahren zur Herstellung eines Unkrautbekämpfungsmittels, dadurch gekennzeichnet, dass man mindestens eine der in Anspruch 1 genannten Verbindungen mit Formulierungshilfsstoffen vermischt.

Europäisches Patentamt

# EUROPÄISCHER RECHERCHENBERICHT

Nummer der Anmeldung

EP 87110681.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DD - A1 - 229 403 (AKADEMIE DER WISSENSCHAFTEN)<br>* Zusammenfassung; Erfindungsanspruch *<br>-- | 1,24, 29,30, 32 | C 07 D 239/55<br>C 07 D 239/54<br>A 01 N 43/54 |
| P,A | EP - A2 - 0 195 346 (HOFFMANN-LA ROCHE)<br>* Ansprüche 1,18,21,23,25 *<br>---- | 1,24, 29,30, 31 | |
| | | | RECHERCHIERTE SACHGEBIETE (Int. Cl.4) |
| | | | C 07 D 239/00 |

Der vorliegende Recherchenbericht wurde für alle Patentansprüche erstellt.

| Recherchenort | Abschlußdatum der Recherche | Prüfer |
|---|---|---|
| WIEN | 05-11-1987 | LUX |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82